# EUROPEAN PATENT APPLICATION

(11) **EP 3 150 636 A1**
(43) Date of publication of application: **05.04.2017**
(21) Application number: 15188057.2
(22) Date of filing: 02.10.2015
(51) Int. Cl.: C07K 16/46, C07K 16/22, C07K 16/28, C07K 16/40

(54) **TETRAVALENT MULTISPECIFIC ANTIBODIES**

(71) Applicant: F. Hoffmann-La Roche AG, 4070 Basel (CH)
(72) Inventor: The designation of the inventor has not yet been filed
(74) Representative: Burger, Alexander

(57) **Abstract**

The present disclosure relates to novel tetravalent multispecific antibodies, their manufacture and use.

## Description

### FIELD OF THE INVENTION

The present invention relates to novel tetravalent multispecific antibodies, their manufacture and use.

### BACKGROUND OF THE INVENTION

Engineered proteins, such as bi- or multispecific antibodies capable of binding two or more antigens are known in the art. Such multispecific binding proteins can be generated using cell fusion, chemical conjugation, or recombinant DNA techniques.

A wide variety of recombinant multispecific antibody formats have been developed in the recent past, e.g. tetravalent bispecific antibodies by fusion of, e.g. an IgG antibody format and single chain domains (see e.g. Coloma, M.J., et. al., Nature Biotech. 15 (1997) 159-163; WO 2001/077342; and Morrison, S.L., Nature Biotech. 25 (2007) 1233-1234.

One drawback in production of multispecific antibodies is formation of various undesired side products apart from the desired functional molecule. Mispairing includes the pairing of wrong heavy chains with each other as well as pairing of a light chain with a wrong heavy chain counterpart or undesired pairing of light chains.

One approach to circumvent the problem of mispaired byproducts when producing bispecific antibodies aiming at forcing the pairing of two different antibody heavy chains by introducing mutations into the CH3 domains to modify the contact interface is known as 'knobs-into-holes' technology. On one chain bulky amino acids were replaced by amino acids with short side chains to create a 'hole'. Conversely, amino acids with large side chains were introduced into the other CH3 domain, to create a 'knob'. By coexpressing these two heavy chains (and two identical light chains, which have to be appropriate for both heavy chains), high yields of heterodimer formation ('knob-hole') versus homodimer formation ('hole-hole' or 'knob-knob') was observed (Ridgway, J.B., et al., Protein Eng. 9 (1996) 617-621; and WO 96/027011). The percentage of heterodimer could be further increased by remodeling the interaction surfaces of the two CH3 domains using a phage display approach and the introduction of a disulfide bridge to stabilize the heterodimers (Merchant, A.M., et al., Nature Biotech. 16 (1998) 677-681; Atwell, S., et al., J. Mol. Biol. 270 (1997) 26-35). New approaches for the knobs-into-holes technology are described in e.g. in EP 1 870 459 A1. Although this format appears very attractive, no data describing progression towards the clinic are currently available. One important constraint of this strategy is that in order to prevent formation of mispaired byproducts the light chains of the two parent antibodies have to be identical to prevent mispairing and formation of inactive molecules. Thus this technique is not appropriate as a basis for easily developing recombinant, tri-or tetraspecific antibodies against three or four antigens starting from two antibodies against the first and the second antigen, as either the heavy chains of these antibodies and/or the identical light chains have to be optimized first and then further antigen binding peptides against the third and fourth antigen have to be added.

Other approaches for supporting pairing of Fc domains are described in WO 2013/02362 relating to heterodimerized polypeptides. WO 2013/12733 relates to polypeptides comprising heterodimeric Fc regions. WO 2012/131555 relates to engineered hetero-dimeric immunoglobulins. EP 2647707 relates to engineered hetero-dimeric immunoglobulins.

One approach of circumventing the problem of mispaired byproducts in bispecific antibody production aims at forcing the pairing of a light chain polypeptide with its correct heavy chain counterpart. This approach, known as the "CrossMab technology" is based on a domain crossover between heavy and light chains thereby creating different domain arrangements for heavy chains and light chains of different specificity. WO 2009/080251, WO 2009/080252, WO 2009/080253, WO 2009/080254 and Schaefer, W. et al, PNAS, 108 (2011) 11187-1191 relate to bivalent, bispecific IgG antibodies with a domain crossover. WO 2010/145792 and WO 2010/145792 relate to tetravalent antigen binding proteins with a domain crossover.

The multispecific antibodies with a VH/VL replacement/exchange in one binding site to prevent light chain mispairing (CrossMab^{VH-VL}) which are described in WO2009/080252, (see also Schaefer, W. et al, PNAS, 108 (2011) 11187-1191) clearly reduce the byproducts caused by the mismatch of a light chain against a first antigen with the wrong heavy chain against the second antigen (compared to approaches without such domain exchange). However their preparation is not completely free of side products. The main side product is based on a Bence-Jones -type interaction of the wrong light chain with the domain-exchanged heavy chain (see also Schaefer, W. et al, PNAS, 108 (2011) 11187-1191; in Fig. S1I of the Supplement).

Therefore there is still a need for further reduction of such side products improve e.g. the purity of such bispecific antibodies.

### SUMMARY OF THE INVENTION

In one aspect, the present invention relates to a tetravalent multispecific antibody, comprising
1. one core antibody formed by a full length antibody, the full length antibody comprising two Fab fragments specifically binding to a first antigen, and
2. two additional Fab fragments, wherein said additional Fab fragments are fused both either at the C-termini or the N-termini of the heavy chains of the core antibody, wherein the additional Fab fragments specifically bind to a second antigen,
   i) wherein either
      - the Fab fragments specifically binding to the first antigen, or
      - the Fab fragments specifically binding to the second antigen comprise a domain crossover such that the variable heavy chain domain (VH) and the variable light chain domain (VL) are replaced by each other, and
   ii) wherein either
      - the Fab fragments specifically binding to the first antigen, or
      - the Fab fragments specifically binding to the second antigen comprise the following amino acid substitutions in the constant light chain domain (CL) and the constant heavy chain domain 1 (CH1):
      - the amino acid at position 124 in the CL domain is substituted by K, R or H (numbering according to Kabat), and
      - the amino acid at position 147 or 213 in the CH1 domain is substituted by E or D (numbering according to Kabat EU index).

In another aspect, the present invention relates to a tetravalent multispecific antibody, comprising
a) one core antibody formed by a full length antibody, the full length antibody comprising two Fab fragments specifically binding to a first antigen and a second antigen, respectively, and
b) two additional Fab fragments, wherein said additional Fab fragments are fused both either at the C-termini or the N-termini of the heavy chains of the core antibody,
   wherein the additional Fab fragment fused to a heavy chain exhibits the same antigen binding specificity than the Fab fragment of the core antibody arranged
   on said heavy chain,
   i) wherein either
      - the Fab fragments specifically binding to the first antigen, or
      - the Fab fragments specifically binding to the second antigen comprise a domain crossover such that the variable heavy chain domain (VH) and the variable light chain domain (VL) are replaced by each other, and
   ii) wherein either
      - the Fab fragments specifically binding to the first antigen, or
      - the Fab fragments specifically binding to the second antigen comprise the following amino acid substitutions in the constant light chain domain (CL) and the constant heavy chain domain 1 (CH1):
      - the amino acid at position 124 in the CL domain is substituted by K, R or H (numbering according to Kabat), and
      - the amino acid at position 147 or 213 in the CH1 domain is substituted by E or D (numbering according to Kabat EU index).

One embodiment of the invention relates to a multispecific antibody, wherein in the Fab fragments comprising the amino acid substitutions of ii) the amino acids at positions 147 and 213 in the CH1 domain are substituted independently from each other by E or D (numbering according to Kabat EU index).

One embodiment of the invention relates to a multispecific antibody, wherein in the Fab fragments comprising the amino acid substitutions of ii) the amino acids at position 123 and 124 in the CL domain are substituted independently from each other by K, R or H (numbering according to Kabat).

One embodiment of the invention relates to a multispecific antibody, wherein in the Fab fragments comprising the amino acid substitutions of ii) the amino acids at positions 147 and 213 in the CH1 domain are substituted independently from each other by E or D and the amino acids at position 123 and 124 in the CL domain are substituted independently from each other by K, R or H (numbering according to Kabat).

One embodiment of the invention relates to a multispecific antibody, wherein the Fab fragments that do not comprise the amino acid substitutions as defined under ii) comprise the following amino acid substitution in the CL domain the amino acid at position 124 is substituted by E or D.

One embodiment of the invention relates to a multispecific antibody, wherein the additional Fab fragments of b) are fused to the C-termini of the heavy chains of the core antibody.

One embodiment of the invention relates to an antibody, which is bispecific.

One embodiment of the invention relates to a multispecific antibody, wherein the multispecific antibody comprises two constant heavy chain domains 3 (CH3), which are altered to promote heterodimerization by generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains.

One embodiment of the invention relates to a multispecific antibody, wherein the multispecific antibody comprises two CH3 domains which are altered to promote heterodimerization by substituting at least one original amino acid residue in one of the CH3 domains by a positively charged amino acid, and substituting at least one original amino acid residue in the other one of the CH3 domains by a negatively charged amino acid.

One embodiment of the invention relates to a multispecific antibody, wherein the multispecific antibody comprises two CH3 domains, which are altered to promote heterodimerization by introduction of at least one cysteine residue in each CH3 domain such that a disulfide bond is formed between the CH3 domains.

Another aspect of the invention is a nucleic acid encoding the antibody according to the invention.

Another aspect of the invention is an expression vector comprising a nucleic acid according to the invention.

Another aspect of the invention is a host cell comprising a nucleic acid according to the invention.

Another aspect of the invention is a pharmaceutical or diagnostic composition comprising the multispecific antibody according to the invention.

Another aspect of the invention is an immunoconjugate comprising the multispecific antibody according to the invention.

According to the invention, the ratio of a desired multispecific antibody compared to undesired side products like e.g. Bence Jones-type product can be improved by the introduction of substitutions of charged amino acids with the opposite charges at specific amino acid positions in the CH1 and CL domains. Furthermore, aggregation behavior is improved in the antibodies of the invention resulting in a lower aggregate fraction, thereby improving the overall yield of the desired antibody molecule.

### DESCRIPTION OF THE FIGURES

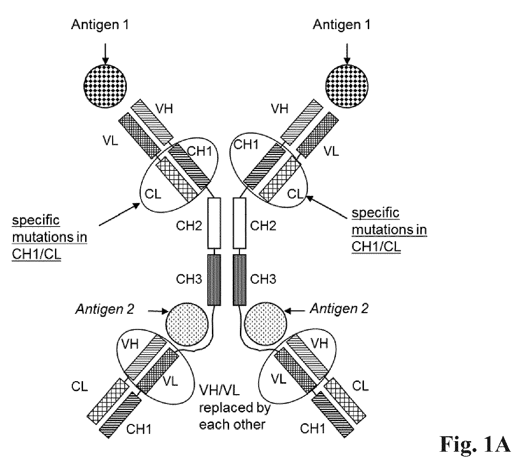

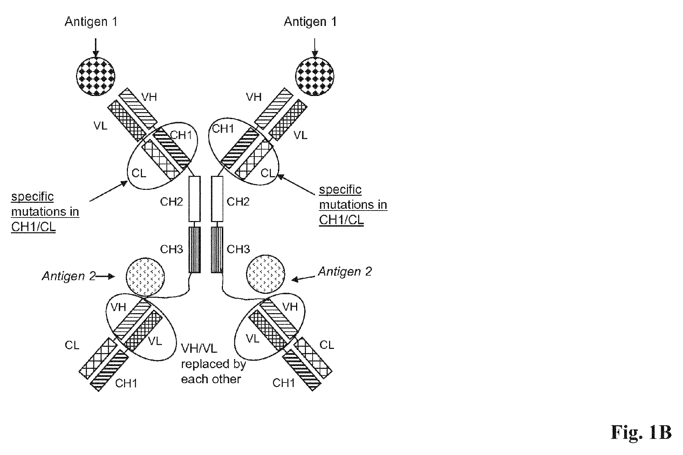

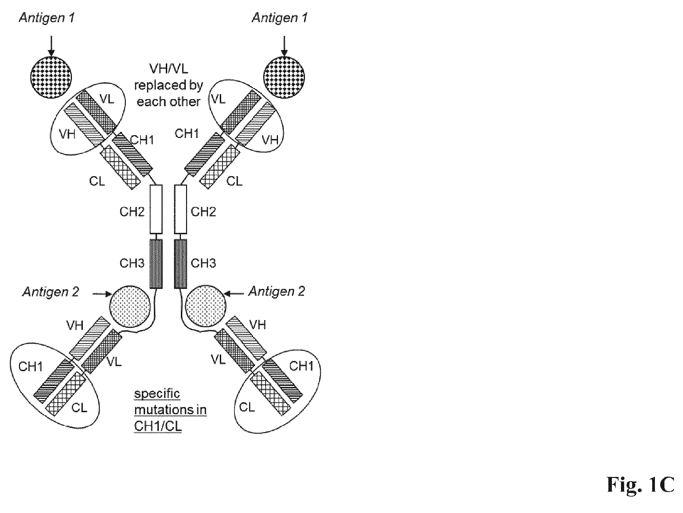

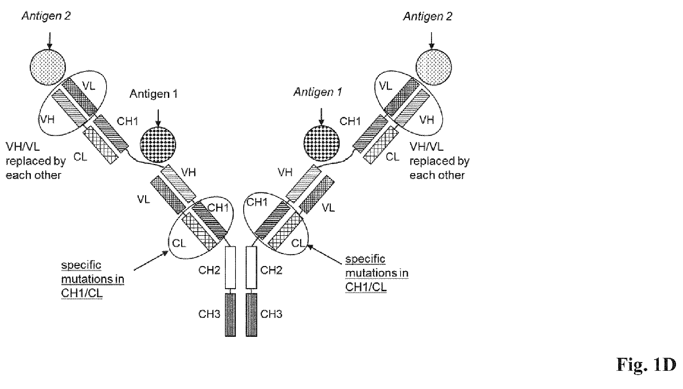

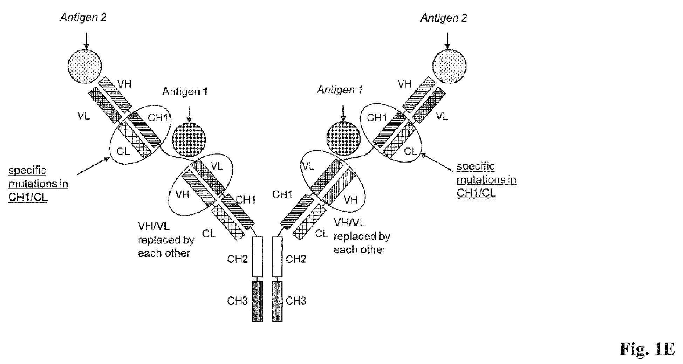

**Figure 1****:** Exemplary illustrations of tetravalent multispecific antibodies according to the first aspect of the invention, i.e. antibodies comprising a core antibody comprising two Fab fragments, which bind to the same antigen.
**Figure 1A** **and B:** Tetravalent multispecific antibody comprising a core antibody with two Fab fragments that specifically bind to a first antigen, wherein to the C-terminus of each one of the heavy chains an additional Fab fragment specifically binding to a second antigen is fused via a peptide connector. Said additional Fab fragments comprise a domain crossover of the VH and VL domains. Within the core antibody, the CH1/CL interface is modified by specific mutations introducing amino acids with positive charges in the CL domain and amino acids with negative charges in the CH1 domain.
**Figure 1C****:** Tetravalent multispecific antibody comprising a core antibody with two Fab fragments that specifically bind to a first antigen, wherein to the C-terminus of each one of the heavy chains an additional Fab fragment specifically binding to a second antigen is fused via a peptide connector. Said additional Fab fragments comprise modifications within the CH1/CL interface by specific mutations introducing amino acids with positive charges in the CL domain and amino acids with negative charges in the CH1 domain. The Fab fragments of the core antibody comprise a domain crossover of the VH and VL domains.
**Figure 1D****:** Tetravalent multispecific antibody comprising a core antibody with two Fab fragments that specifically bind to a first antigen, wherein to the N-terminus of each one of the heavy chains an additional Fab fragment specifically binding to a second antigen is fused via a peptide connector. Said additional Fab fragments comprise a domain crossover of the VH and VL domains. Within the core antibody, the CH1/CL interface is modified by specific mutations introducing amino acids with positive charges in the CL domain and amino acids with negative charges in the CH1 domain.
**Figure 1E****:** Tetravalent multispecific antibody comprising a core antibody with two Fab fragments that specifically bind to a first antigen, wherein to the N-terminus of each one of the heavy chains an additional Fab fragment specifically binding to a second antigen is fused via a peptide connector. Said additional Fab fragments comprise modifications within the CH1/CL interface by specific mutations introducing amino acids with positive charges in the CL domain and amino acids with negative charges in the CH1 domain. The Fab fragments of the core antibody comprise a domain crossover of the VH and VL domains.

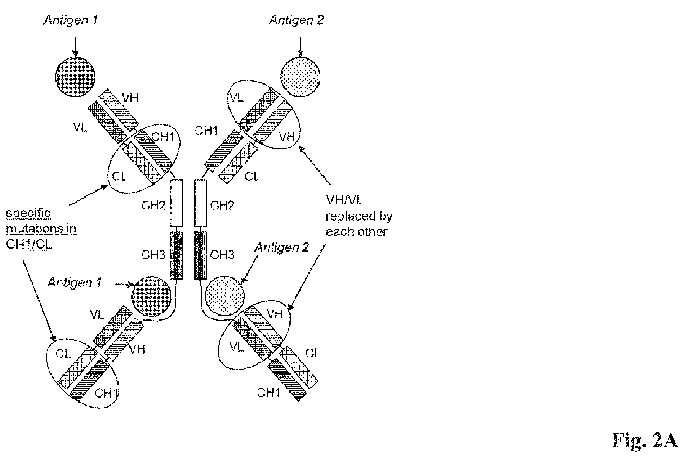

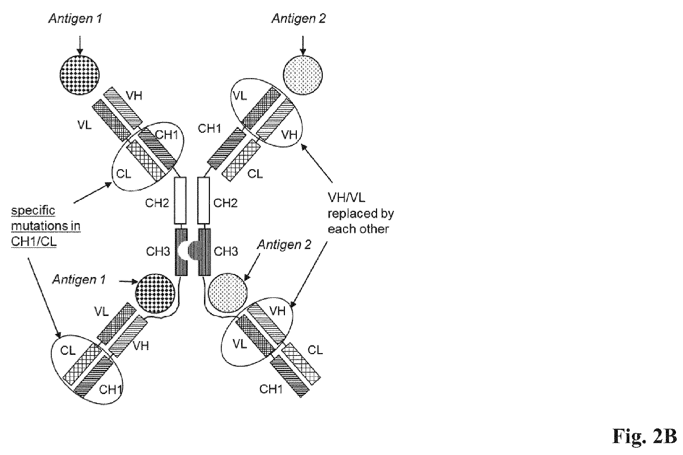

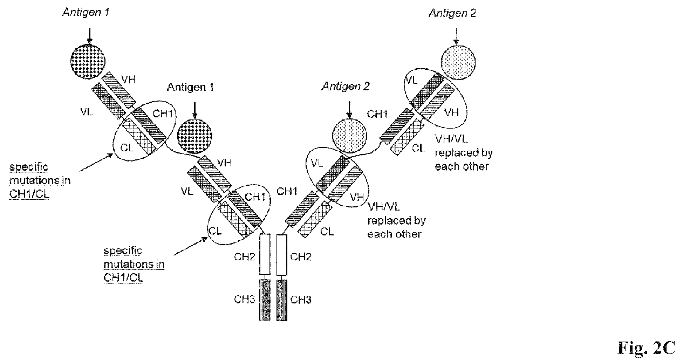

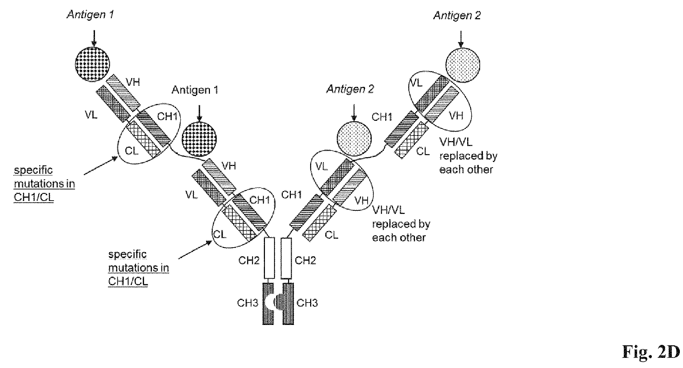

**Figure 2****:** Exemplary illustrations of tetravalent multispecific antibodies according to the second aspect of the invention, i.e. antibodies comprising a core antibody comprising two Fab fragments, which bind to different antigens.
**Figure 2A****:** Tetravalent multispecific antibody comprising a core antibody with two Fab fragments, wherein one Fab fragment specifically binds to a first antigen and the other Fab fragment specifically binds to a second antigen. To the C-terminus of each one of the heavy chains an additional Fab fragment is fused. Said additional Fab fragment specifically binds to the same antigen as the heavy chain to which it is fused (in this example: to the heavy chain specifically binding to the first antigen an additional Fab fragment is fused, which specifically binds to the first antigen; in contrast to the heavy chain specifically binding to the second antigen an additional Fab fragment is fused, which specifically binds to the second antigen). The Fab fragment of the heavy chain to which said additional Fab fragment is fused, and said additional Fab fragment both comprise the same modifications: in this example the Fab fragments specifically binding to the second antigen comprise a domain crossover of the VH and VL domains, and within the Fab fragments specifically binding to the first antigen the CH1/CL interface is modified by specific mutations introducing amino acids with positive charges in the CL domain and amino acids with negative charges in the CH1 domain.
**Figure 2B****:** Tetravalent multispecific antibody comprising a core antibody with two Fab fragments, wherein one Fab fragment specifically binds to a first antigen and the other Fab fragment specifically binds to a second antigen. To the C-terminus of each one of the heavy chains an additional Fab fragment is fused. Said additional Fab fragment specifically binds to the same antigen as the heavy chain to which it is fused (in this example: to the heavy chain specifically binding to the first antigen an additional Fab fragment is fused, which specifically binds to the first antigen; in contrast to the heavy chain specifically binding to the second antigen an additional Fab fragment is fused, which specifically binds to the second antigen). The Fab fragment of the heavy chain to which said additional Fab fragment is fused, and said additional Fab fragment both comprise the same modifications: in this example the Fab fragments specifically binding to the second antigen comprise a domain crossover of the VH and VL domains, and within the Fab fragments specifically binding to the first antigen the CH1/CL interface is modified by specific mutations introducing amino acids with positive charges in the CL domain and amino acids with negative charges in the CH1 domain. The core antibody comprises modifications in the CH3/CH3 interface that support heterodimerization of the heavy chains, e.g. knobs-into-holes mutations, mutations with amino acids of opposite charges in the different CH3 domains and/or additional disulfide bridges.
**Figure 2C****:** Tetravalent multispecific antibody comprising a core antibody with two Fab fragments, wherein one Fab fragment specifically binds to a first antigen and the other Fab fragment specifically binds to a second antigen. To the N-terminus of each one of the heavy chains an additional Fab fragment is fused. Said additional Fab fragment specifically binds to the same antigen as the heavy chain to which it is fused (in this example: to the heavy chain specifically binding to the first antigen an additional Fab fragment is fused, which specifically binds to the first antigen; in contrast to the heavy chain specifically binding to the second antigen an additional Fab fragment is fused, which specifically binds to the second antigen). The Fab fragment of the heavy chain to which said additional Fab fragment is fused, and said additional Fab fragment both comprise the same modifications: in this example the Fab fragments specifically binding to the second antigen comprise a domain crossover of the VH and VL domains, and within the Fab fragments specifically binding to the first antigen the CH1/CL interface is modified by specific mutations introducing amino acids with positive charges in the CL domain and amino acids with negative charges in the CH1 domain.

**Figure 2D****:** Tetravalent multispecific antibody comprising a core antibody with two Fab fragments, wherein one Fab fragment specifically binds to a first antigen and the other Fab fragment specifically binds to a second antigen. To the N-terminus of each one of the heavy chains an additional Fab fragment is fused. Said additional Fab fragment specifically binds to the same antigen as the heavy chain to which it is fused (in this example: to the heavy chain specifically binding to the first antigen an additional Fab fragment is fused, which specifically binds to the first antigen; in contrast to the heavy chain specifically binding to the second antigen an additional Fab fragment is fused, which specifically binds to the second antigen). The Fab fragment of the heavy chain to which said additional Fab fragment is fused, and said additional Fab fragment both comprise the same modifications: in this example the Fab fragments specifically binding to the second antigen comprise a domain crossover of the VH and VL domains, and within the Fab fragments specifically binding to the first antigen the CH1/CL interface is modified by specific mutations introducing amino acids with positive charges in the CL domain and amino acids with negative charges in the CH1 domain. The core antibody comprises modifications in the CH3/CH3 interface that support heterodimerization of the heavy chains, e.g. knobs-into-holes mutations, mutations with amino acids of opposite charges in the different CH3 domains and/or additional disulfide bridges.

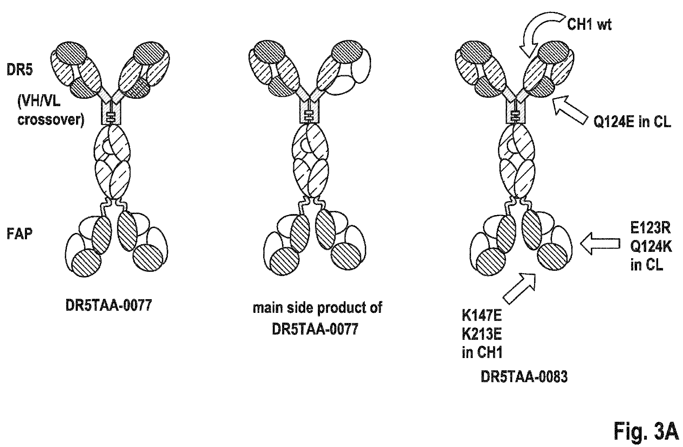

**Figure 3A****:** Domain arrangement and amino acid substitutions of tetravalent anti-DR5-FAP antibodies (Example 2). DR5TAA-0077 control antibody comprises a VH/VL domain crossover in the binding arms of the core antibody, which specifically bind to DR5. The main side product (middle) is an antibody with one non-functional binding site, as the antibody comprises three FAP-specific light chains. DR5TAA-0083 antibody of the invention is indicated on the right side, specifying the amino acid substitutions in the CH1/CL interface.

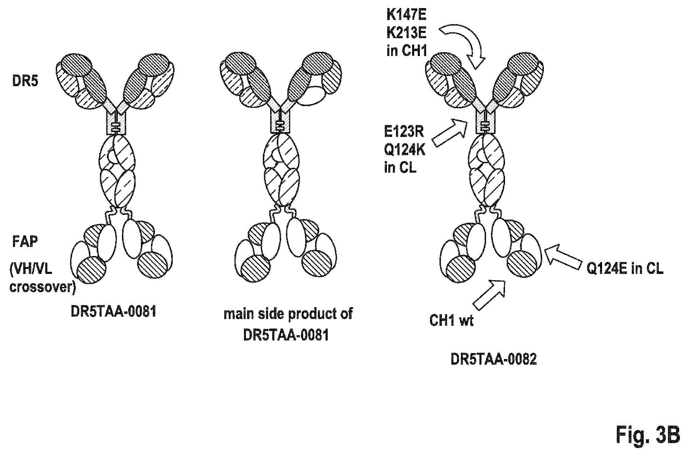

**Figure 3B****:** Domain arrangement and amino acid substitutions of tetravalent anti-DR5-FAP antibodies (Example 2). DR5TAA-0081 control antibody comprises a VH/VL domain crossover in the binding arms of the additional Fab fragments, which specifically bind to FAP. The main side product (middle) is an antibody with one non-functional binding site, as the antibody comprises three FAP-specific light chains. DR5TAA-0082 antibody of the invention is indicated on the right side, specifying the amino acid substitutions in the CH1/CL interface.

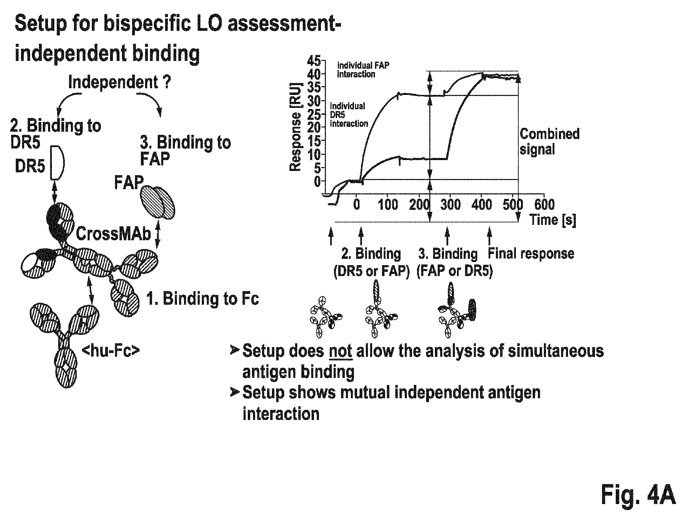

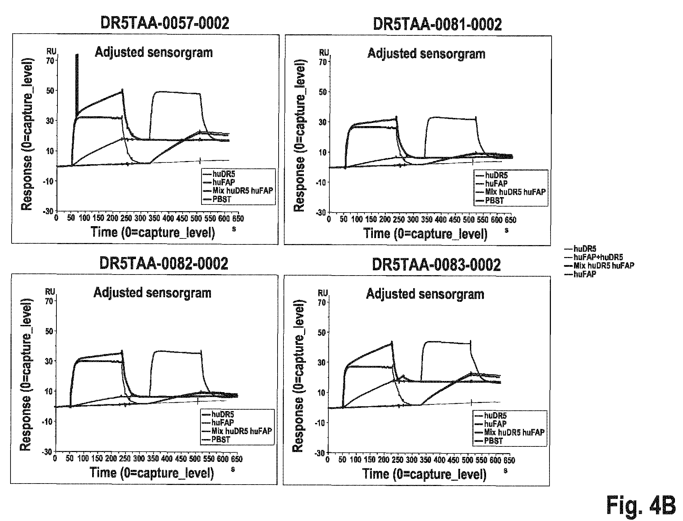

**Figure 4A****-B:** Fig 4A: Exemplary sensorgramm for independent binding of tetravalent anti-DR5-FAP antibodies as assessed by Biacore™ according to Example 3 Fig 4B: Sensorgrams for independent binding of tetravalent anti-DR5-FAP antibodies DR5TAA-0057(control)/ DR5TAA-0077 (control)/DR5TAA-0078 (control).

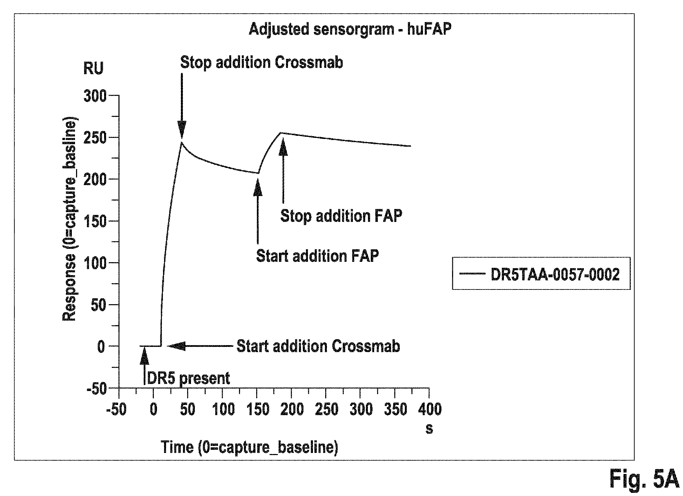

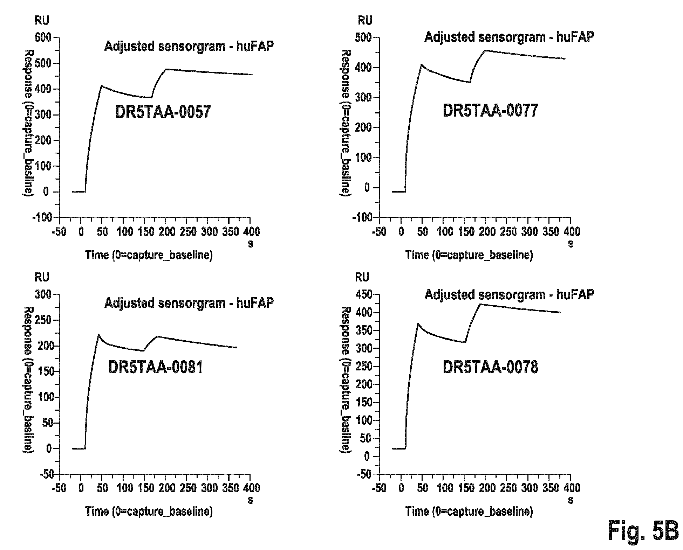

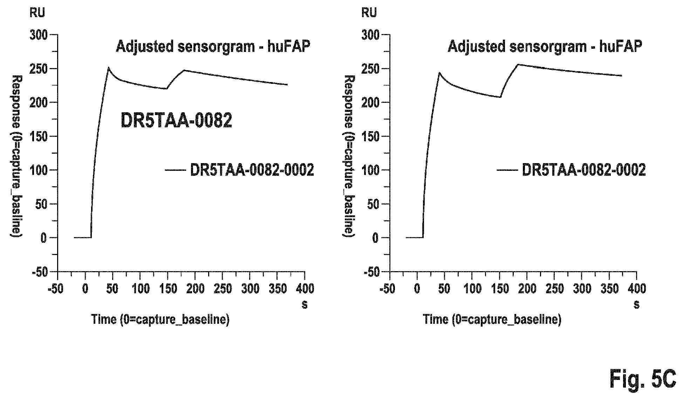

**Figure 5A****-C:** Fig 5A: Exemplary sensorgramm for simultaneous binding of tetravalent anti-DR5-FAP antibodies as assessed by Biacore™ according to Example 4. Fig 5B: Sensorgrams for simultaneous binding of tetravalent anti-DR5-FAP antibodies DR5TAA-0057(control)/ DR5TAA-0077 (control)/DR5TAA-0078 (control)/ DR5TAA-0081 (control). Fig 5C: Sensorgrams of simultaneous binding of tetravalent anti-DR5-FAP antibodies DR5TAA-0082, DR5TAA-0083

### DETAILED DESCRIPTION OF THE INVENTION

### 1. Definitions

The terms "a", "an" and "the" generally include plural referents, unless the context clearly indicates otherwise.

The terms "either ... or ..." as used herein refers to alternative features which may not be realized at the same time. Hence, when referred to "either X or Y" herein, it is meant that only X, or only Y, but not "X and Y" may be realized.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

"Multispecific antibodies" bind two or more different epitopes (for example, two, three, four, or more different epitopes). The epitopes may be on the same or different antigens. An example of a multispecific antibody is a "bispecific antibody" which binds two different epitopes.

When an antibody possesses more than one specificity, the recognized epitopes may be associated with a single antigen or with more than one antigen.

The term "valent" as used herein denotes the presence of a specified number of binding sites in an antibody molecule. A natural antibody for example has two binding sites and is bivalent. As such, the term "trivalent" denotes the presence of three binding sites in an antibody molecule. The antibody according to the invention is at least trivalent, i.e. comprises at least three antigen binding sites.

"Antibody specificity" refers to selective recognition of a particular epitope of an antigen by the antibody. Natural antibodies, for example, are monospecific. The term "monospecific antibody" as used herein denotes an antibody that has one or more binding sites each of which bind to the same epitope of the same antigen.

An epitope is a region of an antigen that is bound by an antibody. The term "epitope" includes any polypeptide determinant capable of specific binding to an antibody. In certain embodiments, epitope determinants include chemically active surface groupings of molecules such as amino acids, glycan side chains, phosphoryl, or sulfonyl, and, in certain embodiments, may have specific three dimensional structural characteristics, and/or specific charge characteristics. In certain embodiments, an antibody is said to specifically bind an antigen when it preferentially recognizes its target antigen in a complex mixture of proteins and/or macromolecules.

As used herein, the terms "binding" and "specific binding" refer to the binding of the antibody to an epitope of the antigen in an *in vitro* assay, preferably in a plasmon resonance assay (BIAcore, GE-Healthcare Uppsala, Sweden) with purified wild-type antigen.

The affinity of the binding of an antibody to an antigen is defined by the terms kₐ (rate constant for the association of the antibody from the antibody/antigen complex), k_{D} (dissociation constant), and K_{D} (k_{D}/ka). In one embodiment binding or that/which specifically binds to means a binding affinity (K_{D}) of 10⁻⁸ mol/l or less, in one embodiment 10⁻⁸ M to 10⁻¹³ mol/l. Thus, an multispecific antibody according to the invention specifically binds to each antigen for which it is specific with a binding affinity (K_{D}) of 10⁻⁸ mol/l or less, e.g. with a binding affinity (K_{D}) of 10⁻⁸ to 10⁻¹³ mol/l; in one embodiment with a binding affinity (K_{D}) of 10⁻⁹ to 10⁻¹³ mol/l.

The terms "monoclonal antibody" or "monoclonal antibody composition" as used herein refer to a preparation of antibody molecules of a single amino acid composition.

The term "chimeric" antibody refers to an antibody in which a portion of the heavy and/or light chain is derived from a particular source or species, while the remainder of the heavy and/or light chain is derived from a different source or species.

The term "humanized antibody" refers to antibodies in which the framework or the CDRs have been modified to comprise the CDR of an immunoglobulin of different specificity as compared to that of the parent immunoglobulin. In one preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Other forms of humanized antibodies encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole, et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The terms "binding site" or "antigen-binding site" as used herein denotes the region(s) of an antibody molecule to which a ligand (e.g. the antigen or antigen fragment of it) actually binds and which is derived from an antibody. The antigen-binding site includes antibody heavy chain variable domains (VH) and/or antibody light chain variable domains (VL), or pairs of VH/VL.

The antigen-binding sites that specifically bind to the desired antigen can be derived a) from known antibodies specifically binding to the antigen or b) from new antibodies or antibody fragments obtained by de novo immunization methods using inter alia either the antigen protein or nucleic acid or fragments thereof or by phage display.

An antigen-binding site of an antibody according to the invention can contain six complementarity determining regions (CDRs) which contribute in varying degrees to the affinity of the binding site for antigen. There are three heavy chain variable domain CDRs (CDRH1, CDRH2 and CDRH3) and three light chain variable domain CDRs (CDRL1, CDRL2 and CDRL3). The extent of CDR and framework regions (FRs) is determined by comparison to a compiled database of amino acid sequences in which those regions have been defined according to variability among the sequences. Also included within the scope of the invention are functional antigen binding sites comprised of fewer CDRs (i.e., where binding specificity is determined by three, four or five CDRs). For example, less than a complete set of 6 CDRs may be sufficient for binding.

The "light chains" of antibodies (immunoglobulins) from any vertebrate species can be assigned to one of two distinct types, called kappa (κ) and lambda (λ), based on the amino acid sequences of their constant domains. A wild type light chain typically contains two immunoglobulin domains, usually one variable domain (VL) that is important for binding to an antigen and a constant domain (CL).

Several different types of "heavy chains" exist that define the class or isotype of an antibody. A wild type heavy chain contains a series of immunoglobulin domains, usually with one variable domain (VH) that is important for binding antigen and several constant domains (CH1, CH2, CH3, etc.).

The term "Fc domain" is used herein to define a C-terminal region of an immunoglobulin heavy chain that contains at least a portion of the constant region. For example in natural antibodies, the Fc domain is composed of two identical protein fragments, derived from the second and third constant domains of the antibody's two heavy chains in IgG, IgA and IgD isotypes; IgM and IgE Fc domains contain three heavy chain constant domains (CH domains 2-4) in each polypeptide chain.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv, scFab); and multispecific antibodies formed from antibody fragments.

As used herein, a "Fab fragment" refers to an antibody fragment comprising a light chain fragment comprising a VL domain and a constant domain of a light chain (CL), and a VH domain and a first constant domain (CH1) of a heavy chain.

The "variable domains" or "variable region" as used herein denotes each of the pair of light and heavy chains which is involved directly in binding the antibody to the antigen. The variable domain of a light chain is abbreviated as "VL" and the variable domain of a light chain is abbreviated as "VH". The variable domains of human light chains and heavy chains have the same general structure. Each variable domain comprises four framework (FR) regions, the sequences of which are widely conserved. The FR are connected by three "hypervariable regions" (or "complementarity determining regions", CDRs). CDRs on each chain are separated by such framework amino acids. Therefore, the light and heavy chains of an antibody comprise from N- to C-terminal direction the domains FR1, CDR1, FR2, CDR2, FR3, CDR3, and FR4. The FR adopt a β-sheet conformation and the CDRs may form loops connecting the β-sheet structure. The CDRs in each chain are held in their three-dimensional structure by the FR and form together with the CDRs from the other chain an "antigen binding site". Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991).

The term "constant domains" or "constant region" as used within the current application denotes the sum of the domains of an antibody other than the variable region. The constant region is not directly involved in binding of an antigen, but exhibits various effector functions.

Depending on the amino acid sequence of the constant region of their heavy chains, antibodies are divided in the classes: IgA, IgD, IgE, IgG and IgM, and several of these may are further divided into subclasses, such as IgG1, IgG2, IgG3, and IgG4, IgA1 and IgA2. The heavy chain constant regions that correspond to the different classes of antibodies are called α, δ, ε, γ and µ, respectively. The light chain constant regions (CL) which can be found in all five antibody classes are called κ (kappa) and λ (lambda). The "constant domains" as used herein are from human origin, which is from a constant heavy chain region of a human antibody of the subclass IgG1, IgG2, IgG3, or IgG4 and/or a constant light chain kappa or lambda region. Such constant domains and regions are well known in the state of the art and e.g. described by Kabat, et al., Sequences of Proteins of Immunological Interest, 5th ed., Public Health Service, National Institutes of Health, Bethesda, MD (1991).

The term "full length antibody" as used herein denotes an antibody consisting of two antibody heavy chains and two antibody light chains. A heavy chain of a full length antibody is a polypeptide consisting in N-terminal to C-terminal direction of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH1-HR-CH2-CH3; and optionally an antibody heavy chain constant domain 4 (CH4) in case of an antibody of the subclass IgE. In one embodiment the heavy chain of a full length antibody is a polypeptide consisting in N-terminal to C-terminal direction of VH, CH1, HR, CH2 and CH3. The light chain of a full length antibody is a polypeptide consisting in N-terminal to C-terminal direction of an antibody light chain variable domain (VL), and an antibody light chain constant domain (CL), abbreviated as VL-CL. The antibody light chain constant domain (CL) may be of κ (kappa) or 1 (lambda) isotype. The antibody chains are linked together via interchain disulfide bonds between the CL domain and the CH1 domain (i.e. between the light and heavy chain) and between the hinge regions of the full length antibody heavy chains. Examples of typical full length antibodies are natural antibodies like IgG (e.g. IgG 1 and IgG2), IgM, IgA, IgD, and IgE.)

The antibodies according to the invention can be from a single species e.g. human, or they can be chimerized or humanized antibodies. The full length antibodies forming the core antibody of the antibody according to the invention comprise two antigen binding sites each formed by a pair of VH and VL.

The full length antibody forming the core antibody of the antibody according to the invention may either comprise two Fab fragments of the same antigen binding specificity (first aspect, see Figures 1 A-D) or may comprise two Fab fragments of different antigen binding specificity (second aspect, Figures 2 A-D).

In case the core antibody comprises two Fab fragments of the same antigen binding specificity, the full length antibody comprises two identical Fab fragments. Both Fab fragments may either (a) comprise the same domain crossover of VH and VL domains, resulting in a light chain of a VH-CL domain arrangement and a heavy chain comprising a VL-CH1 domain arrangement (from N- to C-terminal direction) (see Figures 1B and ID), or (b) both Fab fragments do not comprise a domain crossover, thereby comprising a light chain of a VL-CL domain arrangement and a heavy chain comprising a VH-CH1 domain arrangement (from N- to C-terminal direction) (see Figures 1A and 1C).

In case the core antibody comprises two Fab fragments of different antigen binding specificity, the full length antibody comprises two Fab fragments, wherein exactly one of the Fab fragments includes a domain crossover of VH and VL domains, resulting in a light chain of a VH-CL domain arrangement and a heavy chain comprising a VL-CH1 domain arrangement (from N- to C-terminal direction) (see Figures 2A-D, binding arm binding to antigen 2). In one preferred embodiment, the other Fab fragment does not comprise a domain crossover, thereby comprising a light chain of a VL-CL domain arrangement and a heavy chain comprising a VH-CH1 domain arrangement (from N- to C-terminal direction). The "C-terminus of the heavy or light chain" of said full length antibody denotes the last amino acid at the C-terminus of said heavy or light chain.

The term "domain crossover" within the Fab fragment as referred to herein means that in the antibody binding arm (i.e. the Fab fragment) deviating from the natural domain architecture of antibodies at least one heavy chain domain was substituted by its corresponding light chain domain and vice versa. There are three general types of domain crossovers, (i) the crossover of the CH1 and the CL domains, which leads to crossover light chains of a VL-CH1 structure and crossover heavy chains comprising a VH-CL structure, (ii) the crossover of the VH and the VL domains, which leads to crossover light chains of a VH-CL structure and crossover heavy chains comprising a VL-CH1 structure, and (iii) the crossover of the <VL-CL> polypeptide and the <VH-CH1> polypeptide ("Fab crossover"), which leads to crossover light chains of a VH-CH1 structure and crossover heavy chains comprising a VL-CL structure (all aforementioned domain arrangements are indicated in N-terminal to C-terminal direction).

Within the terms of the present invention "replaced by each other" with respect to corresponding heavy and light chain domains refers to the aforementioned domain crossover strategies. As such, when CH1 and CL domains are "replaced by each other" it is referred to the domain crossover mentioned under item (i) and the resulting heavy and light chain domain architecture. Accordingly, when VH and VL are "replaced by each other" it is referred to the domain crossover mentioned under item (ii); and when the CH1 and CL domains are "replaced by each other" and the VH1 and VL domains are "replaced by each other" it is referred to the domain crossover mentioned under item (iii). Bispecific antibodies including domain crossovers are disclosed, e.g. in WO 2009/080251, WO 2009/080252, WO 2009/080253, WO 2009/080254 and Schaefer, W. et al, PNAS, 108 (2011) 11187-1191.

The antibody according to the invention essentially comprises Fab fragments including a crossover of the VH and the VL domains as mentioned under item (ii). According to the general concept of the invention, the Fab fragments specifically binding to the same antigen(s) are constructed of the same domain arrangement. Hence, in case more than one Fab fragments including a crossover of the VH and the VL domains are present in the antibody according to the invention, said Fab fragments specifically bind to the same antigen(s). Within the antibody according to the invention, the Fab fragments specifically binding to a different antigen than the Fab fragment(s) comprising the domain crossover of the VH and the VL domains do not comprise said same domain crossover.

An "additional Fab fragment" that is comprised in an antibody according to the invention refers to a further Fab fragment that is fused to an N-terminus or C-terminus of a heavy chain of said full length antibody, in one embodiment via a peptide connector.

The antibody according to the invention is arranged such that either additional Fab fragments fused to the heavy chains of the full length antibody specifically bind to a second antigen (in this case the core antibody comprises two Fab fragments of the same antigen binding specificity, see Figures 1A-D), or the additional Fab fragment fused to a heavy chain specifically binds to the same antigen as the heavy chain on which it is arranged (in this case the core antibody comprises two Fab fragments of different antigen binding specificities, see Figures 2A-D)

The additional Fab fragments are fused via the N-terminal heavy chain part (VH domain) or their light chain part (VL domain) to the C-termini of the heavy chains of the core antibody; or via the C-terminal heavy chain part (CH1 domain) or their light chain part (CL domain) to the N-termini of the heavy chains of the core antibody. In one embodiment, the additional Fab fragments are fused via the heavy chain part to the C- or N termini of the heavy chains of the core antibody.

The term "peptide connector" as used within the invention denotes a peptide with amino acid sequences, which is preferably of synthetic origin. Within an antibody according to the invention, peptide connectors are used to fuse the additional Fab fragments to the C-or N-terminus of the heavy chains of the core antibody. In one embodiment said peptide connectors are peptides with an amino acid sequence with a length of at least 5 amino acids, in another embodiment with a length of 5 to 100 amino acids, in yet another embodiment of 10 to 50 amino acids. In one embodiment said peptide connector is

(GₓS)ₙ or (GₓS)ₙGₘ

with G = glycine, S = serine, and
x = 3, n= 3, 4, 5 or 6, m= 0, 1, 2 or 3; or
x = 4, n= 2, 3, 4 or 5, m= 0, 1, 2 or 3.

In one embodiment x = 4 and n= 2 or 3, in another embodiment x = 4, n= 2. In one embodiment said peptide connector is (G₄S)₂.

The term "tertiary structure" as used herein refers to the geometric shape of the antibody according to the invention. The tertiary structure comprises a polypeptide chain backbone comprising the antibody domains, while amino acid side chains interact and bond in a number of ways.

The term "amino acid" as used herein denotes an organic molecule possessing an amino moiety located at α-position to a carboxylic group. Examples of amino acids include: arginine, glycine, ornithine, lysine, histidine, glutamic acid, asparagic acid, isoleucine, leucine, alanine, phenylalanine, tyrosine, tryptophane, methionine, serine, and proline. The amino acid employed is optionally in each case the L-form. The term "positively charged" or "negatively charged" amino acid refers to the amino acid side-chain charge at pH 7.4. Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table - Amino acids with specific properties**

| **Amino Acid** | **3-Letter** | **1-Letter** | **Side-chain polarity** | **Side-chain charge (pH 7.4)** |
|---|---|---|---|---|
| Alanine | Ala | A | nonpolar | neutral |
| Arginine | Arg | R | basic polar | positive |
| Asparagine | Asn | N | polar | neutral |
| Aspartic acid | Asp | D | acidic polar | negative |
| Cysteine | Cys | C | nonpolar | neutral |
| Glutamic acid | Glu | E | acidic polar | negative |
| Glutamine | Gln | Q | polar | neutral |
| Glycine | Gly | G | nonpolar | neutral |
| Histidine | His | H | basic polar | positive (10%) |
| | | | | neutral (90%) |
| Isoleucine | Ile | I | nonpolar | neutral |
| Leucine | Leu | L | nonpolar | neutral |
| Lysine | Lys | K | basic polar | positive |
| Methionine | Met | M | nonpolar | neutral |
| Phenylalanine | Phe | F | nonpolar | neutral |
| Proline | Pro | P | nonpolar | neutral |
| Serine | Ser | S | polar | neutral |
| Threonine | Thr | T | polar | neutral |
| Tryptophan | Trp | W | nonpolar | neutral |
| Tyrosine | Tyr | Y | polar | neutral |
| Valine | Val | V | nonpolar | neutral |

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991), which is referred to herein as "numbering according to Kabat et al.". In particular, for variable domains and for the light chain constant domain CL of kappa and lambda isotype, the Kabat numbering system (see pages 647-660) of Kabat, et al., Sequences of Proteins of Immunological Interest, 5th ed., Public Health Service, National Institutes of Health, Bethesda, MD (1991) is used and is herein referred to as "numbering according to Kabat" and, nonwithstanding this, for the constant heavy chain domains (CH1, Hinge, CH2 and CH3) the Kabat EU index numbering system (see pages 661-723) is used and is herein referred to as "numbering according to EU index of Kabat".

Amino acid substitutions (or mutations) within the polypeptide chains of the multispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and antigen binding, but may further improve the yield of the recombinant production, protein stability or facilitate the purification. In certain embodiments, antibody variants having one or more conservative amino acid substitutions are provided.

The antibody according to the invention is produced by recombinant means. Methods for recombinant production of antibodies are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective (modified) light and heavy chains are inserted into expression vectors by standard methods. Expression is performed in appropriate prokaryotic or eukaryotic host cells, like CHO cells, NS0 cells, SP2/0 cells, HEK293 cells, COS cells, PER.C6 cells, yeast, or E. coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

"Polynucleotide" or "nucleic acid" as used interchangeably herein, refers to polymers of nucleotides of any length, and includes DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a vector are capable of directing the expression of nucleic acids to which they are operatively linked. When the expression vector is introduced into an appropriate host cell, it can be transcribed and translated into a polypeptide. When transforming host cells in methods according to the invention, "expression vectors" are used; thereby the term "vector" in connection with transformation of host cells as described herein means "expression vector". An "expression system" usually refers to a suitable host cell comprised of an expression vector that can function to yield a desired expression product.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

The term "transformation" as used herein refers to the process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, F.N, et al., PNAS 69 (1972) 7110 et seq.

The term "host cell" as used in the current application denotes any kind of cellular system which can be engineered to generate the antibodies according to the current invention. This term however excludes human cells within a human being.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

Antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a cleaved variant heavy chain). This may be the case where the final two C-terminal amino acids of the heavy chain are glycine (G446) and lysine (K447, numberings according to Kabat EU index).

Therefore, amino acid sequences of heavy chains including CH3 domains with a free C-terminus are denoted herein without C-terminal glycine-lysine dipeptide if not indicated otherwise. In contrast, amino acid sequences of heavy chains comprising an additional Fab fragment fused to its C-terminus are denoted herein including the C-terminal glycine-lysine dipeptide.

In one embodiment of the invention an antibody comprising a heavy chain including a CH3 domain with a free C-terminus as specified herein, comprises an additional C-terminal glycine-lysine dipeptide (G446 and K447, numbering according to EU index of Kabat). In one embodiment of the invention an antibody comprising a heavy chain including a CH3 domain with a free C-terminus as specified herein, comprises an additional C-terminal glycine residue (G446, numbering according to EU index of Kabat).

Compositions of the invention, such as the pharmaceutical compositions described herein, comprise a population of antibodies of the invention. The population of antibodies may comprise antibodies having a full-length heavy chain and antibodies having a cleaved variant heavy chain. In one embodiment, the population of antibodies consists of a mixture of antibodies having a full-length heavy chain and antibodies having a cleaved variant heavy chain, wherein at least 50%, at least 60%, at least 70%, at least 80% or at least 90% of the antibodies have a cleaved variant heavy chain.

In one embodiment of the invention a composition comprising a population of antibodies of the invention comprises an antibody comprising a heavy chain including a CH3 domain with a free C-terminus as specified herein with an additional C-terminal glycine-lysine dipeptide (G446 and K447, numbering according to EU index of Kabat). In one embodiment of the invention a composition comprising a population of antibodies of the invention comprises an antibody comprising a heavy chain including a CH3 domain with a free C-terminus as specified herein with an additional C-terminal glycine residue (G446, numbering according to EU index of Kabat).

In one embodiment of the invention such a composition comprises a population of antibodies comprised of antibodies comprising a heavy chain including a CH3 domain as specified herein; antibodies comprising a heavy chain including a CH3 domain with a free C-terminus as specified herein with an additional C-terminal glycine residue (G446, numbering according to EU index of Kabat); and antibodies comprising a heavy chain including a CH3 domain with a free C-terminus as specified herein with an additional C-terminal glycine-lysine dipeptide (G446 and K447, numbering according to EU index of Kabat).

Purification of antibodies (recovering the antibodies from the host cell culture) is performed in order to eliminate cellular components or other contaminants, e.g. other cellular nucleic acids or proteins, by standard techniques, including alkaline/SDS treatment, CsCI banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. To administer an antibody according to the invention by certain routes of administration, it may be necessary to coat the antibody with, or co-administer the antibody with, a material to prevent its inactivation. For example, the antibody may be administered to a subject in an appropriate carrier, for example, liposomes, or a diluent. Pharmaceutically acceptable diluents include saline and aqueous buffer solutions.

A "pharmaceutically acceptable carrier" refers to an ingredient in a pharmaceutical composition, other than an active ingredient, which is nontoxic to a subject. Pharmaceutically acceptable carriers includes any and all solvents, dispersion media, coatings, antibacterial and antifungal agents, isotonic and absorption delaying agents, and the like that are physiologically compatible. In one preferred embodiment, the carrier is suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, in one embodiment the carrier is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

The term "package insert" is used to refer to instructions customarily included in commercial packages of therapeutic products, that contain information about the indications, usage, dosage, administration, combination therapy, contraindications and/or warnings concerning the use of such therapeutic products.

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

"Human VEGF" as used herein refers to human vascular endothelial growth factor (VEGF/VEGF-A) which is described in e.g. Leung, D.W., et al., Science 246 (1989) 1306-9; Keck, P.J., et al., Science 246 (1989) 1309-12 and Connolly, D.T., et al., J. Biol. Chem. 264 (1989) 20017-24. VEGF is involved in the regulation of normal and abnormal angiogenesis and neovascularization associated with tumors and intraocular disorders (Ferrara, N., et al., Endocr. Rev. 18 (1997) 4-25; Berkman, R.A., et al., J. Clin. Invest. 91 (1993) 153-159; Brown, L.F., et al., Human Pathol. 26 (1995) 86-91; Brown, L.F., et al., Cancer Res. 53 (1993) 4727-4735; Mattern, J., et al., Brit. J. Cancer. 73 (1996) 931-934; and Dvorak, H., et al., Am. J. Pathol. 146 (1995) 1029-1039). VEGF is a homodimeric glycoprotein that has been isolated from several sources. VEGF shows highly specific mitogenic activity for endothelial cells.

Human "ANG-2" as used herein refers to human angiopoietin-2 (ANG-2) (alternatively abbreviated with ANGPT2 or ANG2) which is described in Maisonpierre, P.C., et al, Science 277 (1997) 55-60 and Cheung, A.H., et al., Genomics 48 (1998) 389-91. The angiopoietins-1 and -2 were discovered as ligands for the Ties, a family of tyrosine kinases that is selectively expressed within the vascular endothelium (Yancopoulos, G.D., et al., Nature 407 (2000) 242-48). There are now four definitive members of the angiopoietin family. Angiopoietin-3 and -4 (Ang-3 and Ang-4) may represent widely diverged counterparts of the same gene locus in mouse and man (Kim, I., et al., FEBS Let, 443 (1999) 353-56; Kim, I., et al., J Biol Chem 274 (1999) 26523-28).

The term "PD1", also known as Programmed cell death protein 1, is a type I membrane protein of 288 amino acids that was first described in 1992 (Ishida et al., EMBO J., 11 (1992), 3887-3895). PD-1 is a member of the extended CD28/CTLA-4 family of T cell regulators and has two ligands, PD-L1 (B7-H1, CD274) and PD-L2 (B7-DC, CD273). The protein's structure includes an extracellular IgV domain followed by a transmembrane region and an intracellular tail. The intracellular tail contains two phosphorylation sites located in an immunoreceptor tyrosine-based inhibitory motif and an immunoreceptor tyrosine-based switch motif, which suggests that PD-1 negatively regulates TCR signals. This is consistent with binding of SHP-1 and SHP-2 phosphatases to the cytoplasmic tail of PD-1 upon ligand binding. While PD-1 is not expressed on naive T cells, it is upregulated following T cell receptor (TCR)-mediated activation and is observed on both activated and exhausted T cells (Agata et al., Int. Immunology 8 (1996), 765-772). These exhausted T-cells have a dysfunctional phenotype and are unable to respond appropriately. Although PD-1 has a relatively wide expression pattern its most important role is likely as a coinhibitory receptor on T cells (Chinai et al, Trends in Pharmacological Sciences 36 (2015), 587-595). Current therapeutic approaches thus focus on blocking the interaction of PD-1 with its ligands to enhance T cell response. The terms "Programmed Death 1," "Programmed Cell Death 1," "Protein PD-1," "PD-1," PD1," "PDCD1," "hPD-1" and "hPD-I" can be used interchangeably, and include variants, isoforms, species homologs of human PD-1, and analogs having at least one common epitope with PD-1. The amino acid sequence of human PD1 is shown in UniProt (www.uniprot.org) accession no. Q15116.

The term "TIM3", the abbreviation for "T cell Immunoglobulin- and Mucin domain-containing molecule 3", also known as HAVCR2, KIM-3, TIMD3, and FLJ14428, refers to a T helper cell type 1-specific cell surface protein that regulates macrophage activation and the severity of inflammatory conditions. TIM3 is also associated with cancer, in particular, with cancer stem cells. The nucleotide and protein sequences of TIM3 are known for many species. For example, the human amino acid sequence can be found under Uniprot accession number Q8TDQ0. The human protein is characterized by an extracellular domain comprising an Ig like domain and a mucin domain (further comprising O-linked and N-linked glycosylation sites) comprising approximately amino acids 22-202, a transmembrane domain (amino acids 203-223), and an intracellular (cytoplasmic) domain (amino acids 224-301). For the human TIM3 protein the extracellular domain comprises approximately amino acids 22-202, the transmembrane domain comprises approximately amino acids 203-223, and the cytoplasmic domain comprises approximately amino acids 224-301. The term "Tim-3" includes variants, isoforms, species homologs of human Tim-3, and analogs having at least one common epitope with Tim-3.

The term "death receptor 5 (DR5)", as used herein, refers to any native DR5 from any vertebrate source, including mammals such as primates (e.g. humans) and rodents (e.g., mice and rats), unless otherwise indicated. The term encompasses "full-length," unprocessed DR5 as well as any form of DR5 that results from processing in the cell. The term also encompasses naturally occurring variants of DR5, e.g., splice variants or allelic variants.

The term "Fibroblast activation protein (FAP)", as used herein, refers to any native FAP 20 from any vertebrate source, including mammals such as primates (e.g. humans) and rodents (e.g., mice and rats), unless otherwise indicated. The term encompasses "full-length," unprocessed FAP as well as any form of FAP that results from processing in the cell. The term also encompasses naturally occurring variants of FAP, e.g., splice variants or allelic variants. Preferably, an anti-FAP antibody of the invention binds to the extracellular domain of FAP.

### 2. Detailed description of the embodiments of the invention

### I. Multispecific antibody

Multispecific antibodies with a domain replacement/exchange in one binding arm (CrossMabVH-VL) are described in detail in WO2009/080252 and Schaefer, W. et al, PNAS, 108 (2011) 11187-1191 (which are incorporated as reference herein). They clearly reduce the byproducts caused by the mismatch of a light chain against a first antigen with the wrong heavy chain against the second antigen (compared to approaches without such domain exchange). However their preparation is not completely free of side products. The main side product is based on a Bence-Jones-type interaction (see also Schaefer, W. et al, PNAS, 108 (2011) 11187-1191; in Fig. S1I of the Supplement).

Herein is disclosed an approach for further improving such antibodies, e.g. by further reduction of side products and by reducing the aggregation behaviour to improve the yield of such multispecific antibodies by the introduction of substitutions of charged amino acids with the opposite charge at specific amino acid positions in the CH1 and CL domains.

Hence, the present invention relates to a tetravalent multispecific antibody with a domain crossover within Fab fragments of one binding specificity. Within the antibody one set of Fab fragments of the same specificity comprise the following amino acid substitutions in the constant light chain domain (CL) and the constant heavy chain domain 1 (CH1): (i) the amino acid at position 124 in the CL domain is substituted by K, R or H (numbering according to Kabat), and (ii) the amino acid at position 147 or 213 in the CH1 domain is substituted by E or D (numbering according to Kabat EU index), while the other Fab fragments of a different binding specificity do not comprise said amino acid substitutions.

In a first aspect, the present invention relates to a tetravalent multispecific antibody, comprising
a) one (in one embodiment exactly one) core antibody formed by a full length antibody, the full length antibody comprising two Fab fragments specifically binding to a first antigen, and
b) two (in one embodiment exactly two) additional Fab fragments, wherein said additional Fab fragments are fused both either at the C-termini or the N-termini of the heavy chains of the core antibody,
   wherein the additional Fab fragments specifically bind to a second antigen,
   i) wherein either
      - the Fab fragments specifically binding to the first antigen, or
      - the Fab fragments specifically binding to the second antigen comprise a domain crossover such that the variable heavy chain domain (VH) and the variable light chain domain (VL) are replaced by each other (in one embodiment only the variable heavy chain domain (VH) and the variable light chain domain (VL) are replaced by each other), and
   ii) wherein either
      - the Fab fragments specifically binding to the first antigen, or
      - the one or two Fab fragments specifically binding to the second antigen comprise the following amino acid substitutions in the constant light chain domain (CL) and the constant heavy chain domain 1 (CH1):
      - the amino acid at position 124 in the CL domain is substituted by K, R or H (numbering according to Kabat), and
      - the amino acid at position 147 or 213 in the CH1 domain is substituted by E or D (numbering according to Kabat EU index).

A schematic drawing of a multispecific antibody according to this first aspect is depicted in Figures 1A-D. According to this aspect, the core antibody is a full length antibody comprising two Fab fragments, which bind to the same antigen (e.g. a first antigen). In one embodiment of this aspect, the core antibody is a monospecific antibody. In one embodiment of this aspect, the Fab fragments of the core antibody do not comprise a domain crossover of the VH and the VL domains.

In a second aspect, the present invention relates to a tetravalent multispecific antibody, comprising
a) one (in one embodiment exactly one) core antibody formed by a full length antibody, the full length antibody comprising two Fab fragments specifically binding to a first antigen and a second antigen, respectively, and
b) two (in one embodiment exactly two) additional Fab fragments, wherein said additional Fab fragments are fused both either at the C-termini or the N-termini of the heavy chains of the core antibody,
   wherein the additional Fab fragment fused to a heavy chain exhibits the same antigen binding specificity than the Fab fragment of the core antibody arranged
   on said heavy chain,
   i) wherein either
      - the Fab fragments specifically binding to the first antigen, or
      - the Fab fragments specifically binding to the second antigen comprise a domain crossover such that the variable heavy chain domain (VH) and the variable light chain domain (VL) are replaced by each other (in one embodiment only the variable heavy chain domain (VH) and the variable light chain domain (VL) are replaced by each other), and
   ii) wherein either
      - the Fab fragments specifically binding to the first antigen, or
      - the Fab fragments specifically binding to the second antigen comprise the following amino acid substitutions in the constant light chain domain (CL) and the constant heavy chain domain 1 (CH1):
      - the amino acid at position 124 in the CL domain is substituted by K, R or H (numbering according to Kabat), and
      - the amino acid at position 147 or 213 in the CH1 domain is substituted by E or D (numbering according to Kabat EU index).

A schematic drawing of a multispecific antibody according to this second aspect is depicted in Figures 2A-D. According to this aspect, the core antibody is a full length antibody comprising two Fab fragments, which bind to different antigens (e.g. a first Fab fragment specifically binding to a first antigen, and a second Fab fragment specifically binding to a second antigen). As indicated in Figures 2A-D, within a multispecific antibody of said second aspect one heavy chain that includes one additional Fab fragment carries two Fab fragments of the same antigen binding specificity (i.e. one of the Fab fragments is the Fab fragment of the core antibody and the other Fab fragment is the additional Fab fragment that is fused to the heavy chain). In one embodiment of this second aspect, the core antibody is a bispecific antibody.

Hence, the invention is based on a general antibody domain arrangement for multispecific antibodies, wherein a tetravalent multispecific antibody is formed by fusion of two additional Fab fragments to a core antibody. The core antibody is a full length antibody, which may either comprise two Fab fragments of the same antigen binding specificity (first aspect, see Figures 1A-D) or which may comprise two Fab fragments of different antigen binding specificity (second aspect, Figures 2A-D). The multispecific antibodies according to both aspects as mentioned above are equally applicable in combination with the further embodiments listed below.

A domain crossover of the VH and VL domains are present in the Fab fragments specifically binding to the same (e.g. a first) antigen. Thereby, the light chain domain arrangement of said Fab fragments differs from the domain arrangement of the Fab fragments specifically binding to another (a different, e.g. a second) antigen, according to the CrossMab technology known from the prior art. In addition, one set of Fab fragments specifically binding to the same antigen (i.e. independent from the domain crossover, e.g. the Fab fragments binding to the "first" or the "second" antigen as exemplified before, however not both sets of Fab fragments including the same substitutions), included amino acid substitutions within the CH1 / CL interface, thereby introducing amino acids of opposite charges in the distinct heavy chain and light chain pair, which supports dimerization of the correct heavy chain and light chain pair.

### Additional amino acid substitutions in the CH1/CL interface

In one embodiment of an antibody according to the invention, the Fab fragments that do not comprise the domain crossover of i) comprise the amino acid substitutions of ii).

In one embodiment of an antibody according to the invention, in the Fab fragments comprising the amino acid substitutions of ii)
- the amino acids at positions 147 and 213 in the CH1 domain are substituted independently from each other by E or D (numbering according to Kabat EU index).

In one embodiment of an antibody according to the invention, in the Fab fragments comprising the amino acid substitutions of ii)
- the amino acids at position 123 and 124 in the CL domain are substituted independently from each other by K, R or H (numbering according to Kabat).

In one preferred embodiment of an antibody according to the invention, in the Fab fragments comprising the amino acid substitutions of ii)
- the amino acids at positions 147 and 213 in the CH1 domain are substituted independently from each other by E or D (numbering according to Kabat EU index); and
- the amino acids at position 123 and 124 in the CL domain are substituted independently from each other by K, R or H (numbering according to Kabat).

In one embodiment of an antibody according to the invention, the Fab fragments that do not comprise the amino acid substitutions as defined under ii) comprise the following amino acid substitution in the CL domain
- the amino acid at position 124 is substituted by E or D.

Hence, in one embodiment of an antibody according to the invention
ii) either
   - the Fab fragments specifically binding to the first antigen, or
   - the Fab fragments specifically binding to the second antigen comprise the following amino acid substitutions in the constant light chain domain (CL) and the constant heavy chain domain 1 (CH1):
   - the amino acid at position 124 in the CL domain is substituted by K, R or H (numbering according to Kabat), and
   - the amino acid at position 147 or 213 in the CH1 domain is substituted by E or D (numbering according to Kabat EU index), and
iii) the one or two Fab fragments that do not comprise the amino acid substitutions as defined under ii) comprise the following amino acid substitution in the CL domain:
   - the amino acid at position 124 is substituted by E or D.

In one embodiment of an antibody according to the invention, in the Fab fragments comprising the amino acid substitutions of ii)
- the amino acids at positions 147 and 213 in the CH1 domain are substituted independently from each other by E or D (numbering according to Kabat EU index), and
   iii) the Fab fragments that do not comprise the amino acid substitutions as defined under ii) comprise the following amino acid substitution in the CL domain:
      the amino acid at position 124 is substituted by E or D.

In one embodiment of an antibody according to the invention, in the Fab fragments comprising the amino acid substitutions of ii)
- the amino acids at position 123 and 124 in the CL domain are substituted independently from each other by K, R or H (numbering according to Kabat), and
   iii) the Fab fragments that do not comprise the amino acid substitutions as defined under ii) comprise the following amino acid substitution in the CL domain:
      the amino acid at position 124 is substituted by E or D.

In one embodiment of an antibody according to the invention, in the Fab fragments comprising the amino acid substitutions of ii)
- the amino acids at positions 147 and 213 in the CH1 domain are substituted independently from each other by E or D (numbering according to Kabat EU index); and
- the amino acids at position 123 and 124 in the CL domain are substituted independently from each other by K, R or H (numbering according to Kabat); and
   iii) the Fab fragments that do not comprise the amino acid substitutions as defined under ii) comprise the following amino acid substitution in the CL domain:
- the amino acid at position 124 is substituted by E or D.

In one embodiment of an antibody according to the invention, the Fab fragments comprising the amino acid substitutions of ii) comprise a light chain constant domain CL of kappa isotype. In one embodiment of an antibody according to the invention, the Fab fragments comprising the amino acid substitutions of ii) comprise a light chain constant domain CL of lambda isotype.

In one embodiment of an antibody according to the invention, the additional Fab fragments are fused to the C-terminus of the heavy chains of the core antibody.

In one embodiment of an antibody according to the invention, the additional Fab fragments are fused to the N-terminus of the heavy chains of the core antibody.

### Heterodimerization of Fc region

In one embodiment of the invention, the antibody according to the invention comprises two constant heavy chain domains 3 (CH3) which are altered to promote heterodimerization according to different approaches known in the prior art (e.g. by knobs-into-holes modifications and/or introduction of additional cysteine bridges within the CH3/CH3 interface).

In one aspect of an antibody according to the invention, the multispecific antibody comprises two constant heavy chain domains 3 (CH3), which are altered to promote heterodimerization by generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains (corresponding to the general approach of the knobs-into-holes technology).

In one preferred embodiment of this aspect, said amino acid residue having a larger side chain volume than the original amino acid residue is selected from R, F, Y and W. In one preferred embodiment of this aspect, said amino acid residue having a smaller side chain volume than the original amino acid residue is selected from A, S, T and V. In one preferred embodiment of this aspect, one of the CH3 domains comprises a T366W mutation and the other one of the CH3 domains comprises T366S, L368A and 407V mutations (numberings according to EU index of Kabat).

In one aspect of an antibody according to the invention, the multispecific antibody comprises two CH3 domains which are altered to promote heterodimerization by substituting at least one original amino acid residue in one of the CH3 domains by a positively charged amino acid, and substituting at least one original amino acid residue in the other one of the CH3 domains by a negatively charged amino acid.

In one preferred embodiment of this aspect, said positively charged amino acid is selected from K, R and H. In one preferred embodiment of this aspect, said negatively charged amino acid is selected from E or D. In one preferred embodiment of this aspect, one of the CH3 domains comprises R409D and K370E mutations and the other one of the CH3 domains comprises D399K and E357K mutations (numberings according to EU index of Kabat).

In one aspect of an antibody according to the invention, the multispecific antibody comprises two CH3 domains, which are altered to promote heterodimerization by introduction of at least one cysteine residue in each CH3 domain such that a disulfide bond is formed between the CH3 domains.

In one preferred embodiment of this aspect, one of the CH3 domains comprises an E356C or an S354C mutation, and the other one of the CH3 domains comprises a Y349C mutation (numberings according to EU index of Kabat). In one preferred embodiment of this aspect, one of the CH3 domains comprises an S354C mutation, and the other one of the CH3 domains comprises a Y349C mutation (numberings according to EU index of Kabat).

In one embodiment of an antibody according to the invention, the multispecific antibody comprises two CH3 domains, which are altered to promote heterodimerization, wherein one of the CH3 domains comprises a T366W mutation and the other one of the CH3 domains comprises T366S, L368A and 407V mutations (numberings according to EU index of Kabat); and wherein any one of the aforementioned CH3 domains comprises an E356C or an S354C mutation, and the other one of the aforementioned CH3 domains comprises a Y349C mutation (numberings according to EU index of Kabat). In one embodiment of an antibody according to the invention, the multispecific antibody comprises two CH3 domains, which are altered to promote heterodimerization, wherein one of the CH3 domains comprises a T366W mutation and the other one of the CH3 domains comprises T366S, L368A and 407V mutations (numberings according to EU index of Kabat); and wherein any one of the aforementioned CH3 domains comprises an S354C mutation, and the other one of the aforementioned CH3 domains comprises a Y349C mutation (numberings according to EU index of Kabat).

### Peptide connector

In one embodiment of an antibody according to the invention, the one or two additional Fab fragments of b) are fused via a peptide connector to the heavy chains of the core antibody.

In one embodiment of an antibody according to the invention, the peptide connector has a length of at least 5 amino acids. In one embodiment of an antibody according to the invention, the peptide connector has a length of 5 - 100 amino acids. In one embodiment of an antibody according to the invention, the peptide connector has a length of 10 - 50 amino acids.

In one embodiment of an antibody according to the invention, the peptide connector is a glycine-serine linker.

In one embodiment of an antibody according to the invention, the peptide connector is

(GₓS)ₙ or (GₓS)ₙGₘ

with G = glycine, S = serine, and
x = 3, n= 3, 4, 5 or 6, m= 0, 1, 2 or 3; or
x = 4, n= 2, 3, 4 or 5, m= 0, 1, 2 or 3.

In one embodiment x = 4 and n= 2 or 3, in another embodiment x = 4, n= 2. In one embodiment said peptide connector is (G₄S)₂.

### Antibody isotypes

In one embodiment the core antibody of an antibody according to the invention is a full length IgG antibody. In one embodiment the constant domains of an antibody according to the invention are of human IgG1 or IgG4 subclass. In one embodiment, the CH3 domain is derived from a human IgG1 antibody. In one embodiment, the multispecific antibody is devoid of a CH4 domain.

### Antibody variants

In one embodiment of the antibody according to the invention, either
- the Fab fragments specifically binding to the first antigen, or
- the Fab fragments specifically binding to the second antigen
comprise a domain crossover such that the VH domain and the VL domain are replaced by each other, and wherein the other Fab fragments that do not comprise said domain crossover comprise a light chain including a CL and a VL domain and a heavy chain including a CH1 and a VH domain.

In one embodiment of the antibody according to the invention, the additional Fab fragments of b) are fused to the C-termini of the heavy chains of the core antibody.

In one embodiment of the invention the multispecific antibody is an isolated antibody.

In one embodiment of an antibody according to the invention, the antibody is bispecific, trispecific or tetraspecific. In one embodiment of an antibody according to the invention, the antibody is bispecific.

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics.

### a) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright, A. and Morrison, S.L., TIBTECH 15 (1997) 26-32. The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A. et al., J. Mol. Biol. 336 (2004) 1239-1249; Yamane-Ohnuki, N. et al., Biotech. Bioeng. 87 (2004) 614-622. Examples of cell lines capable of producing defucosylated antibodies include Lec13 CHO cells deficient in protein fucosylation (Ripka, J. et al., Arch. Biochem. Biophys. 249 (1986) 533-545; US 2003/0157108; and WO 2004/056312, especially at Example 11), and knockout cell lines, such as alpha-1,6-fucosyltransferase gene, FUT8, knockout CHO cells (see, e.g., Yamane-Ohnuki, N. et al., Biotech. Bioeng. 87 (2004) 614-622; Kanda, Y. et al., Biotechnol. Bioeng. 94 (2006) 680-688; and WO 2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; and US 2005/0123546. Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

### b) Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express FcγRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of in vitro assays to assess ADCC activity of a molecule of interest is described in U.S. Patent No. 5,500,362 (see, e.g. Hellstrom, I. et al., Proc. Natl. Acad. Sci. USA 83 (1986) 7059-7063; and Hellstrom, I. et al., Proc. Natl. Acad. Sci. USA 82 (1985) 1499-1502); U.S. Patent No. 5,821,337 (see Bruggemann, M. et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes, R. et al., Proc. Natl. Acad. Sci. USA 95 (1998) 652-656. C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro, H. et al., J. Immunol. Methods 202 (1996) 163-171; Cragg, M.S. et al., Blood 101 (2003) 1045-1052; and Cragg, M.S. and M.J. Glennie, Blood 103 (2004) 2738-2743). FcRn binding and in vivo clearance/half life determinations can also be performed using methods known in the art (see, e.g., Petkova, S.B. et al., Int. Immunol. 18 (2006: 1759-1769).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields, R.L. et al., J. Biol. Chem. 276 (2001) 6591-6604)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either improved or diminished) C1q binding and/or Complement Dependent Cytotoxicity (CDC), e.g., as described in US Patent No. 6,194,551, WO 99/51642, and Idusogie, E.E. et al., J. Immunol. 164 (2000) 4178-4184.

Antibodies with increased half lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan, A.R. and Winter, G., Nature 322 (1988) 738-740; US 5,648,260; US 5,624,821; and WO 94/29351 concerning other examples of Fc region variants.

### c) Antibody Derivatives

In certain embodiments, an antibody provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

### Immunoconjugate

In one embodiment of the invention, an immunoconjugate comprising the multispecific antibody according to the invention, is provided. In one embodiment, such immunoconjugate comprises the multispecific antibody coupled to a cytotoxic agent.

### II. Recombinant method

Antibodies may be produced using recombinant methods and compositions, e.g., as described in U.S. Patent No. 4,816,567.

In one embodiment, isolated nucleic acid encoding a multispecific antibody according to the invention is provided. Such nucleic acid may encode an amino acid sequence comprising the VL and/or an amino acid sequence comprising the VH of the antibody (e.g., the light and/or heavy chains of the antibody).

In a further embodiment, one or more vectors (e.g., expression vectors) comprising such nucleic acid are provided.

In a further embodiment, a host cell comprising such nucleic acid is provided. In a further embodiment, a host cell comprising such vector (e.g. such expression vector) is provided.

In one such embodiment, a host cell comprises (e.g., has been transformed with): a first vector comprising a nucleic acid that encodes an amino acid sequence of the light chain of the core antibody of the multispecific antibody, a second vector comprising a nucleic acid that encodes an amino acid sequence of the heavy chain of the core antibody of the multispecific antibody, to which an additional Fab fragment is fused, optionally a third vector comprising a nucleic acid that encodes an amino acid sequence of the heavy chain of the core antibody of the multispecific antibody to which no additional Fab fragment is fused (in case the multispecific antibody is a trivalent antibody), a fourth vector comprising a nucleic acid that encodes an amino acid sequence of the light chain of the one or two additional Fab fragments.

In one such embodiment, a host cell comprises (e.g., has been transformed with): a first vector comprising a nucleic acid that encodes an amino acid sequence of the first light chain of the core antibody of the multispecific antibody, a second vector comprising a nucleic acid that encodes an amino acid sequence of the second light chain of the core antibody of the multispecific antibody, a third vector comprising a nucleic acid that encodes an amino acid sequence of the first heavy chain of the core antibody of the multispecific antibody, and a fourth vector comprising a nucleic acid that encodes an amino acid sequence of the first light chain of the core antibody of the multispecific antibody.

In one embodiment, the host cell is eukaryotic, e.g. a Chinese Hamster Ovary (CHO) cell or lymphoid cell (e.g., Y0, NS0, Sp20 cell).

In one embodiment, a method of producing a multispecific antibody according to the invention is provided, wherein the method comprises culturing such host cell so that the antibody is produced.

In one embodiment, a method of making a multispecific antibody according to the invention is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody according to the invention, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR- CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### III. Pharmaceutical compositions

In one embodiment, a composition comprising a multispecific antibody according to the invention is provided. In one embodiment, a pharmaceutical or diagnostic composition comprising a multispecific antibody according to the invention is provided.

In one embodiment, a pharmaceutical composition comprising the multispecific antibody according to the invention in combination with at least one pharmaceutically acceptable carrier is provided.

Pharmaceutical compositions of an antibody according to the invention are prepared by mixing such antibody having the desired degree of purity with one or more optional pharmaceutically acceptable carriers (Remington's Pharmaceutical Sciences, 16th edition, Osol, A. (ed.) (1980)), in the form of lyophilized compositions or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody compositions are described in US Patent No. 6,267,958. Aqueous antibody compositions include those described in US Patent No. 6,171,586 and WO 2006/044908, the latter compositions including a histidine-acetate buffer.

The composition herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the antibody, which matrices are in the form of shaped articles, e.g. films, or microcapsules.

The compositions to be used for in vivo administration are generally sterile. Sterility may be readily accomplished, e.g., by filtration through sterile filtration membranes.

### IV. Therapeutic Methods and Compositions

Any of the multispecific antibodies provided herein may be used in therapeutic methods.

In one aspect, a multispecific antibody according to the invention for use as a medicament is provided. In further aspects, a multispecific antibody according to the invention for use in treating cancer is provided. In certain embodiments, a multispecific antibody according to the invention for use in a method of treatment is provided. In certain embodiments, the invention provides a multispecific antibody according to the invention for use in a method of treating an individual having cancer comprising administering to the individual an effective amount of the multispecific antibody according to the invention. In one such embodiment, the method further comprises administering to the individual an effective amount of at least one additional therapeutic agent, e.g., as described below.

An "individual" according to any of the above embodiments is preferably a human.

In a further aspect, the invention provides for the use of a multispecific antibody according to the invention in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent, e.g., as described below. An "individual" according to any of the above embodiments may be a human.

In a further aspect, the invention provides a method for treating cancer. In one embodiment, the method comprises administering to an individual having cancer an effective amount of a multispecific antibody according to the invention. In one such embodiment, the method further comprises administering to the individual an effective amount of at least one additional therapeutic agent, as described below. An "individual" according to any of the above embodiments may be a human.

In a further aspect, the invention provides pharmaceutical compositions comprising any of the multispecific antibodies according to the invention provided herein, e.g., for use in any of the above therapeutic methods. In one embodiment, a pharmaceutical composition comprises any of the multispecific antibodies according to the invention provided herein and a pharmaceutically acceptable carrier. In another embodiment, a pharmaceutical composition comprises any of the multispecific antibodies according to the invention provided herein and at least one additional therapeutic agent, e.g., as described below.

Antibodies of the invention can be used either alone or in combination with other agents in a therapy. For instance, an antibody of the invention may be co-administered with at least one additional therapeutic agent.

Such combination therapies noted above encompass combined administration (where two or more therapeutic agents are included in the same or separate compositions), and separate administration, in which case, administration of the antibody of the invention can occur prior to, simultaneously, and/or following, administration of the additional therapeutic agent and/or adjuvant. Antibodies of the invention can also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the composition, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.5mg/kg - 10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

It is understood that any of the above compositions or therapeutic methods may be carried out using an immunoconjugate of the invention in place of or in addition to a multispecific antibody according to the invention.

### V. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to a multispecific antibody according to the invention.

### 3. Specific embodiments of the invention

In the following specific embodiments of the invention are listed.
1. A tetravalent multispecific antibody, comprising
   a) one core antibody formed by a full length antibody, the full length antibody comprising two Fab fragments specifically binding to a first antigen, and
   b) two additional Fab fragments, wherein said additional Fab fragments are fused both either at the C-termini or the N-termini of the heavy chains of the core antibody,
      wherein the additional Fab fragments specifically bind to a second antigen,
      i) wherein either
         - the Fab fragments specifically binding to the first antigen, or
         - the Fab fragments specifically binding to the second antigen comprise a domain crossover such that the variable heavy chain domain (VH) and the variable light chain domain (VL) are replaced by each other, and
      ii) wherein either
         - the Fab fragments specifically binding to the first antigen, or
         - the Fab fragments specifically binding to the second antigen comprise the following amino acid substitutions in the constant light chain domain (CL) and the constant heavy chain domain 1 (CH1):
         - the amino acid at position 124 in the CL domain is substituted by K, R or H (numbering according to Kabat), and
         - the amino acid at position 147 or 213 in the CH1 domain is substituted by E or D (numbering according to Kabat EU index).
2. A tetravalent multispecific antibody, comprising
   a) one core antibody formed by a full length antibody, the full length antibody comprising two Fab fragments specifically binding to a first antigen and a second antigen, respectively, and
   b) two additional Fab fragments, wherein said additional Fab fragments are fused both either at the C-termini or the N-termini of the heavy chains of the core antibody,
      wherein the additional Fab fragment fused to a heavy chain exhibits the same antigen binding specificity than the Fab fragment of the core antibody arranged on said heavy chain,
      i) wherein either
         - the Fab fragments specifically binding to the first antigen, or
         - the Fab fragments specifically binding to the second antigen comprise a domain crossover such that the variable heavy chain domain (VH) and the variable light chain domain (VL) are replaced by each other, and
      ii) wherein either
         - the Fab fragments specifically binding to the first antigen, or
         - the Fab fragments specifically binding to the second antigen comprise the following amino acid substitutions in the constant light chain domain (CL) and the constant heavy chain domain 1 (CH1):
         - the amino acid at position 124 in the CL domain is substituted by K, R or H (numbering according to Kabat), and
         - the amino acid at position 147 or 213 in the CH1 domain is substituted by E or D (numbering according to Kabat EU index).
3. The antibody according to embodiment 1, wherein the core antibody is a monospecific antibody.
4. The antibody according to embodiment 1 or 2, wherein the core antibody is a bispecific antibody.
5. The antibody according to any one of embodiments 1 to 4, wherein the Fab fragments that do not comprise the domain crossover of i) comprise the amino acid substitutions of ii).
6. The antibody according to any one of embodiments 1 to 5, wherein in the Fab fragments comprising the amino acid substitutions of ii)
   - the amino acids at positions 147 and 213 in the CH1 domain are substituted independently from each other by E or D (numbering according to Kabat EU index).
7. The antibody according to any one of embodiments 1 to 6, wherein in the Fab fragments comprising the amino acid substitutions of ii)
   - the amino acids at position 123 and 124 in the CL domain are substituted independently from each other by K, R or H (numbering according to Kabat).
8. The antibody according to any one of embodiments 1 to 7, wherein in the Fab fragments comprising the amino acid substitutions of ii)
   - the amino acids at position 123 and 124 in the CL domain are substituted independently from each other by K, R or H (numbering according to Kabat); and
   - the amino acids at positions 147 and 213 in the CH1 domain are substituted independently from each other by E or D (numbering according to Kabat EU index).
9. The antibody according to any one of embodiments 1 to 8, wherein the Fab fragments that do not comprise the amino acid substitutions as defined under ii) comprise the following amino acid substitution in the CL domain
   - the amino acid at position 124 is substituted by E or D.
10. The antibody according to any one of embodiments 1 to 9, wherein the Fab fragments comprising the amino acid substitutions of ii) comprise a light chain constant domain CL of kappa isotype.
11. The antibody according to any one of embodiments 1 to 9, wherein the Fab fragments comprising the amino acid substitutions of ii) comprise a light chain constant domain CL of lambda isotype.
12. The antibody according to any one of embodiments 1 to 10, wherein in the Fab fragments comprising the amino acid substitutions of ii)
   - the amino acids at position 123 and 124 in the CL domain are substituted independently from each other by K, R or H (numbering according to Kabat); and
   - the amino acids at positions 147 and 213 in the CH1 domain are substituted independently from each other by E or D (numbering according to Kabat EU index); and
   wherein the one or two Fab fragments comprising the amino acid substitutions of ii) comprise a light chain constant domain CL of kappa isotype.
13. The antibody according to any one of embodiments 1 to 12, wherein the additional Fab fragments of b) are fused to the C-termini of the heavy chains of the core antibody.
14. The antibody according to any one of embodiments 1 to 13, wherein either
   - the Fab fragments specifically binding to the first antigen, or
   - the Fab fragments specifically binding to the second antigen comprise a domain crossover such that the VH domain and the VL domain are replaced by each other, and wherein the other Fab fragments that do not comprise said domain crossover comprise a light chain including a CL and a VL domain and a heavy chain including a CH1 and a VH domain.
15. The antibody according to any one of embodiments 1 to 14, wherein the antibody is bispecific, trispecific or tetraspecific.
16. The antibody according to any one of embodiments 1 to 15, wherein the antibody is bispecific.
17. The antibody according to any one embodiments 1 to 16, wherein the additional Fab fragments of b) are fused via a peptide connector to the heavy chains of the core antibody.
18. The antibody according to embodiment 17, wherein the peptide connector has a length of at least 5 amino acids.
19. The antibody according to embodiment 17 or 18, wherein the peptide connector is a peptide consisting of a sequential arrangement of glycine and serine residues.
20. The antibody according to any one of embodiments 1 to 19, wherein the multispecific antibody comprises two constant heavy chain domains 3 (CH3), which are altered to promote heterodimerization by generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains.
21. The antibody according to embodiment 20, wherein said amino acid residue having a larger side chain volume than the original amino acid residue is selected from R, F, Y and W.
22. The antibody according to embodiment 20 or 21, wherein said amino acid residue having a smaller side chain volume than the original amino acid residue is selected from A, S, T and V.
23. The antibody according to any one of embodiments 20 to 22, wherein said amino acid residue having a larger side chain volume than the original amino acid residue is selected from R, F, Y and W; and said amino acid residue having a smaller side chain volume than the original amino acid residue is selected from A, S, T and V.
24. The antibody according to any one of embodiments 20 to 23, wherein one of the CH3 domains comprises a T366W mutation and the other one of the CH3 domains comprises T366S, L368A and 407V mutations (numberings according to EU index of Kabat).
25. The antibody according to any one of embodiments 1 to 19, wherein the multispecific antibody comprises two CH3 domains which are altered to promote heterodimerization by substituting at least one original amino acid residue in one of the CH3 domains by a positively charged amino acid, and substituting at least one original amino acid residue in the other one of the CH3 domains by a negatively charged amino acid.
26. The antibody according to embodiment 25, wherein said positively charged amino acid is selected from K, R and H.
27. The antibody according to embodiment 25 or 26, wherein said negatively charged amino acid is selected from E or D.
28. The antibody according to any one of embodiments 25 to 27, wherein said positively charged amino acid is selected from K, R and H; and said negatively charged amino acid is selected from E or D.
29. The antibody according to any one of embodiments 25 to 27, wherein one of the CH3 domains comprises R409D and K370E mutations and the other one of the CH3 domains comprises D399K and E357K mutations (numberings according to EU index of Kabat).
30. The antibody according to any one of embodiments 1 to 29, wherein the multispecific antibody comprises two CH3 domains, which are altered to promote heterodimerization by introduction of at least one cysteine residue in each CH3 domain such that a disulfide bond is formed between the CH3 domains.
31. The antibody according to embodiment 30, wherein one of the CH3 domains comprises an E356C or an S354C mutation, and the other one of the CH3 domains comprises a Y349C mutation (numberings according to EU index of Kabat).
32. The antibody according to embodiment 30 or 31, wherein one of the CH3 domains comprises an S354C mutation, and the other one of the CH3 domains comprises a Y349C mutation (numberings according to EU index of Kabat).
33. An isolated nucleic acid encoding the antibody according to any one of embodiments 1 to 32.
34. An expression vector comprising a nucleic acid according to embodiment 33.
35. A host cell comprising a nucleic acid according to embodiment 33.
36. A host cell comprising an expression vector according to embodiment 34.
37. A composition comprising the antibody according to any one of embodiments 1 to 32.
38. A pharmaceutical or diagnostic composition comprising the antibody according to any one of embodiments 1 to 32.
39. A pharmaceutical composition comprising the according to any one of embodiments 1 to 32 in combination with at least one pharmaceutically acceptable carrier.
40. An immunoconjugate comprising the antibody according to any one of embodiments 1 to 32.
41. The immunoconjugate of embodiment 40 comprising the antibody coupled to a cytotoxic agent.
42. The antibody according to any one of embodiments 1 to 32 for use as a medicament.
43. The antibody according to any one of embodiments 1 to 32 for use in treating cancer.
44. The immunoconjugate according to embodiment 40 or 41 for use as a medicament.
45. The immunoconjugate according to embodiment 40 or 41 for use in treating cancer.
46. A method of treatment of a patient suffering from a disease by administering a multispecific antibody according to any one of embodiments 1 to 32 to the patient in the need of such treatment.
47. The method of embodiment 46, wherein the disease is cancer.
48. A method of treatment of a patient suffering from a disease by administering an immunoconjugate according to embodiment 40 or 41 to the patient in the need of such treatment.
49. The method of embodiment 48, wherein the disease is cancer.
50. A method of producing a multispecific antibody, comprising culturing a host cell according to embodiment 35 or 36 so that the multispecific antibody is produced.
51. A method of producing a multispecific antibody, comprising culturing a host cell comprising expression vectors comprising nucleic acids encoding for an antibody according to any one of embodiments 1 to 32 so that the multispecific antibody is produced.
52. A method for the preparation of the antibody according to any one of embodiments 1 to 32, comprising the steps of
   - transforming a host cell with expression vectors comprising nucleic acids encoding the antibody,
   - culturing said host cell under conditions that allow synthesis of said antibody, and
   - recovering said antibody from said host cell culture.
53. A tetravalent bispecific antibody that specifically binds to human DR5 and human FAP, comprising
   a) one core antibody formed by a full length antibody, the full length antibody comprising two Fab fragments specifically binding to human DR5, and
   b) two additional Fab fragments, wherein said additional Fab fragments are fused both either at the C-termini or the N-termini of the heavy chains of the core antibody,
      wherein the additional Fab fragments specifically bind to a human FAP,
      i) wherein
         - the Fab fragments specifically binding to human DR5 comprise a domain crossover such that the variable heavy chain domain (VH) and the variable light chain domain (VL) are replaced by each other, and
      ii) wherein
         - the Fab fragments specifically binding to human FAP comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
54. The antibody according to embodiments 53,
   - wherein the Fab fragments specifically binding to human DR5 comprise the following amino acid substitution in the CL domain:
   - the amino acid at position 124 is substituted by E or D.
55. A tetravalent bispecific antibody that specifically binds to human DR5 and human FAP, comprising
   a) one core antibody formed by a full length antibody, the full length antibody comprising two Fab fragments specifically binding to human DR5, and
   b) two additional Fab fragments, wherein said additional Fab fragments are fused both either at the C-termini or the N-termini of the heavy chains of the core antibody,
      wherein the additional Fab fragments specifically bind to a human FAP,
      iii) wherein
         - the Fab fragments specifically binding to human FAP comprise a domain crossover such that the variable heavy chain domain (VH) and the variable light chain domain (VL) are replaced by each other, and
      iv) wherein
         - the Fab fragments specifically binding to human DR5 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
56. The antibody according to embodiments 53,
   - wherein the Fab fragments specifically binding to human FAP comprise the following amino acid substitution in the CL domain:
   - the amino acid at position 124 is substituted by E or D.
57. A tetravalent bispecific antibody that specifically binds to human DR5 and human FAP, comprising
   a) one core antibody formed by a full length antibody, the full length antibody comprising two Fab fragments specifically binding to human FAP, and
   b) two additional Fab fragments, wherein said additional Fab fragments are fused both either at the C-termini or the N-termini of the heavy chains of the core antibody,
      wherein the additional Fab fragments specifically bind to a human DR5,
      v) wherein
         - the Fab fragments specifically binding to human FAP comprise a domain crossover such that the variable heavy chain domain (VH) and the variable light chain domain (VL) are replaced by each other, and
      vi) wherein
         - the Fab fragments specifically binding to human DR5 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
58. The antibody according to embodiments 53,
   - wherein the Fab fragments specifically binding to human FAP comprise the following amino acid substitution in the CL domain:
   - the amino acid at position 124 is substituted by E or D.
59. A tetravalent bispecific antibody that specifically binds to human DR5 and human FAP, comprising
   a) one core antibody formed by a full length antibody, the full length antibody comprising two Fab fragments specifically binding to human FAP, and
   b) two additional Fab fragments, wherein said additional Fab fragments are fused both either at the C-termini or the N-termini of the heavy chains of the core antibody,
      wherein the additional Fab fragments specifically bind to a human DR5,
      vii) wherein
         - the Fab fragments specifically binding to human DR5 comprise a domain crossover such that the variable heavy chain domain (VH) and the variable light chain domain (VL) are replaced by each other, and
      viii) wherein
         - the Fab fragments specifically binding to human FAP comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
60. The antibody according to embodiments 53,
   - wherein the Fab fragments specifically binding to human DR5 comprise the following amino acid substitution in the CL domain:
   - the amino acid at position 124 is substituted by E or D.
61. The bispecific antibody according to any one of the embodiments 53 to 60 that specifically binds to human DR5 and human FAP, wherein
   a) the antibody comprises a variable heavy chain domain (VH) according to SEQ ID NO: 33 (<VH DR5>) and a variable light chain domain (VL) according to SEQ ID NO: 34 (<VL DR5>); and
   b) the antibody comprises a variable heavy chain domain (VH) according to SEQ ID NO: 35 (<VH FAP>) and a variable light chain domain (VL) according to SEQ ID NO: 36 (<VL FAP>).
62. The bispecific antibody according to embodiment 54 that specifically binds to human DR5 and human FAP, wherein
   c) the antibody comprises a variable heavy chain domain (VH) according to SEQ ID NO: 33 (<VH DR5>) and a variable light chain domain (VL) according to SEQ ID NO: 34 (<VL DR5>); and
   d) the antibody comprises a variable heavy chain domain (VH) according to SEQ ID NO: 35 (<VH FAP>) and a variable light chain domain (VL) according to SEQ ID NO: 36 (<VL FAP>).
63. A tetravalent bispecific antibody that specifically binds to human PD1 and human TIM3, comprising
   a) one core antibody formed by a full length antibody, the full length antibody comprising two Fab fragments specifically binding to human PD1, and
   b) two additional Fab fragments, wherein said additional Fab fragments are fused both either at the C-termini or the N-termini of the heavy chains of the core antibody,
      wherein the additional Fab fragments specifically bind to a human TIM3,
      ix) wherein
         - the Fab fragments specifically binding to human PD1 comprise a domain crossover such that the variable heavy chain domain (VH) and the variable light chain domain (VL) are replaced by each other, and
      x) wherein
         - the Fab fragments specifically binding to human TIM3 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
64. The antibody according to embodiments 53,
   - wherein the Fab fragments specifically binding to human PD1 comprise the following amino acid substitution in the CL domain:
   - the amino acid at position 124 is substituted by E or D.
65. A tetravalent bispecific antibody that specifically binds to human PD1 and human TIM3, comprising
   a) one core antibody formed by a full length antibody, the full length antibody comprising two Fab fragments specifically binding to human PD1, and
   b) two additional Fab fragments, wherein said additional Fab fragments are fused both either at the C-termini or the N-termini of the heavy chains of the core antibody,
      wherein the additional Fab fragments specifically bind to a human TIM3,
      xi) wherein
         - the Fab fragments specifically binding to human TIM3 comprise a domain crossover such that the variable heavy chain domain (VH) and the variable light chain domain (VL) are replaced by each other, and
      xii) wherein
         - the Fab fragments specifically binding to human PD1 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
66. The antibody according to embodiments 53,
   - wherein the Fab fragments specifically binding to human TIM3 comprise the following amino acid substitution in the CL domain:
   - the amino acid at position 124 is substituted by E or D.
67. A tetravalent bispecific antibody that specifically binds to human PD 1 and human TIM3, comprising
   a) one core antibody formed by a full length antibody, the full length antibody comprising two Fab fragments specifically binding to human TIM3, and
   b) two additional Fab fragments, wherein said additional Fab fragments are fused both either at the C-termini or the N-termini of the heavy chains of the core antibody,
      wherein the additional Fab fragments specifically bind to a human PD1,
      xiii) wherein
         - the Fab fragments specifically binding to human TIM3 comprise a domain crossover such that the variable heavy chain domain (VH) and the variable light chain domain (VL) are replaced by each other, and
      xiv) wherein
         - the Fab fragments specifically binding to human PD1 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
68. The antibody according to embodiments 53,
   - wherein the Fab fragments specifically binding to human TIM3 comprise the following amino acid substitution in the CL domain:
   - the amino acid at position 124 is substituted by E or D.
69. A tetravalent bispecific antibody that specifically binds to human PD 1 and human TIM3, comprising
   a) one core antibody formed by a full length antibody, the full length antibody comprising two Fab fragments specifically binding to human TIM3, and
   b) two additional Fab fragments, wherein said additional Fab fragments are fused both either at the C-termini or the N-termini of the heavy chains of the core antibody,
      wherein the additional Fab fragments specifically bind to a human PD1,
      xv) wherein
         - the Fab fragments specifically binding to human PD1 comprise a domain crossover such that the variable heavy chain domain (VH) and the variable light chain domain (VL) are replaced by each other, and
      xvi) wherein
         - the Fab fragments specifically binding to human TIM3 comprise the following amino acid substitutions in the constant light chain domain (CL) and the constant heavy chain domain 1 (CH1):
         - the amino acid at position 123 and 124 in the CL domain is substituted by K, R or H (preferably K or R) (numbering according to Kabat), and
         - the amino acid at position 147 and 213 in the CH1 domain is substituted by E or D (numbering according to Kabat EU index).
70. The antibody according to embodiments 53,
   - wherein the Fab fragments specifically binding to human PD1 comprise the following amino acid substitution in the CL domain:
   - the amino acid at position 124 is substituted by E or D.
71. The bispecific antibody according to any one of the embodiments 63 to 70 that specifically binds to human PD1 and human TIM3, wherein
   e) the antibody comprises a variable heavy chain domain (VH) according to SEQ ID NO: 46 (<VH PD1>) and a variable light chain domain (VL) according to SEQ ID NO: 47 (<VL PD1>); and
   f) the antibody comprises a variable heavy chain domain (VH) according to SEQ ID NO: 48 (<VH TIM3>) and a variable light chain domain (VL) according to SEQ ID NO: 49 (<VL TIM3>).
72. The bispecific antibody according to any one of the embodiments 63 to 70 that specifically binds to human PD1 and human TIM3, wherein
   g) the antibody comprises a variable heavy chain domain (VH) according to SEQ ID NO: 46 (<VH PD1>) and a variable light chain domain (VL) according to SEQ ID NO: 47 (<VL PD1>); and
   h) the antibody comprises a variable heavy chain domain (VH) according to SEQ ID NO: 50 (<VH TIM3>) and a variable light chain domain (VL) according to SEQ ID NO: 51 (<VL TIM3>).

### DESCRIPTION OF THE AMINO ACID SEQUENCES

| | |
|---|---|
| **SEQ ID NO:1** | light chain (LC) <Ang-2> wild type (wt) |
| **SEQ ID NO:2** | heavy chain (HC) <Ang-2> wild type (wt) |
| **SEQ ID NO:3** | heavy chain (HC) <VEGF> with VH-VL exchange wild type (wt) |
| **SEQ ID NO:4** | light chain (LC) <VEGF> with VH-VL exchange wild type (wt) |
| **SEQ ID NO:5** | light chain (LC) <Ang-2> with Q124K substitution |
| **SEQ ID NO:6** | heavy chain (HC) <Ang-2> with K147E substitution |
| **SEQ ID NO:7** | heavy chain (HC) <Ang-2> with K213E substitution |
| **SEQ ID NO:8** | light chain (LC) <Ang-2> with E123K substitution |
| **SEQ ID NO:9** | light chain (LC) <Ang-2> with Q124K substitution and E123K substitution |
| **SEQ ID NO:10** | heavy chain (HC) <Ang-2> with K147E substitution and K213E substitution |
| **SEQ ID NO:1**1 | light chain (LC) <Ang-2> with Q124R substitution and E123K substitution |
| **SEQ ID NO:12** | light chain (LC) <VEGF> with VH-VL exchange with Q124E substitution |
| **SEQ ID NO:13** | light chain (LC) <Ang-2> with E124K substitution and E123K substitution |
| **SEQ ID NO:14** | heavy chain (HC) <Ang-2> with K147E substitution and K213D substitution |
| **SEQ ID NO:15** | DR5TAA-0057 LC1 (Vl-CL, non crossed, DR5, N-term) |
| **SEQ ID NO:16** | DR5TAA-0057 HC (DR5-Vh-CH1 ...G4S-FAP-Vh-CL) |
| **SEQ ID NO:17** | DR5TAA-0057 LC2 (FAP, V1-CH1, crossed, C-Term) |
| **SEQ ID NO:18** | DR5TAA-0077 LC1 (CDR5, crossed, N-Term, Vh-CL) |
| **SEQ ID NO:19** | DR5TAA-0077 HC (DR5-V1-CH1 ... G4S-FAP-Vh-CH1) |
| **SEQ ID NO:20** | DR5TAA-0077 LC2 (FAP, Vl-CL, C-term) |
| **SEQ ID NO:21** | DR5TAA-0078 LC1 (DR5 crossed, V1-CH1, N-term) |
| **SEQ ID NO:22** | DR5TAA-0078 HC (DR5-Vh-CL G4S-FAP-Vh-CH1) |
| **SEQ ID NO:23** | DR5TAA-0078 LC2 (FAP non crossed, C-term, VL-CL) |
| **SEQ ID NO:24** | DR5TAA-0081 LC1 (FAP crossed, VH-CL) |
| **SEQ ID NO:25** | DR5TAA-0081 HC (DR5- VH-CH1 G4S-FAP-VL-CH1) |
| **SEQ ID NO:26** | DR5TAA-0081 LC2 (DR5, VL-CL) |
| **SEQ ID NO:27** | DR5TAA-0082 LC1 (DR5-VL-CL) |
| **SEQ ID NO:28** | DR5TAA-0082 HC (DR5-Vh-CH1 G4S-FAP-V1-CH1) |
| **SEQ ID NO:29** | DR5TAA-0082 LC2 (FAP crossed-Vh-CL) |
| **SEQ ID NO:30** | DR5TAA-0083 LC1 (DR5-Vh-CL) |
| **SEQ ID NO:31** | DR5TAA-0083 HC (DR5-VL-CH1- G4S-FAP-Vh-CH1) |
| **SEQ ID NO:32** | DR5TAA-0083 LC2 (FAP-V1-CL) |
| **SEQ ID NO:33** | VH <DR5> |
| **SEQ ID NO:34** | VL <DR5> |
| **SEQ ID NO:35** | VH <FAP> |
| **SEQ ID NO:36** | VL <FAP> |
| **SEQ ID NO:37** | heavy chain of 2+2 PD1TIM3_0358: chimeric PD1-0103 / Tim3-0028 |
| **SEQ ID NO:38** | light chain 1 of 2+2 PD1TIM3_0358 |
| **SEQ ID NO:39** | light chain 2 of 2+2 PD1TIM3_0358 |
| **SEQ ID NO:40** | heavy chain of 2+2 PD1TIM3_0359: chimeric PD1-0103 / Tim3-0018 |
| **SEQ ID NO:41** | light chain 1 of 2+2 PD1TIM3_0359 |
| **SEQ ID NO:42** | light chain 2 of 2+2 PD1TIM3_0359 |
| **SEQ ID NO:43** | heavy chain of 2+2 PD1TIM3_0321: chimeric PD1-0103 / Tim3-0038 |
| **SEQ ID NO:44** | light chain 1 of 2+2 PD1TIM3_0321 |
| **SEQ ID NO:45** | light chain 2 of 2+2 PD1TIM3_0321 |
| **SEQ ID NO:46** | VH humanized version of PD1-0103 (=PD1-0103_0312) |
| **SEQ ID NO:47** | VL humanized version of PD1-0103 (=PD1-0103_0312) |
| **SEQ ID NO:48** | VH humanized version of Tim3-0028 (= Tim3-0438) |
| **SEQ ID NO:49** | VL humanized version of Tim3-0028 (= Tim3-0438) |
| **SEQ ID NO:50** | VH humanized version of Tim3_0016 variant (0018) (= Tim3-0434) |
| **SEQ ID NO:51** | VL humanized version of Tim3_0016 variant (0018) (= Tim3-0434) |

### EXAMPLES

The following examples are provided to aid the understanding of the present invention, the true scope of which is set forth in the appended claims. It is understood that modifications can be made in the procedures set forth without departing from the spirit of the invention.

### Materials & general methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the numbering systems according to Kabat (Kabat, E.A., et al., Sequences of Proteins of Immunological Interest, 5th ed., Public Health Service, National Institutes of Health, Bethesda, MD (1991)) as defined above.

### Recombinant DNA techniques

Standard methods were used to manipulate DNA as described in Sambrook, J. et al., Molecular Cloning: A laboratory manual; Cold Spring Harbor Laboratory Press, Cold Spring Harbor, New York, 1989. The molecular biological reagents were used according to the manufacturer's instructions.

### Gene synthesis

Desired gene segments were prepared from oligonucleotides made by chemical synthesis. The 600 - 1800 bp long gene segments, which were flanked by singular restriction endonuclease cleavage sites, were assembled by annealing and ligating oligonucleotides including PCR amplification and subsequently cloned via the indicated restriction sites e.g. KpnI/ SacI or AscI/PacI into a pPCRScript (Stratagene) based pGA4 cloning vector. The DNA sequences of the subcloned gene fragments were confirmed by DNA sequencing. Gene synthesis fragments were ordered according to given specifications at Geneart (Regensburg, Germany).

### DNA sequence determination

DNA sequences were determined by double strand sequencing performed at MediGenomix GmbH (Martinsried, Germany) or Sequiserve GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 was used for sequence creation, mapping, analysis, annotation and illustration.

### Expression vectors

For the expression of the described antibodies, variants of expression plasmids for transient expression (e.g. in HEK293 EBNA or HEK293-F) cells based either on a cDNA organization with or without a CMV-Intron A promoter or on a genomic organization with a CMV promoter were applied.

Beside the antibody expression cassette the vectors contained:
- an origin of replication which allows replication of this plasmid in *E. coli*, and
- a ß-lactamase gene which confers ampicillin resistance in *E. coli*.

The transcription unit of the antibody gene was composed of the following elements:
- unique restriction site(s) at the 5' end
- the immediate early enhancer and promoter from the human cytomegalovirus,
- followed by the Intron A sequence in the case of the cDNA organization,
- a 5'-untranslated region of a human antibody gene,
- an immunoglobulin heavy chain signal sequence,
- the human antibody chain (wildtype or with domain exchange) either as cDNA or as genomic organization with the immunoglobulin exon-intron organization
- a 3' untranslated region with a polyadenylation signal sequence, and
- unique restriction site(s) at the 3' end.

The fusion genes comprising the antibody chains as described below were generated by PCR and/or gene synthesis and assembled by known recombinant methods and techniques by connection of the according nucleic acid segments e.g. using unique restriction sites in the respective vectors. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

Multispecific antibodies were expressed by transient co-transfection of the respective expression plasmids in adherently growing HEK293-EBNA or in HEK29-F cells growing in suspension as described below.

### Transient transfections in HEK293-EBNA system

Multispecific antibodies were expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear antigen; American type culture collection deposit number ATCC # CRL-10852, Lot. 959 218) cultivated in DMEM (Dulbecco's modified Eagle's medium, Gibco®) supplemented with 10% Ultra Low IgG FCS (fetal calf serum, Gibco®), 2 mM L-Glutamine (Gibco®), and 250 µg/ml Geneticin (Gibco®). For transfection FuGENE™ 6 Transfection Reagent (Roche Molecular Biochemicals) was used in a ratio of FuGENE™ reagent (µl) to DNA (µg) of 4:1 (ranging from 3:1 to 6:1). Proteins were expressed from the respective plasmids using a molar ratio of (modified and wildtype) light chain and heavy chain encoding plasmids of 1:1 (equimolar) ranging from 1:2 to 2:1, respectively. Cells were fed at day 3 with L-Glutamine ad 4 mM, Glucose [Sigma] and NAA [Gibco®]. Multispecific antibody containing cell culture supernatants were harvested from day 5 to 11 after transfection by centrifugation and stored at -20°C. General information regarding the recombinant expression of human immunoglobulins in e.g. HEK293 cells is given in: Meissner, P. et al., Biotechnol. Bioeng. 75 (2001) 197-203.

### Transient transfections in HEK293-F system

Multispecific antibodies were generated by transient transfection with the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serum-free FreeStyle™ 293 expression medium (Invitrogen) were transfected with a mix of the four expression plasmids and 293fectin™ or fectin (Invitrogen). For 2 L shake flask (Corning) HEK293-F cells were seeded at a density of 1.0E*6 cells/mL in 600 mL and incubated at 120 rpm, 8% CO2. The day after the cells were transfected at a cell density of ca. 1.5E*6 cells/mL with ca. 42 mL mix of A) 20 mL Opti-MEM (Invitrogen) with 600 µg total plasmid DNA (1 µg/mL) encoding the heavy or modified heavy chain, respectively and the corresponding light chain in an equimolar ratio and B) 20 ml Opti-MEM + 1.2 mL 293 fectin or fectin (2 µl/mL). According to the glucose consumption glucose solution was added during the course of the fermentation. The supernatant containing the secreted antibody was harvested after 5-10 days and antibodies were either directly purified from the supernatant or the supernatant was frozen and stored.

### Protein determination

The protein concentration of purified antibodies and derivatives was determined by determining the optical density (OD) at 280 nm, using the molar extinction coefficient calculated on the basis of the amino acid sequence according to Pace, et al., Protein Science, 1995, 4, 2411-1423.

### Antibody concentration determination in supernatants

The concentration of antibodies and derivatives in cell culture supernatants was estimated by immunoprecipitation with Protein A Agarose-beads (Roche). 60 µL Protein A Agarose beads were washed three times in TBS-NP40 (50 mM Tris, pH 7.5, 150 mM NaCl, 1% Nonidet-P40). Subsequently, 1 -15 mL cell culture supernatant were applied to the Protein A Agarose beads pre-equilibrated in TBS-NP40. After incubation for at 1 hour at room temperature the beads were washed on an Ultrafree-MC-filter column (Amicon) once with 0.5 mL TBS-NP40, twice with 0.5 mL 2x phosphate buffered saline (2xPBS, Roche) and briefly four times with 0.5 mL 100 mM Na-citrate pH 5,0. Bound antibody was eluted by addition of 35 µl NuPAGE® LDS Sample Buffer (Invitrogen). Half of the sample was combined with NuPAGE® Sample Reducing Agent or left unreduced, respectively, and heated for 10 min at 70°C. Consequently, 5-30 µl were applied to a 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.

The concentration of antibodies and derivatives in cell culture supernatants was quantitatively measured by affinity HPLC chromatography. Briefly, cell culture supernatants containing antibodies and derivatives that bind to Protein A were applied to an Applied Biosystems Poros A/20 column in 200 mM KH2PO4, 100 mM sodium citrate, pH 7.4 and eluted from the matrix with 200 mM NaCl, 100 mM citric acid, pH 2,5 on an Agilent HPLC 1100 system. The eluted protein was quantified by UV absorbance and integration of peak areas. A purified standard IgG1 antibody served as a standard.

Alternatively, the concentration of antibodies and derivatives in cell culture supernatants was measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcγ> BI (Dianova) at 0.1 µg/mL for 1 hour at room temperature or alternatively overnight at 4°C and subsequently washed three times with 200 µL/well PBS, 0.05% Tween (PBST, Sigma). 100 µL/well of a dilution series in PBS (Sigma) of the respective antibody containing cell culture supernatants was added to the wells and incubated for 1-2 hour on a microtiterplate shaker at room temperature. The wells were washed three times with 200 µL/well PBST and bound antibody was detected with 100 µl F(ab')2<hFcγ>POD (Dianova) at 0.1 µg/mL as the detection antibody for 1-2 hours on a microtiterplate shaker at room temperature. Unbound detection antibody was washed away three times with 200 µL/well PBST and the bound detection antibody was detected by addition of 100 µL ABTS/well. Determination of absorbance was performed on a Tecan Fluor Spectrometer at a measurement wavelength of 405 nm (reference wavelength 492 nm).

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VH/VL exchange (VH/VL CrossMabs) (and 1 control with CH1/CL exchange (CH1/CL CrossMab)) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs (CH1/CL CrossMab control) were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Determination of binding and binding affinity of multispecific antibodies to the respective antigens using surface plasmon resonance (SPR) (BIACORE)

### VEGF binding was assessed according to the following procedure:

Binding of indicated antibodies to human VEGFA-121 was investigated by surface plasmon resonance using a BIACORE® T200 instrument (GE Healthcare). Around 10000 (RU) of anti His antibody (1 µg/ml anti His antibody; Order Code: 28995056; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

VEFGA-121-His was captured by injecting a 0.5 µg/ml solution for 30 sec at a flow of 5 µl/min. The association was measured by injection of the indicated antibodies in various concentrations in solution for 180 sec at a flow of 30 µl/min starting with 1000 nM in 1:3 serial dilutions. The dissociation phase was monitored for up to 600 sec and triggered by switching from the sample solution to running buffer. The surface was regenerated by 60 sec washing with a Glycine pH 1.5 solution at a flow rate of 30 µl/min. Bulk refractive index differences were corrected by subtracting the response obtained from a anti His antibody surface. Blank injections are also subtracted (= double referencing). For calculation of K_{D} and other kinetic parameters the Langmuir 1:1 model was used.

### Ang-2 binding was assessed according to the following procedure:

Binding of indicated antibodies to human Ang-2-RBD-Fc was investigated by surface plasmon resonance using a BIACORE® T200 instrument (GE Healthcare). Around 8000 (RU) of goat anti human F(ab')₂ (10 µg/ml anti human F(ab)'₂; Order Code: 28958325; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The bispecific antibody was captured by injecting a 5 nM solution for 25 sec at a flow of 5 µl/min. The association was measured by injection of human Ang2-RBD-Fc in various concentrations in solution for 120 sec at a flow of 30 µl/min starting with 100 nM in 1:3 serial dilutions. The dissociation phase was monitored for up to 180 sec and triggered by switching from the sample solution to running buffer. The surface was regenerated by 60 sec washing with a Glycine pH 2.1 solution at a flow rate of 30 µl/min. Bulk refractive index differences were corrected by subtracting the response obtained from a goat anti human F(ab')₂ surface. Blank injections are also subtracted (= double referencing). For calculation of apparent K_{D} the Langmuir 1:1 model was used.

### Assessment of simultaneous DR5- and FAP-binding to the Crossmab

First, around 600 resonance units (RU) of DR5 (20µg/ml) were coupled on a CM5 chip (GE Healthcare BR-1005-30) at pH 5.0 by using an amine coupling kit supplied by the GE Healthcare. The sample and system buffer was PBS-T (10 mM phosphate buffered saline including 0.05% Tween 20) pH 7.4. Flow cell was set to 25 °C and sample block to 12 °C and primed with running buffer twice. Second, 10 µg/ml solution of the bispecific antibody was injected for 30 sec at a flow of 30 µl/min. Third, hFAP (10µg/ml) was injected for 30 sec at a flow of 30 µl/min. The binding response of hFAP depends from the amount of the bispecific antibody bound to hDR5 and shows simultaneous binding. The surface was regenerated by 70 sec washing with a Glycine pH2 solution (GE Healthcare BR-1003-55) at a flow rate of 30 µl/min. Simultaneous binding is shown by an additional specific binding signal of hFAP to the previous DR5 bound Crossmab.

### Assessment of independent DR5- and FAP-binding to the Crossmab

Around 3000 resonance units (RU) of the capturing system (5 µg/ml anti human IgG (Fc); GE Healthcare, BR-1008-39) were coupled on a CM5 chip (GE Healthcare BR-1005-30) at pH 5.0 by using an amine coupling kit supplied by the GE Healthcare. The sample and system buffer was PBS-T (10 mM phosphate buffered saline including 0.05% Tween20 ) pH 7.4. The temperature of the flow cell was set to 25 °C and of the sample block to 12 °C. Before capturing, the flow cell was primed with running buffer twice.

The bispecific antibody was captured by injecting a 5µg/ml solution for 60 sec at a flow of 5 µl/min. Independent binding of each ligand to the bispecific antibody was analyzed by determining the active binding capacity for each ligand, either added sequentially or simultaneously (flow of 10 µl/min):
1. Injection of human DR5 with a concentration of 5µg/ml for 180 sec (identifies the single binding of the antigen).
2. Injection of human FAP with a concentration of 5µg/ml for 180 sec (identifies single binding of the antigen).
3. Injection of human DR5 with a concentration of 5µg/ml and of human FAP with a concentration of 5µg/ml for 180 sec (identifies the binding of DR5 and of FAP at the same time).

The surface was regenerated by 60 sec washing with a 3m MgCl2 solution at a flow rate of 30 µl/min. Bulk refractive index differences were corrected by subtracting the response obtained from an anti-human IgG surface.

The bispecific antibody is able to bind both antigens mutual independently if the resulting final signal of the approach 3 equals the sum of the individual final signals of the approaches 1 and 2.

### PD1 binding was assessed according to the following procedure:

Anti-human Fc IgG was immobilized by amine coupling to the surface of a (Biacore) CM5 sensor chip. The samples were then captured and hu PD1-ECD was bound to them. The sensor chip surface was regenerated after each analysis cycle. The equilibrium constant and kinetic rate constants were finally gained by fitting the data to a 1:1 langmuir interaction model.

About 10,000 response units (RU) of 20 µg/ml anti-human IgG (GE Healthcare #BR-1008-39) were coupled onto all flow cells of a CM5 sensor chip in a Biacore T200 using an amine coupling kit supplied by GE Healthcare. The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

Different samples were injected for 15 seconds with a concentration of 10 nM and consecutively bound to the flow cells 2, 3 and 4. Then a complete set of human PD1-ECD concentrations (300 nM, 100 nM, 2 × 33.3 nM, 11.1 nM, 3.7 nM, 1.2 nM and 2 × 0 nM) was injected over each sample for 300s followed by a dissociation time of 10/600s and two 30s regeneration steps with 3 M MgCl2, of which the last one contained an "extra wash after injection" with running buffer. Finally the double referenced data was fitted to a 1:1 langmuir interaction model with the Biacore T200 Evaluation Software.

### TIM3 binding was assessed according to the following procedure:

Anti-human Fab IgG was immobilized by amine coupling to the surface of a (Biacore) CM5 sensor chip. The samples were then captured and hu Tim3-ECD was bound to them. The sensor chip surface was regenerated after each analysis cycle. The equilibrium constant and kinetic rate constants were finally gained by fitting the data to a 1:1 langmuir interaction model.

About 10,000 response units (RU) of 20 µg/ml anti-human Fab IgG (GE Healthcare #28-9583-25) were coupled onto all flow cells of a CM5 sensor chip in a Biacore T200 using an amine coupling kit supplied by GE Healthcare. The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

Different samples were injected for 30 seconds with a concentration of 10 nM and bound consecutively to the flow cells 2, 3 and 4. Then a complete set of human Tim3-ECD concentrations (600 nM, 200 nM, 2 × 66.7 nM, 22.2 nM, 7.4 nM and 2 × 0 nM) was injected over each sample for 200s followed by a dissociation time of 10/600s and two 30s regeneration steps with Glycine HCl pH 2.1, of which the last one contained an "extra wash after injection" with running buffer. Finally the double referenced data was fitted to a 1:1 langmuir interaction model with the Biacore T200 Evaluation Software.

### Example 1

### Provision of bivalent multispecific antibodies with a VH/VL domain crossover in one binding arm with charged amino acid substitutions in the CH1/CL interface (proof-of-concept)

In order to provide a proof-of-concept demonstrating the effects of substituting distinct amino acids within the CH1/CL interface of a multispecific antibody, exemplary bivalent antibodies with a VH/VL domain crossover in one of their two binding arms were generated.

Antibodies were generated without amino acid substitutions in the CH1/CL interface (control), and with single as well as multiple charged amino acid substitutions in the CH1/CL interface. Exemplarily, multispecific antibodies binding to human Angiopoietin-2 (ANG2) with the non-crossed binding arm and to human VEGF with the VH/VL crossed binding arm were generated. Antibody generation was performed as described in the general methods section by classical molecular biology techniques and by transiently expressing the antibodies in HEK293 cells as described above.

Amino acid substitutions in the respective antibodies is indicated in **Table 1.**

**Table 1: Amino acid substitutions of bivalent multispecific anti-ANG2-VEGF antibodies for proof-of-concept**

| | **CL ANG-2 (position 124)** | **CL ANG-2 (position 123)** | **CH1 ANG-2 (position 147)** | **CH1 ANG-2 (position 213)** | **CH1 VEGF** | **CL VEGF** |
|---|---|---|---|---|---|---|
| Ang2VEGF-0273 | wt: Q124 | wt: E123 | wt: K147 | wt: K213 | wt | wt |
| Ang2VEGF-0396 | **Q124K** | wt | **K147E** | wt | wt | wt |
| Ang2VEGF-0397 | **Q124K** | wt | wt | **K213E** | wt | wt |
| Ang2VEGF-0394 | wt | E123K | **K147E** | wt | wt | wt |
| Ang2VEGF-0395 | wt | **E123K** | wt | **K213E** | wt | wt |
| Ang2VEGF-0274 | **Q124K** | **E123K** | **K147E** | **K213E** | wt | wt |
| Ang2VEGF-0282 | **Q124R** | **E123K** | **K147E** | **K213E** | wt | **Q124E** |
| Ang2VEGF-0283 | **E124K** *(lambda)* | **E123K** | **K147E** | **K213E** | wt | wt: |
| Ang2VEGF-0284 | **Q124R** | **E123K** | **K147E** | **K213D** | wt | wt |
| Ang2VEGF-0285 | **Q124R** | **E123K** | **K147E** | **K213D** | wt | **Q124E** |
| Ang2VEGF-0286 | **Q124K** | **E123K** | **K147E** | **K213E** | wt | **Q124E** |

The multispecific antibodies were expressed using expression plasmids containing the nucleic acids encoding the amino acid sequences depicted in **Table 2.**

**Table 2: Amino acid sequences of bivalent multispecific anti-ANG2-VEGF antibodies for proof-of-concept**

| **Antibody** | **LC ANG-2** | **HC ANG-2** | **HC VEGF** | **LC VEGF** |
|---|---|---|---|---|
| Ang2VEGF-0273 | SEQ ID NO: 1 | SEQ ID NO: 2 | SEQ ID NO: 3 | SEQ ID NO: 4 |
| Ang2VEGF-0396 | SEQ ID NO: 5 | SEQ ID NO: 6 | SEQ ID NO: 3 | SEQ ID NO: 4 |
| Ang2VEGF-0397 | SEQ ID NO: 5 | SEQ ID NO: 7 | SEQ ID NO: 3 | SEQ ID NO: 4 |
| Ang2VEGF-0394 | SEQ ID NO: 8 | SEQ ID NO: 6 | SEQ ID NO: 3 | SEQ ID NO: 4 |
| Ang2VEGF-0395 | SEQ ID NO: 8 | SEQ ID NO: 7 | SEQ ID NO: 3 | SEQ ID NO: 4 |
| Ang2VEGF-0274 | SEQ ID NO: 9 | SEQ ID NO: 10 | SEQ ID NO: 3 | SEQ ID NO: 4 |
| Ang2VEGF-0282 | SEQ ID NO: 11 | SEQ ID NO: 10 | SEQ ID NO: 3 | SEQ ID NO: 12 |
| Ang2VEGF-0283 | SEQ ID NO: 13 | SEQ ID NO: 10 | SEQ ID NO: 3 | SEQ ID NO: 4 |
| Ang2VEGF-0284 | SEQ ID NO: 11 | SEQ ID NO: 14 | SEQ ID NO: 3 | SEQ ID NO: 4 |
| Ang2VEGF-0285 | SEQ ID NO: 11 | SEQ ID NO: 14 | SEQ ID NO: 3 | SEQ ID NO: 12 |
| Ang2VEGF-0286 | SEQ ID NO: 9 | SEQ ID NO: 10 | SEQ ID NO: 3 | SEQ ID NO: 12 |

For all constructs knobs into holes heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349C) (contained in the respective corresponding heavy chain (HC) sequences depicted above).

The expressed multispecific antibodies were purified from the cell culture supernatant by a combination of Protein A affinity chromatography and size exclusion chromatography. All multispecific antibodies can be produced in good yields and are stable.

The obtained products were characterized for identity by mass spectrometry and analytical properties such as purity by SDS-PAGE, monomer content and stability.

### Mass spectrometry

The expected primary structures were analyzed by electrospray ionization mass spectrometry (ESI-MS) of the deglycosylated intact antibodies and deglycosylated/plasmin digested or alternatively deglycosylated/limited LysC digested antibodies.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated antibodies in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

The main side product of bivalent multispecific antibodies with a VH/VL domain crossover is based on a Bence-Jones-type interaction (see also Schaefer, W. et al, PNAS, 108 (2011) 11187-1191; in Fig. S1I of the Supplement). Analysis of the bivalent, multispecific antibodies revealed that the main side product of VH/VL domain crossover multispecific antibodies could be tremendously reduced by introducing oppositely charged amino acids in the CH1/CL interface. Results of the MS analysis are shown in **Table 3.**

**Table 3: Fraction of main side product (Bence-Jones type) formed when expressing bivalent multispecific anti-ANG2-VEGF antibodies for proof-of-concept as determined by mass spectrometry**

| **antibody** | **amino acid substitution** | **[%] Bence-Jones-type side product (by MS)** |
|---|---|---|
| Ang2VEGF-0273 | none (wild type, control) | ∼20 |
| Ang2VEGF-0396 | single substitution in CH1/CL interface | ∼3 |
| Ang2VEGF-0397 | single substitution in CH1/CL interface | ∼3 |
| Ang2VEGF-0394 | single substitution in CH1/CL interface | ∼15 |
| Ang2VEGF-0395 | single substitution in CH1/CL interface | ∼15 |
| Ang2VEGF-0274 | multiple substitution in CH1/CL interface | 0 |
| Ang2VEGF-0282 | multiple substitution in CH1/CL interface | 0 |
| Ang2VEGF-0283 | multiple substitution in CH1/CL interface | 0 |
| Ang2VEGF-0284 | multiple substitution in CH1/CL interface | 0 |
| Ang2VEGF-0285 | multiple substitution in CH1/CL interface | 0 |
| Ang2VEGF-0286 | multiple substitution in CH1/CL interface | 0 |

As indicated by the MS results, introducing oppositely charged amino acids in the CH1/CL interface strongly reduces side product formation when compared to a "wild type" VH/VL exchanged bivalent control antibody lacking those substitutions. As indicated amino acid substitutions of the invention, i.e. substitution of the amino acid at position 124 in the CL domain in combination with a substitution at position 147 or 213 in the corresponding CH1 (see antibodies Ang2VEGF-0396 and Ang2VEGF-0397) domain result in superior reduction of side products when compared to antibody molecules only differing in one amino acid residue (i.e. an exchange of amino acid 123 instead of amino acid 124 in the CL domain; see antibodies Ang2VEGF-0394 and Ang2VEGF-0395). Antibodies comprising multiple amino acid substitutions including the amino acid substitution at position 124 in the CL domain showed no or undetectable Bence-Jones-type side product, indicating that formation of the main side product could be completely suppressed.

All antibodies were producible in good yields. Exemplary production yields of indicated multispecific antibodies were assessed after Protein A purification. Results are shown in **Table 4.**

**Table 4: Production yields [mg/L supernatant] of indicated antibodies**

| **Antibody** | **Yield [mg/L]** |
|---|---|
| Ang2VEGF-0273 | 65 |
| Ang2VEGF-0396 | 80.8 |
| Ang2VEGF-0397 | 68.4 |
| Ang2VEGF-0394 | 79.2 |
| Ang2VEGF-0395 | 93.6 |

Antigen binding was assessed as described in the general methods section. Results are indicated in **Table 5.**

**Table 5: Affinity for ANG2 and VEGF of indicated antibodies**

| **Sample** | **amino acid substitution** | **KD (nM) ANG2** | **KD (nM) VEGF** |
|---|---|---|---|
| Ang2VEGF-0273 | none (wild type, control) | 15 | 6 |
| Ang2VEGF-0396 | single substitution in CH1/CL interface | 17 | 3 |
| Ang2VEGF-0397 | single substitution in CH1/CL interface | 14 | 4 |
| Ang2VEGF-0394 | single substitution in CH1/CL interface | 12 | 3 |
| Ang2VEGF-0395 | single substitution in CH1/CL interface | 15 | 4 |
| Ang2VEGF-0274 | multiple substitution in CH1/CL interface | 17 | 3 |
| Ang2VEGF-0282 | multiple substitution in CH1/CL interface | 14 | 4 |
| Ang2VEGF-0283 | multiple substitution in CH1/CL interface | 15 | 4 |
| Ang2VEGF-0284 | multiple substitution in CH1/CL interface | 13 | 4 |
| Ang2VEGF-0285 | multiple substitution in CH1/CL interface | 14 | 4 |
| Ang2VEGF-0286 | multiple substitution in CH1/CL interface | 12 | 4 |

All tested antibodies specifically bind to both targets, Ang2 and VEGF, and exhibit an antigen affinity in the nanomolar range. The introduction of charged amino acid residues does not impair antigen binding.

In order to assess stability of the antibody constructs, thermal stability as well as aggregation onset temperatures were assessed according to the following procedure.

Samples of the indicated antibodies were prepared at a concentration of 1 mg/mL in 20 mM Histidine/Histidine chloride, 140 mM NaCl, pH 6.0, transferred into a 10 µL micro-cuvette array and static light scattering data as well as fluorescence data upon excitation with a 266 nm laser were recorded with an Optim1000 instrument (Avacta Inc.), while the samples were heated at a rate of 0.1 °C/min from 25°C to 90°C.

The aggregation onset temperature (T_{agg}) is defined as the temperature at which the scattered light intensity starts to increase. The melting temperature (Tₘ) is defined as the inflection point in a fluorescence intensity vs. wavelength graph.
Results are shown in **Table 6.**

**Table 6: Aggregation onset temperature (Tagg) and melting temperature (Tm) of indicated antibodies**

| **Sample** | **amino acid substitution** | **T_{agg} (°C)** | **Tₘ (°C)** |
|---|---|---|---|
| Ang2VEGF-0273 | none (wild type, control) | 56,0 | 61,3 |
| Ang2VEGF-0396 | single substitution in CH1/CL interface | 56,9 | 62,0 |
| Ang2VEGF-0397 | single substitution in CH1/CL interface | 56,0 | 61,7 |
| Ang2VEGF-0394 | single substitution in CH1/CL interface | 56,9 | 62,2 |
| Ang2VEGF-0395 | single substitution in CH1/CL interface | 56,8 | 62,1 |
| Ang2VEGF-0274 | multiple substitution in CH1/CL interface | 53,5 | 58,9 |
| Ang2VEGF-0282 | multiple substitution in CH1/CL interface | 56,9 | 61,4 |
| Ang2VEGF-0283 | multiple substitution in CH1/CL interface | 56,3 | 61,0 |
| Ang2VEGF-0284 | multiple substitution in CH1/CL interface | 56,3 | 61,1 |
| Ang2VEGF-0285 | multiple substitution in CH1/CL interface | 56,3 | 61,1 |
| Ang2VEGF-0286 | multiple substitution in CH1/CL interface | 56,3 | 61,6 |

The results from the proof-of-concept-experiments demonstrate that single amino acid substitutions in the CH1/CL interface are potent to strongly reduce side product formation of multispecific antibodies with a VH/VL domain crossover. Introduction of multiple amino acid substitutions were potent to completely suppress formation of the main Bence-Jones-type side product of multispecific antibodies with a VH/VL domain crossover. Yet all antibody constructs including amino acid substitutions could be stably produced in good yields and bound to their respective antigens in the nanomolar range.

### Example 2:

### Provision of tetravalent multispecific antibodies of the invention with a VH/VL domain crossover in one binding arm with charged amino acid substitutions in the CH1/CL interface

Tetravalent bispecific antibodies specifically binding to DR5 and FAP were generated, expressed and purified as described for the antibodies of Example 1. The tetravalent antibodies of this example comprise a monospecific core antibody that specifically binds to DR5. Two Fab fragments specifically binding to FAP are fused to the C-termini of the heavy chains of the core antibody as depicted in Figures 1A and IB. Antibodies were generated without amino acid substitutions in the CH1/CL interface (control), and with multiple charged amino acid substitutions in the CH1/CL interface.

The domain arrangement of the antibodies as well as amino acid substitutions in the CH1/CL interface ate indicated in **Table 7.**

**interface of tetravalent multispecific anti-DR5-FAP antibodies**

| | **VH/VL crossover on binding arm** | **CL DR5** | **CH1 DR5** | **CL FAP** | **CH1 FAP** |
|---|---|---|---|---|---|
| DR5TAA-0057 (control) | FAP | wt | wt | wt | wt |
| DR5TAA-0077 (control) | DR5 | wt | wt | wt | wt |
| DR5TAA-0078 (control) | DR5 (CH1-CL crossover ( instead of VH/VL) | wt | wt | wt | wt |
| DR5TAA-0081 (control) | FAP | wt | wt | wt | wt |
| DR5TAA-0082 | FAP | **E123R** | **K147E** | **Q124E** | wt |
| | | **Q124K** | **K213E** | | |
| DR5TAA-0083 | DR5 | **Q124E** | **wt** | **E123R** | K147E |
| | | | | **Q124K** | K213E |

The tetravalent multispecific antibodies were expressed using expression plasmids containing the nucleic acids encoding the amino acid sequences depicted in **Table 8.**

**Table 8: Amino acid sequences of tetravalent multispecific anti-DR5-FAP antibodies**

| **Antibody** | **LC** | **HC** | **LC** |
|---|---|---|---|
| | **DR5** | **DR5-FAP** | **FAP** |
| DR5TAA-0057 (control) | SEQ ID NO: 15 | SEQ ID NO: 16 | SEQ ID NO: 17 |
| DR5TAA-0077 (control) | SEQ ID NO: 18 | SEQ ID NO: 19 | SEQ ID NO: 20 |
| DR5TAA-0078 (control) | SEQ ID NO: 21 | SEQ ID NO: 22 | SEQ ID NO: 23 |
| DR5TAA-0081 (control) | SEQ ID NO: 24 | SEQ ID NO: 25 | SEQ ID NO: 26 |
| DR5TAA-0082 | SEQ ID NO: 27 | SEQ ID NO: 28 | SEQ ID NO: 29 |
| DR5TAA-0083 | SEQ ID NO: 30 | SEQ ID NO: 31 | SEQ ID NO: 32 |

The expressed multispecific antibodies were purified from the cell culture supernatant by a combination of Protein A affinity chromatography and size exclusion chromatography.

Aggregate content and production yields were assessed after Protein A affinity chromatography.

**Table 9: Aggregate content and production yield [mg/L supernatant; calculated] after Protein A purification of indicated tetravalent multispecific anti-DR5-FAP antibodies. The LMW content is the difference between 100 and the sum of monomer and aggregate content.**

| | **VH/VL crossover on binding arm** | **aggregates [%]** | **Monomer content by SEC [%]** | **yield [mg/L]** |
|---|---|---|---|---|
| DR5TAA-0057 | CH1-CL in FAP | 28 | 72 | n.a. |
| DR5TAA-0077 (control) | DR5 | 8 | 90 | 27,9 |
| DR5TAA-0081 (control) | FAP | 9 | 89 | 29,6 |
| DR5TAA-0082 | FAP | 1 | 99 | 30,4 |
| DR5TAA-0083 | DR5 | 1 | 99 | 25,4 |

Aggregate formation was reduced in the antibodies of the invention when compared to their respective wild type VH/VL domain crossover control antibodies.

In an additional purification step samples were further processed by preparative SEC. The table 10 summarizes the results of this step.

**Table 10: Summary of the preparative SEC purification. Yield as well as purity by CE-SDS and monomer content by analytical SEC were depicted.**

| Sample | Yield [mg/L] | Main peak content by CE-SDS [%] | Monomer content [%] |
|---|---|---|---|
| DR5TAA-0057- | 26,5 mg/l | 93 | 99 |
| DR5TAA-0077 | 9.2 mg/L | 96,27 | 100 |
| DR5TAA-0078 | 8.92 mg/L | 77,36 | 98,7 |
| DR5TAA-0081 | 18,8mg/l | 96 | 99 |
| DR5TAA-0082 | 19,8 mg/l | 97 | 99 |
| DR5TAA-0083 | 15,3 mg/l | 98 | 99 |

**Table 11: Aggregation onset temperature (Tagg) of indicated antibodies**

| **Sample** | **VH/VL crossover on binding arm** | **amino acid substitution** | **T_{agg} DLS (°C)** |
|---|---|---|---|
| DR5TAA-0057 (control) | FAP | none (wild type, control) | 63 |
| DR5TAA-0077 (control) | DR5 | none (wild type, control) | 62 |
| DR5TAA-0078 (control) | DR5 (CH1-CL crossover instead of VH/VL) | none (wild type, control) | 62 |
| DR5TAA-0081 (control) | FAP | none (wild type, control) | 59 |
| **DR5TAA-0082** | **FAP** | **multiple substitution in CH1/CL interface** | **60** |
| **DR5TAA-0083** | **DR5** | **multiple substitution in CH1/CL interface** | **62** |

All expressed DR5FAP Crossmab were stable and possess a similar aggregation onset temperature (except DR5TAA-0081).

The expected primary structures of the antibodies were analyzed via MS as described in Example 1.

The main side product detectable when producing the corresponding wild type VH/VL domain crossover tetravalent anti-DR5-FAP antibodies is a semi-functional antibody comprising three FAP-light chains and only one DR5-light chain. For illustration purposes, the desired molecules and the respective main side products for wild type antibodies DR5TAA-0077 and DR5TAA-0081 are shown in **Figures 3A** **and** **B** (left and middle image, respectively).

MS analysis of the tetravalent antibodies of the invention revealed that the main side product fraction could be strongly reduced by introducing charged amino acid residues in the CH1/CL interface of the VH/VL domain crossover tetravalent anti-DR5-FAP antibodies.

**Table 12: Fraction of main side product (antibody with three FAP-light chains and one DR5-light chain) and byproduct formed when expressing tetravalent multispecific anti-DR5-FAP antibodies as determined by mass spectrometry***

| **Sample** | **VH/VL crossover on binding arm** | **amino acid substitution** | **Main product DR5FAP CrossMAb [%]** | **[%] Main side product by MS (three FAP-light chains and one DR5-light chain)** | **Byproduct with mispaired LCs: 3x LC(DR5) +1 LC (FAP)** |
|---|---|---|---|---|---|
| DR5TAA-0057 **(control for VH-VL exchange)** | FAP | none (wild type, control) | 90 | 10 | not detected |
| DR5TAA-0077 **(control for VH-VL exchange)** | DR5 | none (wild type, control) | **89** | **9%** | **2%** |
| DR5TAA-0078 **control for CH1-CL exchange)** | CH1-Ck in DR5 | none (wild type, control) | 88 | 7 | 5 |
| DR5TAA-0081 **(control for VH-VL exchange)** | FAP | none (wild type, control) | 85 | 15% | not detected |
| DR5TAA-0082 | FAP | **multiple substitution in CH1/CL interface** | **98** | **2%** | not detected |
| DR5TAA-0083 | DR5 | **multiple substitution in CH1/CL interface** | **100** | **not detected** | not detected |

| | | | | | |
|---|---|---|---|---|---|
| *Please note: quantification means quantitative estimation as ionization efficiency of different molecules may differ by a specific , non-determined factor. As molecule sizes of DR5FAP and its quantified byproducts are similar here, the quantification can be used to compare the integrity of the different DR5FAP molecules | | | | | |

The table above indicates that the introduced charges reduce the mispairing of light chains, compared to the variant without charged residues. Dependent on the charged residues selected the mispairing of light chains can be reduced to the limit of detection. This table clearly shows, that the used charged residues Q124E in the FAP constant light chain, E123R/Q124K in the DR5 constant light (CL) chain, K147E; K213E in CH1 of DR5 can avoid any side product formation. It is beneficial to place the two charge exchanges in the non crossed domains and the single additional charge in the crossed domain.

### Example 3:

### Assessment of independent DR5- and FAP-binding to the Crossmab

Independent binding of DR5 and FAP to the DR5TAA CrossMAb with different domain exchanges as well as with different additionally introduced charged residues is shown. Therefore a <Fc> capturing IgG is immobilized on the chip surface and the different DR5TAA CrossMAbs is captured. In the experiment the targets DR5 and FAP were either added individually or simultaneously to the captured CrossMAb.

**Table 13: Summary of individual and simultaneous binding of FAP and DR5 to different tetravalent DR5FAP CrossMAbs. The table indicates the RUs for each binding event and the combined binding event as well as the ratio between measured and expected signal intensity.**

| **Construct** | **DR5 signal [RU max]** | **FAP signal [RU max]** | **expected signal DR5 + FAP** | **measured signal DR5 + FAP** | **ratio [%]** |
|---|---|---|---|---|---|
| DR5TAA-0057 | 38,7 | 40,6 | 79,3 | 78,3 | 99 |
| DR5TAA-0057 | 37,1 | 40,8 | 77,9 | 76,7 | 98 |
| DR5TAA-0077 | 33,0 | 41,9 | 74,9 | 73,1 | 98 |
| DR5TAA-0078 | 27,1 | 38,4 | 65,5 | 63,6 | 97 |
| DR5TAA-0057 | 32.2 | 19.7 | 52 | 49 | 94 |
| DR5TAA-0081 | 26.7 | 7.2 | 34 | 33 | 97 |
| DR5TAA-0082 | 30.5 | 7.4 | 38 | 35 | 92 |
| DR5TAA-0083 | 26.9 | 18.5 | 45 | 42 | 93 |

The result of this experiment (Table shows that the ratio between the experimentally measured signal intensity of the DR5 and FAP mixture is very similar to the theoretically expected maximal target occupancy (all above 90%). This indicates that the DR5TAA CrossMAbs with different domain exchanges and charged residues are capable of simultaneous binding, independent of the applied molecular design.

### Example 4:

### Assessment of simultaneous DR5- and FAP-binding to the Crossmab

DR5 is immobilized on the chip surface and the DR5TAA CrossMAb is added in the first binding step. In a second step the ligand FAP is added. SPR analysis of the DR5TAA samples revealed that all variants of the DR5TAA CrossMAbs independent of the position of the domain exchange and independent of the introduced charged amino acids, are capable of simultaneous binding to both targets. The results of this experiment (SPR sensorgrams) were depicted in Figures 5A, B and C and show clear simultaneous DR5- and FAP-binding of all CrossMAbs.

### Example 5:

### Production and expression of multispecific antibodies which bind to PD1 and TIM3 with VH/VL domain exchange/replacement (2+2 CrossMAbVh-VL) in two binding arms and with single charged amino acid substitutions in the CH1/CL interfaces

In an example multispecific antibodies which binds to human PD1 and human TIM3 were generated as described in the general methods section by classical molecular biology techniques and were expressed transiently in 293F of Expi293F cells as described above. The multispecific 2+2 *CrossMAb^{VH-VL}* antibodies without charges are described also in WO 2010/145792. The multispecific antibodies were expressed using expression plasmids containing the nucleic acids encoding the amino acid sequences depicted in Table 14.

**Table 14: Amino acid sequences of light chains (LC) and heavy chains (HC), with VH/VL domain exchange/replacement (2+2 CrossMAb^{Vh-VL})**

| 2+2 Antibody | HC | LC1 | LC2 |
|---|---|---|---|
| PD1TIM3_0358 | SEQ ID NO:37 | SEQ ID NO:38 | SEQ ID NO:39 |
| PD1TIM3 0359 | SEQ ID NO:40 | SEQ ID NO:41 | SEQ ID NO:42 |
| PD1TIM3_0321 | SEQ ID NO:43 | SEQ ID NO:44 | SEQ ID NO:45 |

As further examples tetravalent bispecific PD1TIM3 antibodies using the same constructs/IgG backbones as in table 14 but with i) the humanized variable domains of SEQ ID NO:46 (VH anti-PD1) and SEQ ID NO:47 (VL anti-PD1) for the PD1 binding arms and humanized variable domains of SEQ ID NO:48 (VH anti-TIM3) and SEQ ID NO:49 (VL anti-TIM3) for the TIM3 binding arms and with ii) the humanized variable domains of SEQ ID NO:46 (VH anti-PD1) and SEQ ID NO:47 (VL anti-PD1) for the PD1 binding arms and humanized variable domains of SEQ ID NO:50 (VH anti-TIM3) and SEQ ID NO:51 (VL anti-TIM3) for the TIM3 binding arms are expressed.

### Example 6:

### Purification and characterization of multispecific antibodies which bind to PD1 and TIM3

The multispecific antibodies expressed above were purified from the supernatant by a combination of Protein A affinity chromatography and size exclusion chromatography. All multispecific antibodies can be produced in good yields and are stable. The obtained products were characterized for identity by mass spectrometry and analytical properties such as purity by SDS-PAGE, monomer content and stability

### Mass spectrometry

The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Stability of multispecific antibodies

In order to assess stability of the antibody constructs, thermal stability as well as aggregation onset temperatures were assessed according to the following procedure. Samples of the indicated antibodies were prepared at a concentration of 1 mg/mL in 20 mM Histidine/Histidine chloride, 140 mM NaCl, pH 6.0, transferred into a 10 µL micro-cuvette array and static light scattering data as well as fluorescence data upon excitation with a 266 nm laser were recorded with an Optim1000 instrument (Avacta Inc.), while the samples were heated at a rate of 0.1 °C/min from 25°C to 90°C.

The aggregation onset temperature (T_{agg}) is defined as the temperature at which the scattered light intensity starts to increase. The melting temperature (Tₘ) is defined as the inflection point in a fluorescence intensity vs. wavelength graph.

### Example 7:

### Characterization of 2+2 anti-PD1-TIM3 multispecific antibodies

### Binding Elisa

### ELISA for hu PD1

Nunc maxisorp streptavidin coated plates (MicroCoat #11974998001) were coated with 25 µl/well biotinylated PD1-ECD-AviHis at a concentration of 500 ng/ml and incubated at 4°C over night. After washing (3x90 µl/well with PBST-buffer) 25 µl anti PD1 antibody samples were added in increasing concentrations and incubated 1h at RT. After washing (3x90 µl/well with PBST-buffer) 25 µl/well goat-anti-human H+L-POD (JIR, JIR109-036-098) was added in 1:5000 dilution and incubated at RT for 1 h on a shaker. After washing (3x90 µl/well with PBST-buffer) 25 µl/well TMB substrate (Roche, 11835033001) was added and incubated until OD 2 - 3. Measurement took place at 370/492 nm.

### ELISA for hu Tim3

Nunc maxisorp streptavidin coated plates (MicroCoat #11974998001) were coated with 25 µl/well biotinylated Tim3-ECD-AviHis at a concentration of 60 ng/ml and incubated at 4°C overnight. After washing (3x90 µl/well with PBST-buffer) 25 µl anti PD1 antibody samples were added in increasing concentrations and incubated 1h at RT. After washing (3x90 µl/well with PBST-buffer) 25µl/well goat-anti-human H+L-POD (JIR, JIR109-036-098) was added in 1:5000 dilution and incubated at RT for 1h on a shaker. After washing (3x90 µl/well with PBST-buffer) 25 µl/well TMB substrate (Roche, 11835033001) was added and incubated until OD 2 - 3. Measurement took place at 370/492 nm.
ELISA results are listed as EC50 values [nM] in **Table 15.**

**Table 15: Biochemical- and Cell-binding of anti-PD1-TIM3 bispecific antibodies (ELISA)**

| **Antibody** | **Sample** | **huPD1 EC50 [nM]** | **huTIM3 EC50 [nM]** |
|---|---|---|---|
| PD1 IgG (bivalent) | Chimeric PD1-0103 | 0,12 | no binding |
| TIM3 IgG (bivalent) | Chimeric TIM3-0018 | no binding | 0,15 |
| 2+2 (tetravalent) | 2+2 PDITIM3-0359 | 0.09 | 0.11 |
| TIM3 IgG (bivalent) | Chimeric TIM3-0028 | no binding | 0.29 upper plateau at 66% |
| 2+2 (tetravalent) | 2+2 PD1TIM3-0358 | 0,08 | 0.19 upper plateau at 65% |

Avid binding (i.e. binding with both arms) can be detected for antibodies that are bivalent for Tim3 (Chimeric TIM3-0018, 2+2 PD1TIM3-0359, Chimeric TIM3-0028, 2+2 PD1TIM3-0358). Avidity effects were not detected for PD1-binding. EC50 values are comparable for bivalent and tetravalent formats.

### Binding Biacore

### Antigen binding properties of multispecific antibodies which bind to PD1 and TIM3

Binding of the multispecific antibodies to their respective target antigens, i.e. PD 1 and TIM3, was assessed by Biacore®.
Results are indicated in **Table 16.**

**Table 16: Affinity for PD1/Tim3 Bispecific Antibodies**

| **Sample** | **PD1-arm KD [nM]** | **Tim3-arm KD [nM]** |
|---|---|---|
| PD1TIM3-0358 (2+2) | n.d. | 2.2 |
| PD1TIM3-0359 (2+2) | 1.9 | 0.3 |

All tested antibodies specifically bind to both targets, PD1 and TIM3.

## Claims

1. A tetravalent multispecific antibody, comprising
a) one core antibody formed by a full length antibody, the full length antibody comprising two Fab fragments specifically binding to a first antigen, and
b) two additional Fab fragments, wherein said additional Fab fragments are fused both either at the C-termini or the N-termini of the heavy chains of the core antibody,
wherein the additional Fab fragments specifically bind to a second antigen,
i) wherein either
- the Fab fragments specifically binding to the first antigen, or
- the Fab fragments specifically binding to the second antigen comprise a domain crossover such that the variable heavy chain domain (VH) and the variable light chain domain (VL) are replaced by each other, and
ii) wherein either
- the Fab fragments specifically binding to the first antigen, or
- the Fab fragments specifically binding to the second antigen comprise the following amino acid substitutions in the constant light chain domain (CL) and the constant heavy chain domain 1 (CH1):
- the amino acid at position 124 in the CL domain is substituted by K, R or H (numbering according to Kabat), and
- the amino acid at position 147 or 213 in the CH1 domain is substituted by E or D (numbering according to Kabat EU index).

2. A tetravalent multispecific antibody, comprising
a) one core antibody formed by a full length antibody, the full length antibody comprising two Fab fragments specifically binding to a first antigen and a second antigen, respectively, and
b) two additional Fab fragments, wherein said additional Fab fragments are fused both either at the C-termini or the N-termini of the heavy chains of the core antibody,
wherein the additional Fab fragment fused to a heavy chain exhibits the same antigen binding specificity than the Fab fragment of the core antibody arranged on said heavy chain,
i) wherein either
- the Fab fragments specifically binding to the first antigen, or
- the Fab fragments specifically binding to the second antigen comprise a domain crossover such that the variable heavy chain domain (VH) and the variable light chain domain (VL) are replaced by each other, and
ii) wherein either
- the Fab fragments specifically binding to the first antigen, or
- the Fab fragments specifically binding to the second antigen comprise the following amino acid substitutions in the constant light chain domain (CL) and the constant heavy chain domain 1 (CH1):
- the amino acid at position 124 in the CL domain is substituted by K, R or H (numbering according to Kabat), and
- the amino acid at position 147 or 213 in the CH1 domain is substituted by E or D (numbering according to Kabat EU index).

3. The antibody according to claim 1 or 2, wherein the one or two Fab fragments that do not comprise the domain crossover of i) comprise the amino acid substitutions of ii).

4. The antibody according to any one of claims 1 to 3, wherein in the Fab fragments comprising the amino acid substitutions of ii)
- the amino acids at position 123 and 124 in the CL domain are substituted independently from each other by K, R or H (numbering according to Kabat).

5. The antibody according to any one of claims 1 to 4, wherein in the Fab fragments comprising the amino acid substitutions of ii)
- the amino acids at position 123 and 124 in the CL domain are substituted independently from each other by K, R or H (numbering according to Kabat), and
- the amino acids at positions 147 and 213 in the CH1 domain are substituted independently from each other by E or D (numbering according to Kabat EU index).

6. The antibody according to any one of claims 1 to 5, wherein the Fab fragments that do not comprise the amino acid substitutions as defined under ii) comprise the following amino acid substitution in the CL domain
- the amino acid at position 124 is substituted by E or D.

7. The antibody according to any one of claims 1 to 6, wherein the Fab fragments comprising the amino acid substitutions of ii) comprise a light chain constant domain CL of kappa isotype.

8. The antibody according to any one of claims 1 to 7, wherein the additional Fab fragments of b) are fused via a peptide connector to the heavy chains of the core antibody.

9. The antibody according to any one of claims 1 to 8, wherein the multispecific antibody comprises two constant heavy chain domains 3 (CH3), which are altered to promote heterodimerization by:
- generation of a protuberance in one of the CH3 domains by substituting at least one original amino acid residue by an amino acid residue having a larger side chain volume than the original amino acid residue, and generation of a cavity in the other one of the CH3 domains by substituting at least one original amino acid residue by an amino acid residue having a smaller side chain volume than the original amino acid residue, such that the protuberance generated in one of the CH3 domains is positionable in the cavity generated in the other one of the CH3 domains; or
- by substituting at least one original amino acid residue in one of the CH3 domains by a positively charged amino acid, and substituting at least one original amino acid residue in the other one of the CH3 domains by a negatively charged amino acid.

10. The antibody according to any one of claims 1 to 9, wherein the multispecific antibody comprises two CH3 domains, which are altered to promote heterodimerization by introduction of at least one cysteine residue in each CH3 domain such that a disulfide bond is formed between the CH3 domains.

11. An isolated nucleic acid encoding the antibody according to any one of claims 1 to 10.

12. An expression vector comprising the nucleic acid according to claim 11.

13. A host cell comprising the nucleic acid according to claim 11.

14. A pharmaceutical or diagnostic composition comprising the multispecific antibody according to any one of claims 1 to 10.

15. A method of producing a multispecific antibody, comprising culturing a host cell according to claim 13 so that the multispecific antibody is produced.


SEQUENCE LISTING

<110>  F. Hoffmann-La Roche AG

<120>  Tetravalent multispecific antibodies

<130>  33107-EP

<160>  51

<170>  PatentIn version 3.5

<210>  1
<211>  215
<212>  PRT
<213>  Artificial

<220>
<223>  light chain (LC) <Ang-2> wild type (wt)

<400>  1

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu

165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  2
<211>  457
<212>  PRT
<213>  Artificial

<220>
<223>  heavy chain (HC) <Ang-2> wild type (wt)

<400>  2

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe

405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455

<210>  3
<211>  437
<212>  PRT
<213>  Artificial

<220>
<223>  heavy chain (HC) <VEGF> with VH-VL exchange wild type (wt)

<400>  3

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys Asp Lys Thr His Thr Cys Pro Pro Cys Pro Ala Pro
    210                 215                 220

Glu Leu Leu Gly Gly Pro Ser Val Phe Leu Phe Pro Pro Lys Pro Lys
225                 230                 235                 240

Asp Thr Leu Met Ile Ser Arg Thr Pro Glu Val Thr Cys Val Val Val
                245                 250                 255

Asp Val Ser His Glu Asp Pro Glu Val Lys Phe Asn Trp Tyr Val Asp
            260                 265                 270

Gly Val Glu Val His Asn Ala Lys Thr Lys Pro Arg Glu Glu Gln Tyr
        275                 280                 285

Asn Ser Thr Tyr Arg Val Val Ser Val Leu Thr Val Leu His Gln Asp
    290                 295                 300

Trp Leu Asn Gly Lys Glu Tyr Lys Cys Lys Val Ser Asn Lys Ala Leu
305                 310                 315                 320

Pro Ala Pro Ile Glu Lys Thr Ile Ser Lys Ala Lys Gly Gln Pro Arg
                325                 330                 335

Glu Pro Gln Val Cys Thr Leu Pro Pro Ser Arg Asp Glu Leu Thr Lys
            340                 345                 350

Asn Gln Val Ser Leu Ser Cys Ala Val Lys Gly Phe Tyr Pro Ser Asp
        355                 360                 365

Ile Ala Val Glu Trp Glu Ser Asn Gly Gln Pro Glu Asn Asn Tyr Lys
    370                 375                 380

Thr Thr Pro Pro Val Leu Asp Ser Asp Gly Ser Phe Phe Leu Val Ser
385                 390                 395                 400

Lys Leu Thr Val Asp Lys Ser Arg Trp Gln Gln Gly Asn Val Phe Ser

405                 410                 415

Cys Ser Val Met His Glu Ala Leu His Asn His Tyr Thr Gln Lys Ser
            420                 425                 430

Leu Ser Leu Ser Pro
        435

<210>  4
<211>  230
<212>  PRT
<213>  Artificial

<220>
<223>  light chain (LC) <VEGF> with VH-VL exchange wild type (wt)

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Gln Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys
225                 230

<210>  5
<211>  215
<212>  PRT
<213>  Artificial

<220>
<223>  light chain (LC) <Ang-2> with Q124K substitution

<400>  5

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Glu Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  6
<211>  457
<212>  PRT
<213>  Artificial

<220>
<223>  heavy chain (HC) <Ang-2> with K147E substitution

<400>  6

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455

<210>  7
<211>  457
<212>  PRT
<213>  Artificial

<220>
<223>  heavy chain (HC) <Ang-2> with K213E substitution

<400>  7

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455


<210>  8
<211>  215
<212>  PRT
<213>  Artificial

<220>
<223>  light chain (LC) <Ang-2> with E123K substitution

<400>  8

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  9
<211>  215
<212>  PRT
<213>  Artificial

<220>
<223>  light chain (LC) <Ang-2> with Q124K substitution and E123K
       substitution

<400>  9

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  10
<211>  457
<212>  PRT
<213>  Artificial

<220>
<223>  heavy chain (HC) <Ang-2> with K147E substitution and K213E
       substitution

<400>  10

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455

<210>  11
<211>  215
<212>  PRT
<213>  Artificial

<220>
<223>  light chain (LC) <Ang-2> with Q124R substitution and E123K
       substitution

<400>  11

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Arg Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 12
<211> 230
<212> PRT
<213> Artificial

<220>
<223> light chain (LC) <VEGF> with Q124E substitution

<400> 12

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Glu Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys
225                 230

<210>  13
<211>  214
<212>  PRT
<213>  Artificial

<220>
<223>  light chain (LC) <Ang-2> with E124K substitution and E123K
       substitution

<400>  13

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Gly Gln Pro Lys
            100                 105                 110

Ala Ala Pro Ser Val Thr Leu Phe Pro Pro Ser Ser Lys Lys Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  14
<211>  457
<212>  PRT
<213>  Artificial

<220>
<223>  heavy chain (HC) <Ang-2> with K147E substitution and K213D
       substitution

<400>  14

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Asp Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455


<210>  15
<211>  215
<212>  PRT
<213>  Artificial

<220>
<223>  DR5TAA-0057 LC1 (Vl-CL, non crossed, DR5, N-term)

<400>  15

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  16
<211>  689
<212>  PRT
<213>  Artificial

<220>
<223>  DR5TAA-0057 HC (DR5-Vh-CH1 \205G4S-FAP-Vh-CL)

<400>  16

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Gly Gly
        435                 440                 445

Gly Gly Ser Gly Gly Gly Gly Ser Gly Gly Gly Gly Ser Gly Gly Gly
    450                 455                 460

Gly Ser Glu Val Gln Leu Leu Glu Ser Gly Gly Gly Leu Val Gln Pro
465                 470                 475                 480

Gly Gly Ser Leu Arg Leu Ser Cys Ala Ala Ser Gly Phe Thr Phe Ser
                485                 490                 495

Ser His Ala Met Ser Trp Val Arg Gln Ala Pro Gly Lys Gly Leu Glu
            500                 505                 510

Trp Val Ser Ala Ile Trp Ala Ser Gly Glu Gln Tyr Tyr Ala Asp Ser
        515                 520                 525

Val Lys Gly Arg Phe Thr Ile Ser Arg Asp Asn Ser Lys Asn Thr Leu
    530                 535                 540

Tyr Leu Gln Met Asn Ser Leu Arg Ala Glu Asp Thr Ala Val Tyr Tyr
545                 550                 555                 560

Cys Ala Lys Gly Trp Leu Gly Asn Phe Asp Tyr Trp Gly Gln Gly Thr
                565                 570                 575

Leu Val Thr Val Ser Ser Ala Ser Val Ala Ala Pro Ser Val Phe Ile
            580                 585                 590

Phe Pro Pro Ser Asp Glu Gln Leu Lys Ser Gly Thr Ala Ser Val Val
        595                 600                 605

Cys Leu Leu Asn Asn Phe Tyr Pro Arg Glu Ala Lys Val Gln Trp Lys
    610                 615                 620

Val Asp Asn Ala Leu Gln Ser Gly Asn Ser Gln Glu Ser Val Thr Glu
625                 630                 635                 640

Gln Asp Ser Lys Asp Ser Thr Tyr Ser Leu Ser Ser Thr Leu Thr Leu
                645                 650                 655

Ser Lys Ala Asp Tyr Glu Lys His Lys Val Tyr Ala Cys Glu Val Thr
            660                 665                 670

His Gln Gly Leu Ser Ser Pro Val Thr Lys Ser Phe Asn Arg Gly Glu
        675                 680                 685

Cys

<210>  17
<211>  214
<212>  PRT
<213>  Artificial

<220>
<223>  DR5TAA-0057 LC2 (FAP, Vl-CH1, crossed, C-Term)

<400>  17

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
        195                 200                 205

Glu Pro Lys Ser Cys Asp
    210

<210>  18
<211>  224
<212>  PRT
<213>  Artificial

<220>
<223>  DR5TAA-0077 LC1 (DR5, crossed, N-Term, Vh-CL)

<400>  18

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

                20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Val Ala Ala Pro Ser Val Phe Ile Phe
        115                 120                 125

Pro Pro Ser Asp Glu Gln Leu Lys Ser Gly Thr Ala Ser Val Val Cys
    130                 135                 140

Leu Leu Asn Asn Phe Tyr Pro Arg Glu Ala Lys Val Gln Trp Lys Val
145                 150                 155                 160

Asp Asn Ala Leu Gln Ser Gly Asn Ser Gln Glu Ser Val Thr Glu Gln
                165                 170                 175

Asp Ser Lys Asp Ser Thr Tyr Ser Leu Ser Ser Thr Leu Thr Leu Ser
            180                 185                 190

Lys Ala Asp Tyr Glu Lys His Lys Val Tyr Ala Cys Glu Val Thr His
        195                 200                 205

Gln Gly Leu Ser Ser Pro Val Thr Lys Ser Phe Asn Arg Gly Glu Cys
    210                 215                 220

<210>  19
<211>  681
<212>  PRT
<213>  Artificial

<220>
<223>  DR5TAA-0077 HC (DR5-Vl-CH1 G4S-FAP-Vh-CH1)

<400>  19

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
        195                 200                 205

Glu Pro Lys Ser Cys Asp Lys Thr His Thr Cys Pro Pro Cys Pro Ala
    210                 215                 220

Pro Glu Leu Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser His Glu Asp Pro Glu Val Lys Phe Asn Trp Tyr Val

260                 265                 270

Asp Gly Val Glu Val His Asn Ala Lys Thr Lys Pro Arg Glu Glu Gln
        275                 280                 285

Tyr Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Ala
305                 310                 315                 320

Leu Pro Ala Pro Ile Glu Lys Thr Ile Ser Lys Ala Lys Gly Gln Pro
                325                 330                 335

Arg Glu Pro Gln Val Tyr Thr Leu Pro Pro Ser Arg Asp Glu Leu Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Lys Leu Thr Val Asp Lys Ser Arg Trp Gln Gln Gly Asn Val Phe
                405                 410                 415

Ser Cys Ser Val Met His Glu Ala Leu His Asn His Tyr Thr Gln Lys
            420                 425                 430

Ser Leu Ser Leu Ser Pro Gly Lys Ser Gly Gly Gly Gly Ser Gly Gly
        435                 440                 445

Gly Gly Ser Gly Gly Gly Gly Ser Gly Gly Gly Gly Ser Glu Val Gln
    450                 455                 460

Leu Leu Glu Ser Gly Gly Gly Leu Val Gln Pro Gly Gly Ser Leu Arg
465                 470                 475                 480

Leu Ser Cys Ala Ala Ser Gly Phe Thr Phe Ser Ser His Ala Met Ser
                485                 490                 495

Trp Val Arg Gln Ala Pro Gly Lys Gly Leu Glu Trp Val Ser Ala Ile
            500                 505                 510

Trp Ala Ser Gly Glu Gln Tyr Tyr Ala Asp Ser Val Lys Gly Arg Phe
        515                 520                 525

Thr Ile Ser Arg Asp Asn Ser Lys Asn Thr Leu Tyr Leu Gln Met Asn
    530                 535                 540

Ser Leu Arg Ala Glu Asp Thr Ala Val Tyr Tyr Cys Ala Lys Gly Trp
545                 550                 555                 560

Leu Gly Asn Phe Asp Tyr Trp Gly Gln Gly Thr Leu Val Thr Val Ser
                565                 570                 575

Ser Ala Ser Thr Lys Gly Pro Ser Val Phe Pro Leu Ala Pro Ser Ser
            580                 585                 590

Lys Ser Thr Ser Gly Gly Thr Ala Ala Leu Gly Cys Leu Val Lys Asp
        595                 600                 605

Tyr Phe Pro Glu Pro Val Thr Val Ser Trp Asn Ser Gly Ala Leu Thr
    610                 615                 620

Ser Gly Val His Thr Phe Pro Ala Val Leu Gln Ser Ser Gly Leu Tyr
625                 630                 635                 640

Ser Leu Ser Ser Val Val Thr Val Pro Ser Ser Ser Leu Gly Thr Gln
                645                 650                 655

Thr Tyr Ile Cys Asn Val Asn His Lys Pro Ser Asn Thr Lys Val Asp
            660                 665                 670

Lys Lys Val Glu Pro Lys Ser Cys Asp
        675                 680

<210>  20
<211>  215
<212>  PRT
<213>  Artificial

<220>
<223>  DR5TAA-0077 LC2 (FAP, Vl-CL, C-term)

<400>  20

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu

35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  21
<211>  214
<212>  PRT
<213>  Artificial

<220>
<223>  DR5TAA-0078 LC1 (DR5 crossed, Vl-CH1, N-term)

<400>  21

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
        195                 200                 205

Glu Pro Lys Ser Cys Asp
    210

<210> 22
<211> 690
<212> PRT
<213> Artificial

<220>
<223> DR5TAA-0078 HC (DR5-Vh-CL G4S-FAP-Vh-CH1)

<400> 22

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Val Ala Ala Pro Ser Val Phe Ile Phe
        115                 120                 125

Pro Pro Ser Asp Glu Gln Leu Lys Ser Gly Thr Ala Ser Val Val Cys
    130                 135                 140

Leu Leu Asn Asn Phe Tyr Pro Arg Glu Ala Lys Val Gln Trp Lys Val
145                 150                 155                 160

Asp Asn Ala Leu Gln Ser Gly Asn Ser Gln Glu Ser Val Thr Glu Gln
                165                 170                 175

Asp Ser Lys Asp Ser Thr Tyr Ser Leu Ser Ser Thr Leu Thr Leu Ser
            180                 185                 190

Lys Ala Asp Tyr Glu Lys His Lys Val Tyr Ala Cys Glu Val Thr His
        195                 200                 205

Gln Gly Leu Ser Ser Pro Val Thr Lys Ser Phe Asn Arg Gly Glu Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Gly Gly Gly Gly Ser Gly Gly Gly Gly Ser Gly Gly Gly Gly
    450                 455                 460

Ser Gly Gly Gly Gly Ser Glu Val Gln Leu Leu Glu Ser Gly Gly Gly
465                 470                 475                 480

Leu Val Gln Pro Gly Gly Ser Leu Arg Leu Ser Cys Ala Ala Ser Gly
                485                 490                 495

Phe Thr Phe Ser Ser His Ala Met Ser Trp Val Arg Gln Ala Pro Gly
            500                 505                 510

Lys Gly Leu Glu Trp Val Ser Ala Ile Trp Ala Ser Gly Glu Gln Tyr

515                 520                 525

Tyr Ala Asp Ser Val Lys Gly Arg Phe Thr Ile Ser Arg Asp Asn Ser
    530                 535                 540

Lys Asn Thr Leu Tyr Leu Gln Met Asn Ser Leu Arg Ala Glu Asp Thr
545                 550                 555                 560

Ala Val Tyr Tyr Cys Ala Lys Gly Trp Leu Gly Asn Phe Asp Tyr Trp
                565                 570                 575

Gly Gln Gly Thr Leu Val Thr Val Ser Ser Ala Ser Thr Lys Gly Pro
            580                 585                 590

Ser Val Phe Pro Leu Ala Pro Ser Ser Lys Ser Thr Ser Gly Gly Thr
        595                 600                 605

Ala Ala Leu Gly Cys Leu Val Lys Asp Tyr Phe Pro Glu Pro Val Thr
    610                 615                 620

Val Ser Trp Asn Ser Gly Ala Leu Thr Ser Gly Val His Thr Phe Pro
625                 630                 635                 640

Ala Val Leu Gln Ser Ser Gly Leu Tyr Ser Leu Ser Ser Val Val Thr
                645                 650                 655

Val Pro Ser Ser Ser Leu Gly Thr Gln Thr Tyr Ile Cys Asn Val Asn
            660                 665                 670

His Lys Pro Ser Asn Thr Lys Val Asp Lys Lys Val Glu Pro Lys Ser
        675                 680                 685

Cys Asp
    690

<210>  23
<211>  215
<212>  PRT
<213>  Artificial

<220>
<223>  DR5TAA-0078 LC2 (FAP non crossed, C-term, VL-CL)

<400>  23

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  24
<211>  223
<212>  PRT
<213>  Artificial

<220>
<223>  DR5TAA-0081 LC1 (FAP crossed, VH-CL)

<400>  24

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Val Ala Ala Pro Ser Val Phe Ile Phe Pro
        115                 120                 125

Pro Ser Asp Glu Gln Leu Lys Ser Gly Thr Ala Ser Val Val Cys Leu
    130                 135                 140

Leu Asn Asn Phe Tyr Pro Arg Glu Ala Lys Val Gln Trp Lys Val Asp
145                 150                 155                 160

Asn Ala Leu Gln Ser Gly Asn Ser Gln Glu Ser Val Thr Glu Gln Asp
                165                 170                 175

Ser Lys Asp Ser Thr Tyr Ser Leu Ser Ser Thr Leu Thr Leu Ser Lys
            180                 185                 190

Ala Asp Tyr Glu Lys His Lys Val Tyr Ala Cys Glu Val Thr His Gln
        195                 200                 205

Gly Leu Ser Ser Pro Val Thr Lys Ser Phe Asn Arg Gly Glu Cys
    210                 215                 220

<210>  25
<211>  680
<212>  PRT
<213>  Artificial

<220>
<223>  DR5TAA-0081 HC (DR5- VH-CH1  G4S-FAP-VL-CH1)

<400>  25

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
        260                 265                 270

Val Lys Phe Asn Trp Tyr Val Asp Gly Val Glu Val His Asn Ala Lys
    275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Gly Gly
        435                 440                 445

Gly Gly Ser Gly Gly Gly Gly Ser Gly Gly Gly Gly Ser Gly Gly Gly
    450                 455                 460

Gly Ser Glu Ile Val Leu Thr Gln Ser Pro Gly Thr Leu Ser Leu Ser
465                 470                 475                 480

Pro Gly Glu Arg Ala Thr Leu Ser Cys Arg Ala Ser Gln Ser Val Ser
                485                 490                 495

Arg Ser Tyr Leu Ala Trp Tyr Gln Gln Lys Pro Gly Gln Ala Pro Arg
            500                 505                 510

Leu Leu Ile Ile Gly Ala Ser Thr Arg Ala Thr Gly Ile Pro Asp Arg
        515                 520                 525

Phe Ser Gly Ser Gly Ser Gly Thr Asp Phe Thr Leu Thr Ile Ser Arg
    530                 535                 540

Leu Glu Pro Glu Asp Phe Ala Val Tyr Tyr Cys Gln Gln Gly Gln Val
545                 550                 555                 560

Ile Pro Pro Thr Phe Gly Gln Gly Thr Lys Val Glu Ile Lys Ser Ser
                565                 570                 575

Ala Ser Thr Lys Gly Pro Ser Val Phe Pro Leu Ala Pro Ser Ser Lys
            580                 585                 590

Ser Thr Ser Gly Gly Thr Ala Ala Leu Gly Cys Leu Val Lys Asp Tyr
        595                 600                 605

Phe Pro Glu Pro Val Thr Val Ser Trp Asn Ser Gly Ala Leu Thr Ser
    610                 615                 620

Gly Val His Thr Phe Pro Ala Val Leu Gln Ser Ser Gly Leu Tyr Ser
625                 630                 635                 640

Leu Ser Ser Val Val Thr Val Pro Ser Ser Ser Leu Gly Thr Gln Thr
                645                 650                 655

Tyr Ile Cys Asn Val Asn His Lys Pro Ser Asn Thr Lys Val Asp Lys
            660                 665                 670

Lys Val Glu Pro Lys Ser Cys Asp
        675                 680

<210>  26
<211>  215
<212>  PRT
<213>  Artificial

<220>
<223>  DR5TAA-0081 LC2 (DR5, VL-CL)

<400>  26

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  27
<211>  215
<212>  PRT
<213>  Artificial

<220>
<223>  DR5TAA-0082 LC1 (DR5-VL-CL)

<400>  27

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Arg Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  28
<211>  680
<212>  PRT
<213>  Artificial

<220>
<223>  DR5TAA-0082 HC (DR5-Vh-CH1  G4S-FAP-Vl-CH1)

<400>  28

Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Glu Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Glu Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
        260                 265                 270

Val Lys Phe Asn Trp Tyr Val Asp Gly Val Glu Val His Asn Ala Lys
    275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Tyr Thr Leu Pro
        340                 345                 350

Pro Ser Arg Asp Glu Leu Thr Lys Asn Gln Val Ser Leu Thr Cys Leu
    355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
        420                 425                 430

His Asn His Tyr Thr Gln Lys Ser Leu Ser Leu Ser Pro Gly Gly Gly
    435                 440                 445

Gly Gly Ser Gly Gly Gly Gly Ser Gly Gly Gly Gly Ser Gly Gly Gly
    450                 455                 460

Gly Ser Glu Ile Val Leu Thr Gln Ser Pro Gly Thr Leu Ser Leu Ser
465                 470                 475                 480

Pro Gly Glu Arg Ala Thr Leu Ser Cys Arg Ala Ser Gln Ser Val Ser
                485                 490                 495

Arg Ser Tyr Leu Ala Trp Tyr Gln Gln Lys Pro Gly Gln Ala Pro Arg
        500                 505                 510

Leu Leu Ile Ile Gly Ala Ser Thr Arg Ala Thr Gly Ile Pro Asp Arg
        515                 520                 525

Phe Ser Gly Ser Gly Ser Gly Thr Asp Phe Thr Leu Thr Ile Ser Arg
    530                 535                 540

Leu Glu Pro Glu Asp Phe Ala Val Tyr Tyr Cys Gln Gln Gly Gln Val
545                 550                 555                 560

Ile Pro Pro Thr Phe Gly Gln Gly Thr Lys Val Glu Ile Lys Ser Ser
                565                 570                 575

Ala Ser Thr Lys Gly Pro Ser Val Phe Pro Leu Ala Pro Ser Ser Lys
            580                 585                 590

Ser Thr Ser Gly Gly Thr Ala Ala Leu Gly Cys Leu Val Lys Asp Tyr
        595                 600                 605

Phe Pro Glu Pro Val Thr Val Ser Trp Asn Ser Gly Ala Leu Thr Ser
    610                 615                 620

Gly Val His Thr Phe Pro Ala Val Leu Gln Ser Ser Gly Leu Tyr Ser
625                 630                 635                 640

Leu Ser Ser Val Val Thr Val Pro Ser Ser Ser Leu Gly Thr Gln Thr
                645                 650                 655

Tyr Ile Cys Asn Val Asn His Lys Pro Ser Asn Thr Lys Val Asp Lys
            660                 665                 670

Lys Val Glu Pro Lys Ser Cys Asp
        675                 680

<210>  29
<211>  223
<212>  PRT
<213>  Artificial

<220>
<223>  DR5TAA-0082 LC2 (FAP crossed-Vh-CL)

<400>  29

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Val Ala Ala Pro Ser Val Phe Ile Phe Pro
        115                 120                 125

Pro Ser Asp Glu Glu Leu Lys Ser Gly Thr Ala Ser Val Val Cys Leu
    130                 135                 140

Leu Asn Asn Phe Tyr Pro Arg Glu Ala Lys Val Gln Trp Lys Val Asp
145                 150                 155                 160

Asn Ala Leu Gln Ser Gly Asn Ser Gln Glu Ser Val Thr Glu Gln Asp
                165                 170                 175

Ser Lys Asp Ser Thr Tyr Ser Leu Ser Ser Thr Leu Thr Leu Ser Lys
            180                 185                 190

Ala Asp Tyr Glu Lys His Lys Val Tyr Ala Cys Glu Val Thr His Gln
        195                 200                 205

Gly Leu Ser Ser Pro Val Thr Lys Ser Phe Asn Arg Gly Glu Cys
    210                 215                 220

<210>  30
<211>  224
<212>  PRT
<213>  Artificial

<220>
<223>  DR5TAA-0083 LC1 (DR5-Vh-CL)

<400>  30

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser Ala Ser Val Ala Ala Pro Ser Val Phe Ile Phe
        115                 120                 125

Pro Pro Ser Asp Glu Glu Leu Lys Ser Gly Thr Ala Ser Val Val Cys
    130                 135                 140

Leu Leu Asn Asn Phe Tyr Pro Arg Glu Ala Lys Val Gln Trp Lys Val
145                 150                 155                 160

Asp Asn Ala Leu Gln Ser Gly Asn Ser Gln Glu Ser Val Thr Glu Gln
                165                 170                 175

Asp Ser Lys Asp Ser Thr Tyr Ser Leu Ser Ser Thr Leu Thr Leu Ser
            180                 185                 190

Lys Ala Asp Tyr Glu Lys His Lys Val Tyr Ala Cys Glu Val Thr His
        195                 200                 205

Gln Gly Leu Ser Ser Pro Val Thr Lys Ser Phe Asn Arg Gly Glu Cys
    210                 215                 220

<210>  31
<211>  679
<212>  PRT
<213>  Artificial

<220>
<223>  DR5TAA-0083 HC (DR5-VL-CH1-  G4S-FAP-Vh-CH1)

<400>  31

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys Ser Ser Ala Ser
            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
        195                 200                 205

Glu Pro Lys Ser Cys Asp Lys Thr His Thr Cys Pro Pro Cys Pro Ala
    210                 215                 220

Pro Glu Ala Ala Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser His Glu Asp Pro Glu Val Lys Phe Asn Trp Tyr Val

            260                 265                 270

Asp Gly Val Glu Val His Asn Ala Lys Thr Lys Pro Arg Glu Glu Gln
        275                 280                 285

Tyr Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Ala
305                 310                 315                 320

Leu Gly Ala Pro Ile Glu Lys Thr Ile Ser Lys Ala Lys Gly Gln Pro
                325                 330                 335

Arg Glu Pro Gln Val Tyr Thr Leu Pro Pro Ser Arg Asp Glu Leu Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Lys Leu Thr Val Asp Lys Ser Arg Trp Gln Gln Gly Asn Val Phe
                405                 410                 415

Ser Cys Ser Val Met His Glu Ala Leu His Asn His Tyr Thr Gln Lys
            420                 425                 430

Ser Leu Ser Leu Ser Pro Gly Gly Gly Gly Gly Ser Gly Gly Gly Gly
        435                 440                 445

Ser Gly Gly Gly Gly Ser Gly Gly Gly Gly Ser Glu Val Gln Leu Leu
    450                 455                 460

Glu Ser Gly Gly Gly Leu Val Gln Pro Gly Gly Ser Leu Arg Leu Ser
465                 470                 475                 480

Cys Ala Ala Ser Gly Phe Thr Phe Ser Ser His Ala Met Ser Trp Val
                485                 490                 495

Arg Gln Ala Pro Gly Lys Gly Leu Glu Trp Val Ser Ala Ile Trp Ala
            500                 505                 510

Ser Gly Glu Gln Tyr Tyr Ala Asp Ser Val Lys Gly Arg Phe Thr Ile
        515                 520                 525

Ser Arg Asp Asn Ser Lys Asn Thr Leu Tyr Leu Gln Met Asn Ser Leu
    530                 535                 540

Arg Ala Glu Asp Thr Ala Val Tyr Tyr Cys Ala Lys Gly Trp Leu Gly
545                 550                 555                 560

Asn Phe Asp Tyr Trp Gly Gln Gly Thr Leu Val Thr Val Ser Ser Ala
                565                 570                 575

Ser Thr Lys Gly Pro Ser Val Phe Pro Leu Ala Pro Ser Ser Lys Ser
            580                 585                 590

Thr Ser Gly Gly Thr Ala Ala Leu Gly Cys Leu Val Glu Asp Tyr Phe
        595                 600                 605

Pro Glu Pro Val Thr Val Ser Trp Asn Ser Gly Ala Leu Thr Ser Gly
    610                 615                 620

Val His Thr Phe Pro Ala Val Leu Gln Ser Ser Gly Leu Tyr Ser Leu
625                 630                 635                 640

Ser Ser Val Val Thr Val Pro Ser Ser Ser Leu Gly Thr Gln Thr Tyr
                645                 650                 655

Ile Cys Asn Val Asn His Lys Pro Ser Asn Thr Lys Val Asp Glu Lys
            660                 665                 670

Val Glu Pro Lys Ser Cys Asp
        675

<210>  32
<211>  215
<212>  PRT
<213>  Artificial

<220>
<223>  DR5TAA-0083 LC2 (FAP-Vl-CL)

<400>  32

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu

35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Arg Thr Val Ala
            100                 105                 110

Ala Pro Ser Val Phe Ile Phe Pro Pro Ser Asp Arg Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  33
<211>  108
<212>  PRT
<213>  Artificial

<220>
<223>  VH <DR5>

<400>  33

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Thr Thr His Pro
                85                  90                  95

Ile Thr Phe Gly Gln Gly Thr Lys Val Glu Ile Lys
            100                 105

<210>  34
<211>  117
<212>  PRT
<213>  Artificial

<220>
<223>  VL <DR5>

<400>  34

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
            100                 105                 110

Val Thr Val Ser Ser
        115

<210>  35
<211>  118
<212>  PRT
<213>  Artificial

<220>
<223>  VH <FAP>

<400>  35

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Pro Ser
        115


<210>  36
<211>  108
<212>  PRT
<213>  Artificial

<220>
<223>  VL <FAP>

<400>  36

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys
            100                 105

<210>  37
<211>  685
<212>  PRT
<213>  Artificial

<220>
<223>  heavy chain of 2+2 PD1TIM3_0358: chimeric PD1-0103 / Tim3-0028

<400>  37

Glu Val Gln Leu Gln Gln Ser Val Ala Glu Leu Val Arg Pro Gly Ala
1               5                   10                  15

Ser Val Lys Leu Ser Cys Thr Ala Ser Gly Phe Asn Ile Lys Thr Thr
            20                  25                  30

Tyr Met His Trp Val Lys Gln Arg Pro Glu Gln Gly Leu Glu Trp Ile
        35                  40                  45

Gly Arg Ile Asp Pro Ala Asp Asp Asn Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Ala Ala Ile Tyr Tyr Cys
                85                  90                  95

Val Arg Asp Phe Gly Tyr Val Ala Trp Phe Ala Tyr Trp Gly Gln Gly
            100                 105                 110

Thr Leu Val Thr Phe Ser Ala Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Gly Gly Gly Ser Gly Gly Gly Gly Ser Gly Gly Gly Gly Ser Gly
    450                 455                 460

Gly Gly Gly Ser Lys Ile Val Leu Thr Gln Ser Pro Ala Ser Leu Pro
465                 470                 475                 480

Val Ser Leu Gly Gln Arg Ala Thr Ile Ser Cys Arg Ala Ser Glu Ser
                485                 490                 495

Val Asp Thr Ser Asp Asn Ser Phe Ile His Trp Tyr Gln Gln Arg Pro
            500                 505                 510

Gly Gln Ser Pro Lys Leu Leu Ile Tyr Arg Ser Ser Thr Leu Glu Ser
        515                 520                 525

Gly Val Pro Ala Arg Phe Ser Gly Ser Gly Ser Arg Thr Asp Phe Thr
    530                 535                 540

Leu Thr Ile Asp Pro Val Glu Ala Asp Asp Val Ala Thr Tyr Tyr Cys
545                 550                 555                 560

Gln Gln Asn Tyr Asp Val Pro Trp Thr Phe Gly Gly Gly Thr Lys Leu
                565                 570                 575

Glu Ile Lys Ser Ser Ala Ser Thr Lys Gly Pro Ser Val Phe Pro Leu
            580                 585                 590

Ala Pro Ser Ser Lys Ser Thr Ser Gly Gly Thr Ala Ala Leu Gly Cys
        595                 600                 605

Leu Val Lys Asp Tyr Phe Pro Glu Pro Val Thr Val Ser Trp Asn Ser
    610                 615                 620

Gly Ala Leu Thr Ser Gly Val His Thr Phe Pro Ala Val Leu Gln Ser
625                 630                 635                 640

Ser Gly Leu Tyr Ser Leu Ser Ser Val Val Thr Val Pro Ser Ser Ser

645                 650                 655

Leu Gly Thr Gln Thr Tyr Ile Cys Asn Val Asn His Lys Pro Ser Asn
            660                 665                 670

Thr Lys Val Asp Lys Lys Val Glu Pro Lys Ser Cys Asp
        675                 680                 685

<210>  38
<211>  200
<212>  PRT
<213>  Artificial

<220>
<223>  light chain 1 of 2+2 PD1TIM3_0358

<400>  38

Asn Ile Val Met Thr Pro Thr Pro Lys Phe Leu Pro Val Ser Ser Gly
1               5                   10                  15

Asp Arg Val Thr Met Thr Cys Arg Ala Ser Gln Ser Val Asp Asn Tyr
            20                  25                  30

Val Ala Trp Tyr Gln Gln Lys Pro Gly Gln Ser Pro Lys Leu Leu Ile
        35                  40                  45

Tyr Tyr Ala Ser Asn Arg Tyr Ile Gly Val Pro Asp Arg Phe Thr Gly
    50                  55                  60

Ser Gly Ser Gly Thr Asp Phe Thr Phe Thr Ile Ser Ser Val Gln Val
65                  70                  75                  80

Glu Asp Leu Ala Val Tyr Phe Cys Gln Gln His Tyr Ser Ser Pro Tyr
                85                  90                  95

Thr Phe Gly Ser Gly Thr Lys Leu Glu Ile Lys Arg Thr Val Ala Ala
            100                 105                 110

Pro Ser Val Phe Ile Phe Pro Pro Ser Asp Arg Lys Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly
        195                 200

<210> 39
<211> 227
<212> PRT
<213> Artificial

<220>
<223> light chain 2 of 2+2 PD1TIM3_0358

<400> 39

Glu Val Ile Leu Val Glu Ser Gly Gly Gly Leu Val Lys Pro Gly Gly
1               5                   10                  15

Ser Leu Lys Leu Ser Cys Ala Ala Ser Gly Phe Ser Phe Ser Ser Tyr
            20                  25                  30

Thr Met Ser Trp Val Arg Gln Thr Pro Glu Lys Arg Leu Asp Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Glu Met Ser Ser Leu Met Ser Glu Asp Thr Ala Leu Tyr Tyr Cys
                85                  90                  95

Val Leu Leu Thr Gly Arg Val Tyr Phe Ala Leu Asp Ser Trp Gly Gln
            100                 105                 110

Gly Thr Ser Val Thr Val Ser Ser Ala Ser Val Ala Ala Pro Ser Val
        115                 120                 125

Phe Ile Phe Pro Pro Ser Asp Glu Gln Leu Lys Ser Gly Thr Ala Ser
    130                 135                 140

Val Val Cys Leu Leu Asn Asn Phe Tyr Pro Arg Glu Ala Lys Val Gln
145                 150                 155                 160

Trp Lys Val Asp Asn Ala Leu Gln Ser Gly Asn Ser Gln Glu Ser Val
                165                 170                 175

Thr Glu Gln Asp Ser Lys Asp Ser Thr Tyr Ser Leu Ser Ser Thr Leu
            180                 185                 190

Thr Leu Ser Lys Ala Asp Tyr Glu Lys His Lys Val Tyr Ala Cys Glu
        195                 200                 205

Val Thr His Gln Gly Leu Ser Ser Pro Val Thr Lys Ser Phe Asn Arg
    210                 215                 220

Gly Glu Cys
225

<210> 40
<211> 686
<212> PRT
<213> Artificial

<220>
<223> heavy chain of 2+2 PD1TIM3_0359: chimeric PD1-0103 / Tim3-0018

<400> 40

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Ser Thr Ser
            20                  25                  30

Gly Met Ser Val Gly Trp Ile Arg Gln Pro Ser Gly Lys Gly Leu Glu
        35                  40                  45

Trp Leu Ala His Ile Trp Leu Asn Asp Asp Val Phe Phe Asn Pro Ala
    50                  55                  60

Leu Lys Arg Arg Leu Thr Ile Ser Lys Asp Thr Ser Asn Asn Gln Val
65                  70                  75                  80

Phe Leu Gln Ile Ala Ser Val Val Thr Ala Asp Thr Ala Thr Tyr Tyr
                85                  90                  95

Cys Val Arg Ala Asn Gly Tyr Leu Tyr Ala Leu Asp Tyr Trp Gly Gln
            100                 105                 110

Gly Ile Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Glu Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Glu Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Gly Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Gly Gly Gly Gly Ser Gly Gly Gly Gly Ser Gly Gly Gly Gly Ser
    450                 455                 460

Gly Gly Gly Gly Ser Lys Ile Val Leu Thr Gln Ser Pro Ala Ser Leu
465                 470                 475                 480

Pro Val Ser Leu Gly Gln Arg Ala Thr Ile Ser Cys Arg Ala Ser Glu
                485                 490                 495

Ser Val Asp Thr Ser Asp Asn Ser Phe Ile His Trp Tyr Gln Gln Arg
            500                 505                 510

Pro Gly Gln Ser Pro Lys Leu Leu Ile Tyr Arg Ser Ser Thr Leu Glu
        515                 520                 525

Ser Gly Val Pro Ala Arg Phe Ser Gly Ser Gly Ser Arg Thr Asp Phe
    530                 535                 540

Thr Leu Thr Ile Asp Pro Val Glu Ala Asp Asp Val Ala Thr Tyr Tyr
545                 550                 555                 560

Cys Gln Gln Asn Tyr Asp Val Pro Trp Thr Phe Gly Gly Gly Thr Lys
                565                 570                 575

Leu Glu Ile Lys Ser Ser Ala Ser Thr Lys Gly Pro Ser Val Phe Pro
            580                 585                 590

Leu Ala Pro Ser Ser Lys Ser Thr Ser Gly Gly Thr Ala Ala Leu Gly
        595                 600                 605

Cys Leu Val Lys Asp Tyr Phe Pro Glu Pro Val Thr Val Ser Trp Asn
    610                 615                 620

Ser Gly Ala Leu Thr Ser Gly Val His Thr Phe Pro Ala Val Leu Gln
625                 630                 635                 640

Ser Ser Gly Leu Tyr Ser Leu Ser Ser Val Val Thr Val Pro Ser Ser
                645                 650                 655

Ser Leu Gly Thr Gln Thr Tyr Ile Cys Asn Val Asn His Lys Pro Ser
            660                 665                 670

Asn Thr Lys Val Asp Lys Lys Val Glu Pro Lys Ser Cys Asp
        675                 680                 685

<210> 41
<211> 213
<212> PRT
<213> Artificial

<220>
<223> light chain 1 of 2+2 PD1TIM3_0359

<400> 41

Gln Ile Val Leu Thr Gln Ser Pro Ala Ile Met Ser Ala Ser Pro Gly
1               5                   10                  15

Gln Lys Val Thr Ile Thr Cys Ser Ala Ser Ser Ser Val Asn Tyr Thr
            20                  25                  30

Gln Trp Tyr Gln Gln Lys Leu Gly Ser Ser Pro Lys Leu Trp Ile Tyr
        35                  40                  45

Asp Ala Phe Lys Leu Ala Pro Gly Val Pro Ala Arg Phe Ser Gly Ser
    50                  55                  60

Gly Thr Gly Thr Ser Tyr Ser Leu Thr Ile Ser Ser Met Glu Ala Glu
65                  70                  75                  80

Asp Ala Ala Ser Tyr Phe Cys His Gln Trp Ser Ser Tyr Pro Trp Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Arg Lys Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
                180                 185                 190

Cys Glu Val Thr His Gln Gly Leu Ser Ser Pro Val Thr Lys Ser Phe
        195                 200                 205

Asn Arg Gly Glu Cys
    210

<210>  42
<211>  227
<212>  PRT
<213>  Artificial

<220>
<223>  light chain 2 of 2+2 PD1TIM3_0359

<400>  42

Glu Val Ile Leu Val Glu Ser Gly Gly Gly Leu Val Lys Pro Gly Gly
1               5                   10                  15

Ser Leu Lys Leu Ser Cys Ala Ala Ser Gly Phe Ser Phe Ser Ser Tyr
                20                  25                  30

Thr Met Ser Trp Val Arg Gln Thr Pro Glu Lys Arg Leu Asp Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Glu Met Ser Ser Leu Met Ser Glu Asp Thr Ala Leu Tyr Tyr Cys
                85                  90                  95

Val Leu Leu Thr Gly Arg Val Tyr Phe Ala Leu Asp Ser Trp Gly Gln
                100                 105                 110

Gly Thr Ser Val Thr Val Ser Ser Ala Ser Val Ala Ala Pro Ser Val
        115                 120                 125

Phe Ile Phe Pro Pro Ser Asp Glu Gln Leu Lys Ser Gly Thr Ala Ser
    130                 135                 140

Val Val Cys Leu Leu Asn Asn Phe Tyr Pro Arg Glu Ala Lys Val Gln
145                 150                 155                 160

Trp Lys Val Asp Asn Ala Leu Gln Ser Gly Asn Ser Gln Glu Ser Val
                165                 170                 175

Thr Glu Gln Asp Ser Lys Asp Ser Thr Tyr Ser Leu Ser Ser Thr Leu
            180                 185                 190

Thr Leu Ser Lys Ala Asp Tyr Glu Lys His Lys Val Tyr Ala Cys Glu
        195                 200                 205

Val Thr His Gln Gly Leu Ser Ser Pro Val Thr Lys Ser Phe Asn Arg
    210                 215                 220

Gly Glu Cys
225

<210>  43
<211>  685
<212>  PRT
<213>  Artificial

<220>
<223>  heavy chain of 2+2 PD1TIM3_0321: chimeric PD1-0103 / Tim3-0038

<400>  43

Glu Val Gln Leu Gln Gln Ser Gly Ala Glu Pro Leu Lys Pro Gly Ala
1               5                   10                  15

Ser Val Lys Leu Thr Cys Thr Thr Ser Gly Phe Asn Ile Lys Asp Tyr
            20                  25                  30

Tyr Ile His Trp Val Lys Gln Arg Ser Asp Gln Gly Leu Glu Trp Ile
        35                  40                  45

Gly Arg Ile Asp Pro Glu Asp Gly Glu Leu Ile Tyr Ala Pro Lys Phe
    50                  55                  60

Gln Asp Lys Ala Thr Ile Thr Val Asp Thr Ser Ser Asn Ile Ala Tyr
65                  70                  75                  80

Leu Gln Leu Asn Ser Leu Thr Ser Glu Asp Thr Ala Val Tyr Tyr Cys
                85                  90                  95

Ser Arg Asp His Gly Tyr Val Gly Trp Phe Ala Tyr Trp Gly Gln Gly
            100                 105                 110

Thr Leu Val Thr Val Ser Ala Ala Ser Thr Lys Gly Pro Ser Val Phe
        115                 120                 125

Pro Leu Ala Pro Ser Ser Lys Ser Thr Ser Gly Gly Thr Ala Ala Leu

130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Gly Gly Gly Gly Ser Gly Gly Gly Gly Ser Gly Gly Gly Gly Ser Gly
    450                 455                 460

Gly Gly Gly Ser Lys Ile Val Leu Thr Gln Ser Pro Ala Ser Leu Pro
465                 470                 475                 480

Val Ser Leu Gly Gln Arg Ala Thr Ile Ser Cys Arg Ala Ser Glu Ser
                485                 490                 495

Val Asp Thr Ser Asp Asn Ser Phe Ile His Trp Tyr Gln Gln Arg Pro
            500                 505                 510

Gly Gln Ser Pro Lys Leu Leu Ile Tyr Arg Ser Ser Thr Leu Glu Ser
        515                 520                 525

Gly Val Pro Ala Arg Phe Ser Gly Ser Gly Ser Arg Thr Asp Phe Thr
    530                 535                 540

Leu Thr Ile Asp Pro Val Glu Ala Asp Asp Val Ala Thr Tyr Tyr Cys
545                 550                 555                 560

Gln Gln Asn Tyr Asp Val Pro Trp Thr Phe Gly Gly Gly Thr Lys Leu
                565                 570                 575

Glu Ile Lys Ser Ser Ala Ser Thr Lys Gly Pro Ser Val Phe Pro Leu
            580                 585                 590

Ala Pro Ser Ser Lys Ser Thr Ser Gly Gly Thr Ala Ala Leu Gly Cys
        595                 600                 605

Leu Val Lys Asp Tyr Phe Pro Glu Pro Val Thr Val Ser Trp Asn Ser
    610                 615                 620

Gly Ala Leu Thr Ser Gly Val His Thr Phe Pro Ala Val Leu Gln Ser
625                 630                 635                 640

Ser Gly Leu Tyr Ser Leu Ser Ser Val Val Thr Val Pro Ser Ser Ser
                645                 650                 655

Leu Gly Thr Gln Thr Tyr Ile Cys Asn Val Asn His Lys Pro Ser Asn
            660                 665                 670

Thr Lys Val Asp Lys Lys Val Glu Pro Lys Ser Cys Asp
        675                 680                 685

<210>  44
<211>  214
<212>  PRT
<213>  Artificial

<220>
<223>  light chain 1 of 2+2 PD1TIM3_0321

<400>  44

Asn Val Val Met Thr Gln Ser Pro Lys Ser Met Ile Met Ser Val Gly
1               5                   10                  15

Gln Arg Val Thr Leu Asn Cys Lys Ala Ser Glu Asn Val Asp Thr Tyr
            20                  25                  30

Val Ser Trp Tyr Gln Gln Lys Pro Glu Gln Ser Pro Lys Leu Leu Ile
        35                  40                  45

Tyr Gly Ala Ser Asn Arg Tyr Thr Gly Val Pro Asp Arg Phe Thr Gly
    50                  55                  60

Ser Arg Ser Ala Thr Asp Phe Thr Leu Thr Ile Ser Ser Val Gln Ala
65                  70                  75                  80

Glu Asp Leu Ala Val Tyr Tyr Cys Gly Gln Ser Tyr Ser Tyr Pro Trp
                85                  90                  95

Thr Phe Gly Gly Gly Thr Lys Leu Glu Phe Arg Arg Thr Val Ala Ala
            100                 105                 110

Pro Ser Val Phe Ile Phe Pro Pro Ser Asp Arg Lys Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  45
<211>  227
<212>  PRT
<213>  Artificial

<220>
<223>  light chain 2 of 2+2 PD1TIM3_0321

<400>  45

Glu Val Ile Leu Val Glu Ser Gly Gly Gly Leu Val Lys Pro Gly Gly
1               5                   10                  15

Ser Leu Lys Leu Ser Cys Ala Ala Ser Gly Phe Ser Phe Ser Ser Tyr
            20                  25                  30

Thr Met Ser Trp Val Arg Gln Thr Pro Glu Lys Arg Leu Asp Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Glu Met Ser Ser Leu Met Ser Glu Asp Thr Ala Leu Tyr Tyr Cys
                85                  90                  95

Val Leu Leu Thr Gly Arg Val Tyr Phe Ala Leu Asp Ser Trp Gly Gln
            100                 105                 110

Gly Thr Ser Val Thr Val Ser Ser Ala Ser Val Ala Ala Pro Ser Val
        115                 120                 125

Phe Ile Phe Pro Pro Ser Asp Glu Gln Leu Lys Ser Gly Thr Ala Ser
    130                 135                 140

Val Val Cys Leu Leu Asn Asn Phe Tyr Pro Arg Glu Ala Lys Val Gln

145                 150                 155                 160

Trp Lys Val Asp Asn Ala Leu Gln Ser Gly Asn Ser Gln Glu Ser Val
                165                 170                 175

Thr Glu Gln Asp Ser Lys Asp Ser Thr Tyr Ser Leu Ser Ser Thr Leu
            180                 185                 190

Thr Leu Ser Lys Ala Asp Tyr Glu Lys His Lys Val Tyr Ala Cys Glu
        195                 200                 205

Val Thr His Gln Gly Leu Ser Ser Pro Val Thr Lys Ser Phe Asn Arg
    210                 215                 220

Gly Glu Cys
225

<210>  46
<211>  120
<212>  PRT
<213>  Artificial

<220>
<223>  VH humanized version of PD1-0103 (=PD1-0103_0312)

<400>  46

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Ser Phe Ser Ser Tyr
            20                  25                  30

Thr Met Ser Trp Val Arg Gln Ala Pro Gly Lys Gly Leu Glu Trp Val
        35                  40                  45

Ala Thr Ile Ser Gly Gly Gly Arg Asp Ile Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Leu Leu Thr Gly Arg Val Tyr Phe Ala Leu Asp Ser Trp Gly Gln
            100                 105                 110

Gly Thr Leu Val Thr Val Ser Ser
        115                 120

<210>  47
<211>  111
<212>  PRT
<213>  Artificial

<220>
<223>  VL humanized version of PD1-0103 (=PD1-0103_0312)

<400>  47

Asp Ile Val Met Thr Gln Ser Pro Asp Ser Leu Ala Val Ser Leu Gly
1               5                   10                  15

Glu Arg Ala Thr Ile Asn Cys Lys Ala Ser Glu Ser Val Asp Thr Ser
            20                  25                  30

Asp Asn Ser Phe Ile His Trp Tyr Gln Gln Lys Pro Gly Gln Ser Pro
        35                  40                  45

Lys Leu Leu Ile Tyr Arg Ser Ser Thr Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Asn Tyr
                85                  90                  95

Asp Val Pro Trp Thr Phe Gly Gln Gly Thr Lys Val Glu Ile Lys
            100                 105                 110


<210>  48
<211>  119
<212>  PRT
<213>  Artificial

<220>
<223>  VH humanized version of Tim3-0028 (= Tim3-0438)

<400>  48

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Thr Thr
            20                  25                  30

Tyr Met His Trp Val Arg Gln Ala Pro Gly Lys Gly Leu Glu Trp Val
        35                  40                  45

Gly Arg Ile Asp Pro Ala Asp Asp Asn Thr Lys Tyr Ala Pro Lys Phe

50                  55                  60

Gln Gly Lys Ala Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Val Arg Asp Phe Gly Tyr Val Ala Trp Phe Ala Tyr Trp Gly Gln Gly
            100                 105                 110

Thr Leu Val Thr Val Ser Ser
        115

<210>  49
<211>  107
<212>  PRT
<213>  Artificial

<220>
<223>  VL humanized version of Tim3-0028 (= Tim3-0438)

<400>  49

Asp Ile Val Met Thr Gln Ser Pro Leu Ser Leu Pro Val Thr Pro Gly
1               5                   10                  15

Glu Pro Ala Ser Ile Ser Cys Arg Ala Ser Gln Ser Val Asp Asn Tyr
            20                  25                  30

Val Ala Trp Tyr Leu Gln Lys Pro Gly Gln Ser Pro Gln Leu Leu Ile
        35                  40                  45

Tyr Tyr Ala Ser Asn Arg Tyr Ile Gly Val Pro Asp Arg Phe Ser Gly
    50                  55                  60

Ser Gly Ser Gly Thr Asp Phe Thr Leu Lys Ile Ser Arg Val Glu Ala
65                  70                  75                  80

Glu Asp Val Gly Val Tyr Tyr Cys Gln Gln His Tyr Ser Ser Pro Tyr
                85                  90                  95

Thr Phe Gly Gln Gly Thr Lys Val Glu Ile Lys
            100                 105

<210>  50
<211>  120
<212>  PRT
<213>  Artificial

<220>

<223>  VH humanized version of Tim3_0016 variant (0018) (= Tim3-0434)

<400>  50

Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Ser Val Gly Trp Ile Arg Gln Pro Pro Gly Lys Gly Leu Glu
        35                  40                  45

Trp Leu Ala His Ile Trp Leu Asn Asp Asp Val Phe Phe Asn Pro Ala
    50                  55                  60

Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Val Arg Ala Asn Gly Tyr Leu Tyr Ala Leu Asp Tyr Trp Gly Gln
            100                 105                 110

Gly Thr Leu Val Thr Val Ser Ser
        115                 120

<210>  51
<211>  106
<212>  PRT
<213>  Artificial

<220>
<223>  VL humanized version of Tim3_0016 variant (0018) (= Tim3-0434)

<400>  51

Asp Ile Gln Leu Thr Gln Ser Pro Ser Phe Leu Ser Ala Ser Val Gly
1               5                   10                  15

Asp Arg Val Thr Ile Thr Cys Ser Ala Ser Ser Ser Val Ser Tyr Thr
            20                  25                  30

Gln Trp Tyr Gln Gln Lys Pro Gly Lys Ala Pro Lys Leu Trp Ile Tyr
        35                  40                  45

Asp Ala Phe Lys Leu Ala Pro Gly Val Pro Ser Arg Phe Ser Gly Ser
    50                  55                  60

Gly Ser Gly Thr Glu Phe Thr Leu Thr Ile Ser Ser Leu Gln Pro Glu

65                  70                  75                  80

Asp Phe Ala Thr Tyr Phe Cys His Gln Trp Ser Ser Tyr Pro Trp Thr
                85                  90                  95

Phe Gly Gln Gly Thr Lys Leu Glu Ile Lys
            100                 105